# EUROPEAN PATENT SPECIFICATION

(11) **EP 4 263 036 B1**
(45) Date of publication and mention of the grant of the patent: **28.01.2026**
(21) Application number: 21841100.7
(22) Date of filing: 14.12.2021
(51) Int. Cl.: B01F 31/23, B01F 31/20, B01F 35/75, B01F 35/21, C12M 3/06

(54) **SYSTEM, METHOD AND APPARATUS FOR MIXING A FLUID IN A BIOPROCESSING SYSTEM**
SYSTEM, VERFAHREN UND VORRICHTUNG ZUM MISCHEN EINES FLUIDS IN EINEM BIOVERARBEITUNGSSYSTEM
SYSTÈME, PROCÉDÉ ET APPAREIL POUR MÉLANGER UN FLUIDE DANS UN SYSTÈME DE BIOTRAITEMENT

(30) Priority: 15.12.2020 US 202063125858 P
(43) Date of publication of application: 25.10.2023
(73) Proprietor: Global Life Sciences Solutions USA LLC, Marlborough, MA 01752 (US)
(72) Inventor: FONTOVA SOSA, Andreu, Marlborough, Massachusetts 01752 (US); GRAHN, Magnus, Marlborough, Massachusetts 01752 (US); PETITFOURG, Nicolas, Marlborough, Massachusetts 01752 (US)
(74) Representative: Bedford, Grant Richard
(86) International application number: PCT/US2021/063311
(87) International publication number: WO 2022/132770

(56) References cited:
- WO-A1-2016/084001
- DE-U1- 202015 001 877
- US-A- 3 822 865
- US-A1- 2014 024 105
- US-A1- 2016 097 583
- US-B1- 6 194 160

## Description

### TECHNICAL FIELD

Embodiments of the invention relate generally to bioprocessing systems and methods and, more particularly, to a bioprocessing system and methods for the production of cellular immunotherapies.

### DISCUSSION OF ART

Various medical therapies involve the extraction, culture and expansion of cells for use in downstream therapeutic processes. For example, chimeric antigen receptor (CAR) T cell therapy is a cellular therapy that redirects a patient's T cells to specifically target and destroy tumor cells. The basic principle of CAR-T cell design involves recombinant receptors that combine antigen-binding and T-cell activating functions. The general premise of CAR-T cells is to artificially generate T-cells targeted to markers found on cancer cells. Scientists can remove T-cells from a person, genetically alter them, and put them back into the patient for them to attack the cancer cells. CAR-T cells can be derived from either a patient's own blood (autologous) or derived from another healthy donor (allogenic).

The first step in the production of CAR-T cells involves using apheresis, e.g., leukocyte apheresis, to remove blood from a patient's body and separate the leukocytes. After a sufficient quantity of leukocytes have been harvested, the leukapheresis product is enriched for T-cells, which involves depleting unwanted cell types. T-cell subsets having particular bio-markers can then, if desired, be isolated from the enriched sub-population using specific antibody conjugates or markers.

After isolation of targeted T-cells, the cells are activated in a certain environment in which they can actively proliferate. For example, the cells may be activated using magnetic beads coated with anti-CD3/anti-CD28 monoclonal antibodies or cell-based artificial antigen presenting cells (aAPCs), which can be removed from the culture using magnetic separation. The T-cells are then transduced with CAR genes by either an integrating gammaretrovirus (RV) or by lentivirus (LV) vectors. The viral vector uses viral machinery to attach to the patient cells, and, upon entry into the cells, the vector introduces genetic material in the form of RNA. In the case of CAR-T cell therapy, this genetic material encodes the CAR. The RNA is reverse-transcribed into DNA and permanently integrates into the genome of the patient cells; allowing CAR expression to be maintained as the cells divide and are grown to large numbers in a bioreactor. The CAR is then transcribed and translated by the patient cells, and the CAR is expressed on the cell surface.

After the T cells are activated and transduced with the CAR-encoding viral vector, the cells are expanded to large numbers in a bioreactor to achieve a desired cell density. After expansion, the cells are harvested, washed, concentrated and formulated for infusion into a patient.

Existing systems and methods for manufacturing an infusible dose of CAR T cells have typically required many complex operations involving a large number of human touchpoints, which adds time to the overall manufacturing process and increases the risk of contamination. While recent efforts to automate the manufacturing process have eliminated some human touchpoints, these systems may still suffer from high cost, inflexibility and workflow bottlenecks. In particular, systems utilizing increased automation are very costly and inflexible, in that they require customers to adapt their processes to the particular equipment of the system. WIPO International Publication No. WO 2019/106207 discloses systems and methods for bioprocessing which have successfully addressed many of the shortcomings of the prior art. Various related devices and methods are also known (see, for example, US 2016/097583 A1, which discloses the preamble of claims 1 and 8, US 2014/024105 A1, DE 20 2015 001 877 U1, US 6,194,160 B1 and US 3,822,865 A).

In view of the above, however, there is a need for bioprocessing systems and methods that improve upon the teachings contained in the '207 publication in terms of overall functionality, flexibility, adaptability, and ease of use.

### BRIEF DESCRIPTION

Certain embodiments commensurate in scope with the originally claimed subject matter are summarized below. These embodiments are not intended to limit the scope of the claimed subject matter, but rather these embodiments are intended only to provide a brief summary of the possible embodiments. Indeed, the disclosure may encompass a variety of forms that may be similar to or different from the embodiments set forth below.

The present invention is defined by the appended claims.

### DRAWINGS

The present invention will be better understood from reading the following description of non-limiting embodiments, with reference to the attached drawings, wherein below:
FIG. 1 is a schematic illustration of a bioprocessing system according to a disclosed embodiment.
FIG. 2 is a schematic illustration of a bioprocessing system according to another disclosed embodiment.
FIG. 3 is a schematic illustrating of a cell processing and isolation system according to a disclosed embodiment.
FIG. 4 is a perspective view of an isolation module of the cell processing and isolation system of FIG. 3.
FIG. 5 is a top plan view of the isolation module.
FIG. 6 is a perspective view of a stopcock manifold interface of the isolation module, according to a disclosed embodiment.
FIG. 7 is an enlarged, perspective view of the stopcock manifold interface.
FIG. 8 is another perspective view of the isolation module.
FIG. 9 is a rear perspective view of the isolation module.
FIG. 10 is a front, exploded perspective view of the isolation module.
FIG. 11 is a rear, exploded perspective view of the isolation module.
FIG. 12 is an enlarged, perspective view of a bubble sensor assembly of the isolation module.
FIG. 13 is a side, cross-sectional view of the bubble sensor assembly.
FIG. 14 is a front, perspective view of a magnetic field generator assembly of the isolation module, according to a disclosed embodiment.
FIG. 15 is another front, perspective view of the magnetic field generator assembly.
FIG. 16 is a rear, perspective view of the magnetic field generator assembly.
FIG. 17 is a rear, perspective view of a portion of the magnetic field generator assembly.
FIG. 18 is a simplified front perspective view of a carriage of the magnetic field generator assembly.
FIG. 19 is a simplified rear perspective view of the carriage.
FIG. 20 is a cross-sectional view of the magnetic field generator assembly in a retracted position.
FIG. 21 is a cross-sectional view of the magnetic field generator assembly with an isolation holder receive in a slot of the isolation module.
FIG. 22 is a cross-sectional view of the magnetic field generator assembly in an extended position.
FIG. 23 is a cross-sectional view of the magnetic field generator assembly in the extended position within the isolation holder received in the slot.
FIG. 24 is a cross-sectional view of the magnetic field generator assembly in the extended position and locking the isolation holder within the slot.
FIG. 25 is a cross-sectional view of the magnetic field generator assembly illustrating a misalignment position of the isolation holder.
FIG. 26 is a perspective view of a magnetic cell isolation holder for use with the isolation module of FIG. 4, according to a disclosed embodiment.
FIG. 27 is an exploded, perspective view of the magnetic cell isolation holder of FIG. 26.
FIG. 28 is a side elevational view of a column of the magnetic cell isolation holder of FIG. 26.
FIG. 29 is an exploded view of the column of FIG. 28.
FIG. 30 is a perspective view illustrating insertion of the magnetic cell isolation holder into the slot in the isolation module.
FIG. 31 is a perspective view of a magnetic cell isolation holder for use with the isolation module of FIG. 4, according to another disclosed embodiment.
FIG. 32 is a perspective view of a magnetic cell isolation holder of FIG. 31.
FIG. 33 is a top plan view of the magnetic cell isolation holder of FIG. 31, illustrating the magnetic field distribution of the magnetic field generator, in accordance with aspects of the present disclosure.
FIG. 34 is a simplified, perspective view of a magnetic cell isolation holder according to another disclosed embodiment.
FIG. 35 is a simplified, perspective view of a magnetic cell isolation holder according to yet another disclosed embodiment.
FIG. 36 is a schematic illustration of a disposable kit for washing and concentrating cellular products, for use with the processing apparatus of FIG. 3.
FIG. 37A is a schematic illustration of a disposable kit for magnetic cell isolation, for use with the processing apparatus and isolation module of FIG. 3, and showing installation on the processing apparatus and isolation module of FIG. 3.
FIG. 37B is a schematic illustration of the disposable kit for magnetic cell isolation of FIG. 37A, showing installation on the processing apparatus and isolation module of FIG. 3.
FIG. 38 is a flow chart illustrating a magnetic cell isolation workflow/process utilizing the disposable kit of FIGS. 37A and 37B on the processing apparatus and isolation module of FIG. 3.
FIG. 39 is a schematic illustration of a disposable kit for dosing preparation/formulation, for use with the processing apparatus and isolation module of FIG. 3.
FIG. 40 is a schematic illustration of the disposable kit for dosing preparation/formulation of FIG. 39, showing installation on the processing apparatus and isolation module of FIG. 3.
FIG. 41 is a flow chart illustrating a dosing preparation workflow/process utilizing the disposable kit of FIG. 39 on the processing apparatus and isolation module of FIG. 3.
FIG. 42 is a perspective view of a bioprocessing system/apparatus according to an embodiment of the invention, showing a process drawer and cabinet in a closed position.
FIG. 43 is another perspective view of the bioprocessing apparatus of FIG. 42, showing the cabinet in an open position.
FIG. 44 is a perspective view of the cabinet of the bioprocessing apparatus of FIG. 42, illustrating an extended position of vertical drawers thereof.
FIG. 45 is a front elevational view of the cabinet.
FIG. 46 is a perspective view of a housing and process drawer of the bioprocessing apparatus of FIG. 42, illustrating an open position of the process drawer.
FIG. 47 is a top plan view of the process drawer of the bioprocessing apparatus of FIG. 42.
FIG. 48 is a perspective view of a pair of platform rocker assemblies of the process drawer according to an embodiment of the invention.
FIG. 49 is a perspective view of a waste drawer of the bioprocessing apparatus of FIG. 42.
FIG. 50 is a perspective view of a disposable bioprocessing kit for use with the bioprocessing apparatus of FIG. 42.
FIG. 51 is a rear, perspective view of a tray of the disposable bioprocessing kit of FIG. 50.
FIG. 52 is a perspective view of an anchor comb of the disposable bioprocessing kit of FIG. 50.
FIG. 53 is a front elevational view of the anchor comb of FIG. 52.
FIG. 54 is a perspective view of a tubing organizer of the disposable bioprocessing kit of FIG. 50.
FIG. 55 is a perspective view of a sampling card of the disposable bioprocessing kit of FIG. 50.
FIG. 56 is a front elevational view of the sampling card of FIG. 53.
FIG. 57 is a perspective view illustrating insertion of the tray and culture vessels of the disposable kit into the process drawer of the bioprocessing apparatus.
FIG. 58 is a side, cross-sectional view illustrating the tray and culture vessels of the disposable kit received in the processing drawer of the bioprocessing apparatus.
FIG. 59 is a top view of the process drawer of the bioprocessing apparatus, showing various alignment features and sensors of the bioprocessing apparatus.
FIG. 60 is an enlarged, front perspective view of a peristaltic pump assembly of the bioprocessing apparatus, showing alignment and engagement features thereof.
FIG. 61 is an enlarged, rear perspective view of a peristaltic pump assembly of the bioprocessing apparatus, showing alignment and engagement features thereof.
FIG. 62 is a an enlarged, perspective view of a linear actuator array of the bioprocessing apparatus, showing alignment and engagement features thereof.
FIG. 63 is a perspective, cross-sectional view of the process drawer of the bioprocessing apparatus.
FIG. 64 is an exploded, perspective view of a culture vessel of the disposable bioprocessing kit of FIG. 50.
FIG. 65 is a bottom plan view of the culture vessel of FIG. 64.
FIG. 66 is a perspective view of a portion of a rocking assembly of the bioprocessing apparatus of FIG. 42
FIG. 67 is another perspective view of the rocking assembly of FIG. 66.
FIG. 68 is another perspective view of the rocking assembly of FIG. 66, illustrating engagement of the rocking assembly with a culture vessel.
FIG. 69 is a schematic diagram illustrating operation of the rocking assembly of FIG. 66.
FIG. 70 is a cross-sectional view of the process drawer of the bioprocessing apparatus of FIG. 42.
FIG. 71 is another cross-sectional view of the process drawer of the bioprocessing apparatus of FIG. 42, showing a recirculation air flow path.
FIG. 72 is another cross-sectional view of the process drawer of the bioprocessing apparatus of FIG. 42, showing a turbulent recirculation airflow at in interface with culture vessels.
FIG. 73 is a perspective, cross-sectional view of the tray of the disposable bioprocessing kit of FIG. 50, illustrating a recirculation air flow path.
FIG. 74 is a perspective, cross-sectional view of the process drawer and tray of the bioprocessing apparatus, illustrating the recirculation air flow path.
FIG. 75 is another perspective, cross-sectional view of the process drawer and tray of the bioprocessing apparatus, illustrating the recirculation air flow path.
FIG. 76 is another perspective, cross-sectional view of the process drawer and tray of the bioprocessing apparatus, illustrating the recirculation air flow path.
FIG. 77 is a rear, perspective view of a flow-through sensing chamber of the bioprocessing apparatus of FIG. 42, according to an embodiment of the invention.
FIG. 78 is a front, perspective view of the flow-through sensing chamber of FIG. 77.
FIG. 79 is a cross-sectional, perspective view of the flow-through sensing chamber of FIG. 77.
FIG. 80 is a perspective view of a back plate of the flow-through sensing chamber of FIG. 77.
FIG. 81 is an enlarged, perspective view of a backbone of the disposable bioprocessing kit of FIG. 50, illustrating the location of the flow-through sensing chamber.
FIG. 82 is another enlarged, perspective view of a backbone of the disposable bioprocessing kit of FIG. 50, illustrating the location of the flow-through sensing chamber.
FIG. 83 is a schematic illustration showing integration of the flow-through sensing chamber with various sensing devices.
FIG. 84 is another schematic illustration showing integration of the flow-through sensing chamber with various sensing devices.
FIG. 85 is a block diagram illustrating the fluid flow architecture of the bioprocessing apparatus of FIG. 42, according to a disclosed embodiment.
FIG. 86 is a detail view of a portion of the block diagram of FIG. 85, illustrating a first fluid assembly of the fluid flow architecture.
FIG. 87 is a detail view of a portion of the block diagram of FIG. 85, illustrating a second fluid assembly of the fluid flow architecture.
FIG. 88 is a detail view of a portion of the block diagram of FIG. 85, illustrating a sampling assembly of the fluid flow architecture.
FIG. 89 is a detail view of a portion of the block diagram of FIG. 85, illustrating a filtration flow path of the fluid flow architecture.
FIG. 90 is a flowchart illustrating a method for bioprocessing carried out using the bioprocessing apparatus of FIG. 42, according to a disclosed embodiment.
FIG. 91 is a flowchart illustrating a method for bioprocessing carried out using the bioprocessing apparatus of FIG. 42, according to a disclosed embodiment.
FIG. 92 is a flowchart illustrating a method for bioprocessing carried out using the bioprocessing apparatus of FIG. 42, according to a disclosed embodiment.
FIG. 93 is a block diagram illustrating the fluid flow architecture of the bioprocessing apparatus of FIG. 42, according to a disclosed embodiment.
FIG. 94 is a block diagram illustrating the fluid flow architecture of the bioprocessing apparatus of FIG. 42, according to another disclosed embodiment.
FIG. 95 is a block diagram illustrating the fluid flow architecture of the bioprocessing apparatus of FIG. 42, according to yet another disclosed embodiment.
FIG. 96 is a block diagram illustrating the fluid flow architecture of the bioprocessing apparatus of FIG. 42, according to yet another disclosed embodiment.

### DETAILED DESCRIPTION

Reference will be made below in detail to exemplary embodiments, examples of which are illustrated in the accompanying drawings. Wherever possible, the same reference characters used throughout the drawings refer to the same or like parts.

As used herein, the term "flexible" or "collapsible" refers to a structure or material that is pliable, or capable of being bent without breaking, and may also refer to a material that is compressible or expandable. An example of a flexible structure is a bag formed of polyethylene film. The terms "rigid" and "semi-rigid" are used herein interchangeably to describe structures that are "non-collapsible," that is to say structures that do not fold, collapse, or otherwise deform under normal forces to substantially reduce their elongate dimension. Depending on the context, "semi-rigid" can also denote a structure that is more flexible than a "rigid" element, e.g., a bendable tube or conduit, but still one that does not collapse longitudinally under normal conditions and forces.

A "vessel," as the term is used herein, means a flexible bag, a flexible container, a semi-rigid container, a rigid container, or a flexible or semi-rigid tubing, as the case may be. The term "vessel" as used herein is intended to encompass bioreactor vessels having a wall or a portion of a wall that is semi-rigid or rigid, as well as other containers or conduits commonly used in biological or biochemical processing, including, for example, cell culture/purification systems, mixing systems, media/buffer preparation systems, and filtration/purification systems, e.g., chromatography and tangential flow filter systems, and their associated flow paths. As used herein, the term "bag" means a flexible or semi-rigid container or vessel used, for example, as containment device for various fluids and/or media.

As used herein, "fluidly coupled" or "fluid communication" means that the components of the system are capable of receiving or transferring fluid between the components. The term fluid includes gases, liquids, or combinations thereof. As used herein, "electrical communication" or "electrically coupled" means that certain components are configured to communicate with one another through direct or indirect signaling by way of direct or indirect electrical connections. As used herein, "operatively coupled" refers to a connection, which may be direct or indirect. The connection is not necessarily a mechanical attachment.

As used herein, the term "tray" refers to any object, capable of at least temporarily supporting a plurality of components. The tray may be made of a variety of suitable materials. For example, the tray may be made of cost-effective materials suitable for sterilization and single-use disposable products.

As used herein, the term "functionally-closed system" refers to a plurality of components that make up a closed fluid path that may have inlet and outlet ports, to add or remove fluid or air from the system, without compromising the integrity of the closed fluid path (e.g. to maintain an internally sterile biomedical fluid path), whereby the ports may comprise, for example, filters or membranes at each port to maintain the sterile integrity when fluids or air is added or removed from the system. The components, depending on a given embodiment, may comprise but are not limited to, one or more conduits, valves (e.g. multiport diverters), vessels, receptacles, and ports.

Embodiments disclosed herein provide systems and methods for manufacturing cellular immunotherapies from a biological sample (e.g., blood, tissue, etc.). In an embodiment, a method includes genetically modifying a population of cells in a bioreactor vessel to produce a population of genetically modified cells, and expanding the population of genetically modified cells within the bioreactor vessel to generate a number of genetically modified cells sufficient for one or more doses for use in a cell therapy treatment without removing the population of genetically modified cells from the bioreactor vessel. In certain embodiments, one or more of the methods may include activating cells in the same bioreactor vessel using magnetic or non-magnetic beads to produce a population of activated cells prior to genetically modifying the cells, and washing the genetically modified cells on the bioreactor vessel to remove unwanted materials.

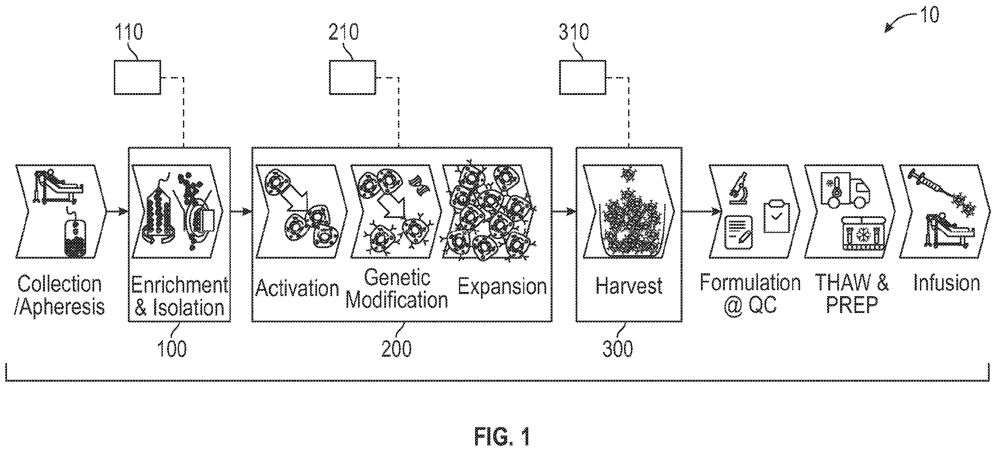

With reference to FIG. 1, a schematic illustration of a bioprocessing system 10 according to a disclosed embodiment. is illustrated. The bioprocessing system 10 is configured for use in the manufacture of cellular immunotherapies (e.g., autologous cellular immunotherapies), where, for example, human blood, fluid, tissue, or cell sample is collected, and a cellular therapy is generated from or based on the collected sample. One type of cellular immunotherapy that can be manufactured using the bioprocessing system 10 is chimeric antigen receptor (CAR) T cell therapy, although other cellular therapies may also be produced using the disclosed system or aspects thereof without departing from the broader aspects of the disclosure. As illustrated in FIG. 1, the manufacture of a CAR T cell therapy generally begins with collection of a patient's blood and separation of the lymphocytes through apheresis. Collection/apheresis may take place in a clinical setting, and the apheresis product is then sent to a laboratory or manufacturing facility for production of CAR T-cells. In particular, once the apheresis product is received for processing, a desired cell population (e.g., white blood cells) is enriched for or separated from the collected blood for manufacturing the cellular therapy, and target cells of interest are isolated from the initial cell mixture. The target cells of interest are then activated, genetically modified to specifically target and destroy tumor cells, and expanded to achieve a desired cell density. After expansion, the cells are harvested, and a dose is formulated. The formulation is often then cryopreserved and delivered to a clinical setting for thawing, preparation and, finally, infusion into the patient.

With further reference to FIG. 1, the bioprocessing system 10 disclosed therein includes a plurality of distinct modules or subsystems that are each configured to carry out a particular subset of manufacturing steps in a substantially automated, functionally-closed and scalable manner. In particular, the bioprocessing system 10 includes a first module 100 configured to carry out the steps of enrichment and isolation, a second module 200 configured to carry out the steps of activation, genetic modification and expansion, and a third module 300 configured to carry out the step of harvesting the expanded cell population. In an embodiment, each module 100, 200, 300 may be communicatively coupled to a dedicated controller (e.g., first controller 110, second controller 210, and third controller 310, respectively). The controllers 110, 210 and 310 are configured to provide substantially automated control over the manufacturing processes within each module. While the first module 100, second module 200 and third module 300 are illustrated as including dedicated controllers for controlling the operation of each module, it is contemplated that a master control unit may be utilized to provide global control over the three modules. Each module 100, 200, 300 is designed to work in concert with the other modules to form a single, coherent bioprocessing system 10, as discussed in detail below.

By automating the processes within each module, product consistency from each module can be increased and costs associated with extensive manual manipulations reduced. In addition, as discussed in detail hereinafter, each module 100, 200, 300 is substantially functionally closed, which helps ensure patient safety by decreasing the risk of outside contamination, ensures regulatory compliance, and helps avoid the costs associated with open systems. Moreover, each module 100, 200, 300 is scalable, to support both development at low patient numbers and commercial manufacturing at high patient numbers.

With further reference to FIG. 1, the particular manner in which the process steps are compartmentalized in distinct modules that each provide for closed and automated bioprocessing allows for efficient utilization of capital equipment to an extent heretofore not seen in the art. As will be appreciated, the step of expanding the cell population to achieve a desired cell density prior to harvest and formulation is typically the most time-consuming step in the manufacturing process, while the enrichment and isolation steps, and the harvesting and formulation steps, as well as activation and genetic modification steps, are much less time consuming. Accordingly, attempts to automate the entire cell therapy manufacturing process, in addition to being logistically challenging, can exacerbate bottlenecks in the process that hamper workflow and decrease manufacturing efficiency. In particular, in a fully-automated process, while the steps of enrichment, isolation, activation and genetic modification of cells can take place rather quickly, expansion of the genetically modified cells takes place very slowly. Accordingly, manufacture of a cellular therapy from a first sample (e.g., the blood of a first patient) would progress quickly until the expansion step, which requires a substantial amount of time to achieve a desired cell density for harvest. With a fully automated system, the entire process/system would be monopolized by the expansion equipment performing expansion of the cells from the first sample, and processing of a second sample could not begin until the entire system was freed up for use. In this respect, with a fully-automated bioprocessing system, the entire system is essentially offline and unavailable for processing of a second sample until the entire cell therapy manufacturing process, from enrichment to harvest/formulation is completed on the first sample.

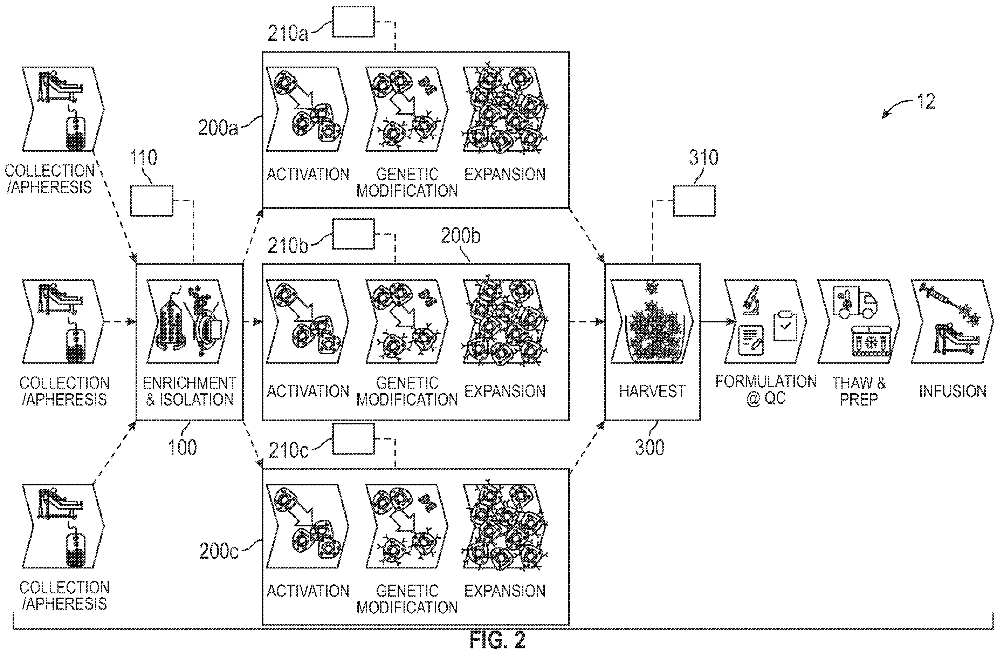

Embodiments of the present disclosure, however, allow for parallel processing of more than one sample (from the same or different patients) to provide for more efficient utilization of capital resources. This advantage is a direct result of the particular manner in which the process steps are separated into the three modules 100, 200, 300, as alluded to above. With particular reference to FIG. 2, in an embodiment, a single first module 100 and/or a single third module 300 can be utilized in conjunction with multiple second modules, e.g., second modules 200*a,* 200*b,* 200*c,* in a bioprocessing system 12, to provide for parallel and asynchronous processing of multiple samples from the same or different patients. For example, a first apheresis product from a first patient may be enriched and isolated using the first module 100 to produce a first population of isolated target cells, and the first population of target cells may then be transferred to one of the second modules, e.g., module 200*a,* for activation, genetic modification and expansion under control of controller 210*a*. Once the first population of target cells is transferred out of the first module 100, the first module is again available for use to process a second apheresis product from, for example, a second patient. A second population of target cells produced in the first module 100 from the sample taken from the second patient can then be transferred to another second module, e.g., second module 200*b*, for activation, genetic modification and expansion under control of controller 201*b.*

Similarly, after the second population of target cells is transferred out of the first module 100, the first module is again available for use to process a third apheresis product from, for example, a third patient. A third target population of cells produced in the first module 100 from the sample taken from the third patient can then be transferred to another second module, e.g., second module 200c, for activation, genetic modification and expansion under control of controller 201c. In this respect, expansion of, for example, CAR-T cells for a first patient can occur simultaneously with the expansion of CAR-T cells for a second patient, a third patient, etc.

This approach also allows the post processing to occur asynchronously as needed. In other words, patient cells may not all grow at the same time. The cultures may reach the final density at different times, but the multiple second modules 200 are not linked, and the third module 300 can be used as needed. With the present disclosure, while samples can be processed in parallel, they do not have to be done in batches.

Harvesting of the expanded populations of cells from the second modules 200*a,* 200*b* and 200*c* can likewise be accomplished using a single third module 300 when each expanded populations of cells are ready for harvest.

Accordingly, by separating the steps of activation, genetic modification and expansion, which is the most time consuming, and which share certain operational requirements and/or require similar culture conditions, into a stand-alone, automated and functionally-closed module, the other system equipment that is utilized for enrichment, isolation, harvest and formulation is not tied up or offline while expansion of one population of cells is carried out. As a result, the manufacture of multiple cell therapies may be carried out simultaneously, maximizing equipment and floorspace usage and increasing overall process and facility efficiency. It is envisioned that additional second modules may be added to the bioprocessing system 10 to provide for the parallel processing of any number of cell populations, as desired. Accordingly, the bioprocessing system of the present disclosure allows for plug-and-play like functionality, which enables a manufacturing facility to scale up or scale down with ease.

In an embodiment, the first module 100 may be any system or device capable of producing, from an apheresis product taken from a patient, a target population of enriched and isolated cells for use in a biological process, such as the manufacture of immunotherapies and regenerative medicines. The third module 300 may be any system or device capable of harvesting and/or formulating CAR-T cells or other modified cells produced by the second module 200 for infusion into a patient, for use in cellular immunotherapies or regenerative medicine. In certain embodiments, the first module 100 and the third module 300 are similarly or identically configured, such that the first module 100 may first be utilized for enrichment and isolation of cells (which are then transferred to the second module 200 for activation, transduction and expansion (and in some embodiments, harvesting)), and then also used at the end of the process for cell harvesting and/or formulation. In this respect, in some embodiments, the same equipment can be utilized for the front-end cell enrichment and isolation steps, as well as the back-end harvesting and/or formulation steps.

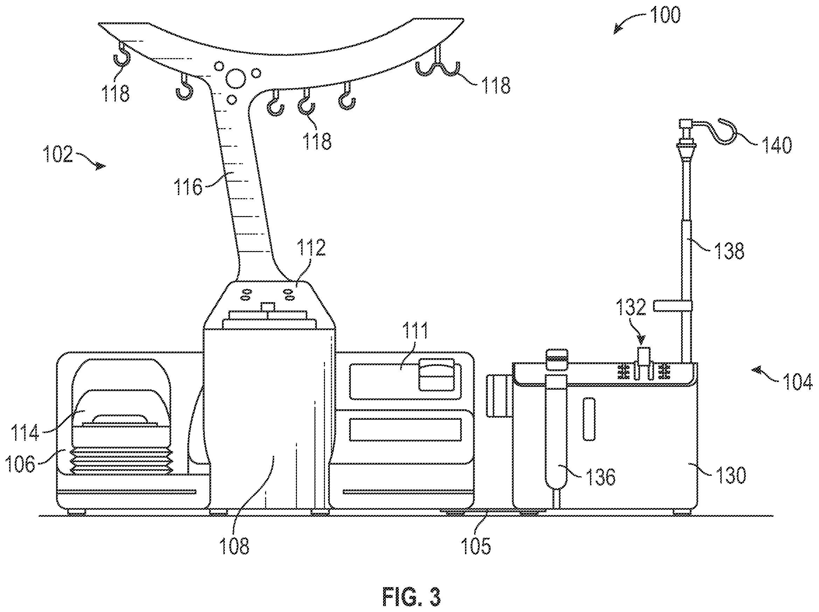

Referring now to FIG. 3, an exemplary configuration of the first module 100 (and in some embodiments, the third module 300) is illustrated. In an embodiment the first module 100 (and third module 300) includes a processing apparatus 102 and an isolation module 104. In an embodiment, the processing apparatus 102 and the isolation module 104 may be mechanically interconnected with one another, such as via a bracket 105 mounted to the respective bottoms of the devices. The processing apparatus 102 may be, for example, a Sefia S-2000 cell processing instrument, available from Cytiva. In an embodiment the processing apparatus may be configurated the same as, or substantially similar to, apparatus 900 disclosed in WIPO International Publication No. WO 2019/106207. The processing apparatus 102 thus includes a base 106 that houses a centrifugal processing chamber 108, a high dynamic range peristaltic pump assembly 111, a stopcock manifold interface 112, and a heating-cooling-mixing chamber (thermal mixer) 114. As indicated below, the stopcock manifold interface 112 is configured to receive a single-use, disposable kit specifically configured for performing cell concentration, platelet removal and density gradient-based separation, washing, and/or final formulation, and provides a simple and reliable means of interfacing multiple fluid or gas lines together using, for example, luer fittings. Within the base 106 is a motor drivingly connected to a plurality (in this case, four) of output shafts that are operable to move stopcocks of the disposable kit between open and closed positions under control of a controller. In an embodiment, the pump assembly 111 is rated to provide flow rates as low as about 3 mL/min and as high as about 150 mL/min). The processing apparatus 102 may further include a suite of sensors configured to monitor various parameters of the apparatus 102, itself, and of various fluids handled by the apparatus 102.

As further shown in FIG. 3, the processing apparatus 102 of the first and/or third module 100, 300 also includes a generally T-shaped hanger assembly 116 that extends from the base 106 and includes a plurality of hooks 118 for suspending a plurality of bags for containing or receiving fluids used in the bioprocessing operations carried out by the first or third modules. In an embodiment, there may be six hooks. Each hook may include an integrated weight sensor or load cell (not shown) for monitoring the weight of each vessel/bag. In an embodiment, the bags may be, for example, a sample source bag, a process bag, an isolation buffer bag, a washing bag, one or more storage bags, a post-isolation waste bag, a washing waste bag, a media bag, a release bag, and/or a collection bag, depending on the particular processes being carried out. The processing apparatus 102 also includes a centralized control unit, e.g., controller 110, for carrying out one or more bioprocessing operations according to algorithm(s) stored in memory in an automated or semi-automated manner.

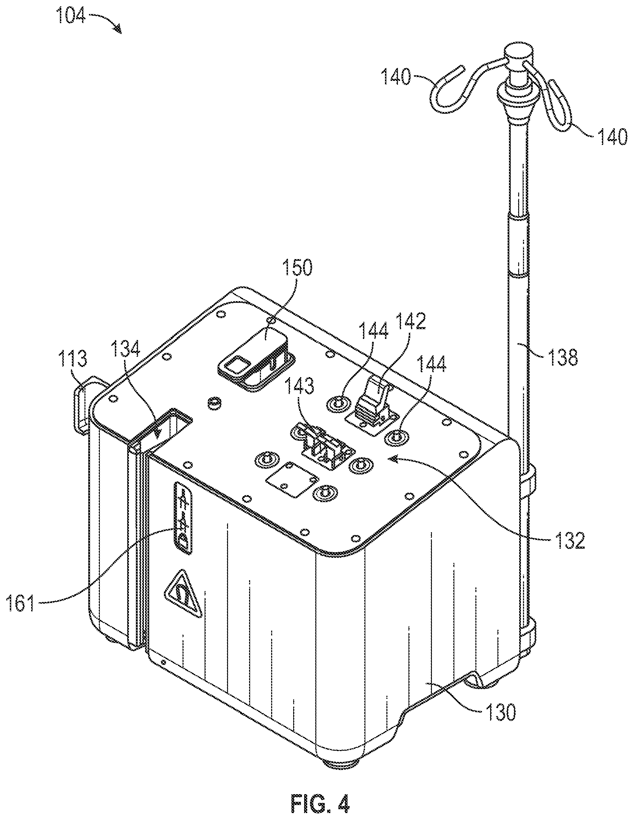

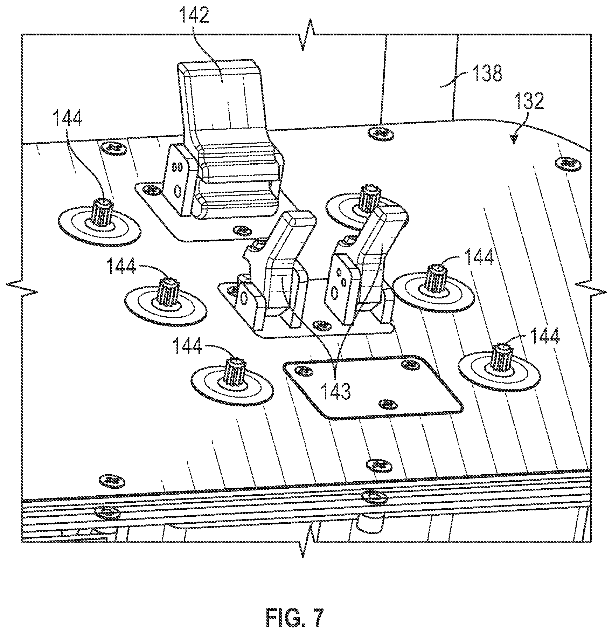

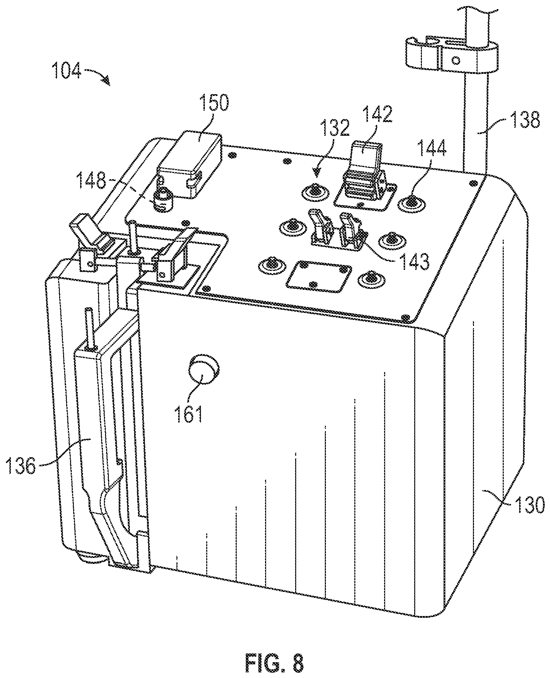

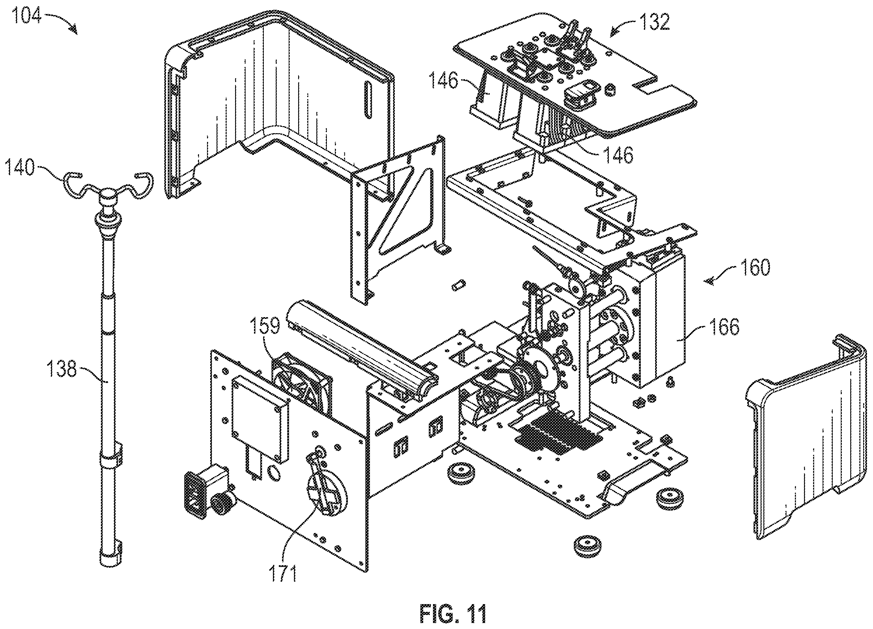

With further reference to FIG. 3, and with more specific reference to FIGS. 4-11, the isolation module 104 of the first and/or third module 100, 300 is shown. The isolation module 104 includes a base/housing 130, a stopcock manifold interface 132 located on the base 130 and configured to receive a stopcock manifold of a single use, disposable cell processing kit, and a vertical aperture or slot 134 in the base configured to removably receive a magnetic cell isolation holder 136 of the isolation module 104, the purpose of which will be described hereinafter. The isolation module 104 further includes a support pole 138 having one or more hooks 140 or pegs for suspension of fluid bags or vessels therefrom. In an embodiment, the hook 140 may be configured with or connected to a load cell for real-time mass monitoring of the contents of the bag. While FIG. 3, illustrates the isolation module 104 as having two hooks 140, more or fewer than two hooks may be present. For example, in an embodiment, the isolation module 104 has four hooks 140. In an embodiment, the inner surfaces of the housing 130 and/or base structure thereof may be coated or covered with an electrically conductive paint or coating to shield from EMC perturbations, for example. In an embodiment, the housing 130 may be manufactured from plastic, while the base structure that supports the housing may be metallic, although in certain embodiments, both the base structure and housing may be formed from plastic or similar nonconductive material.

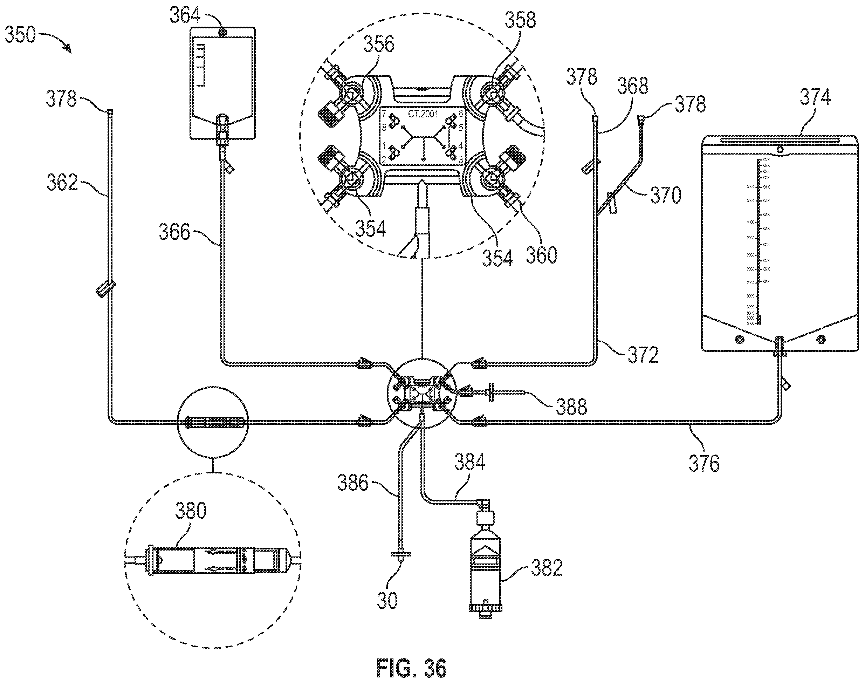

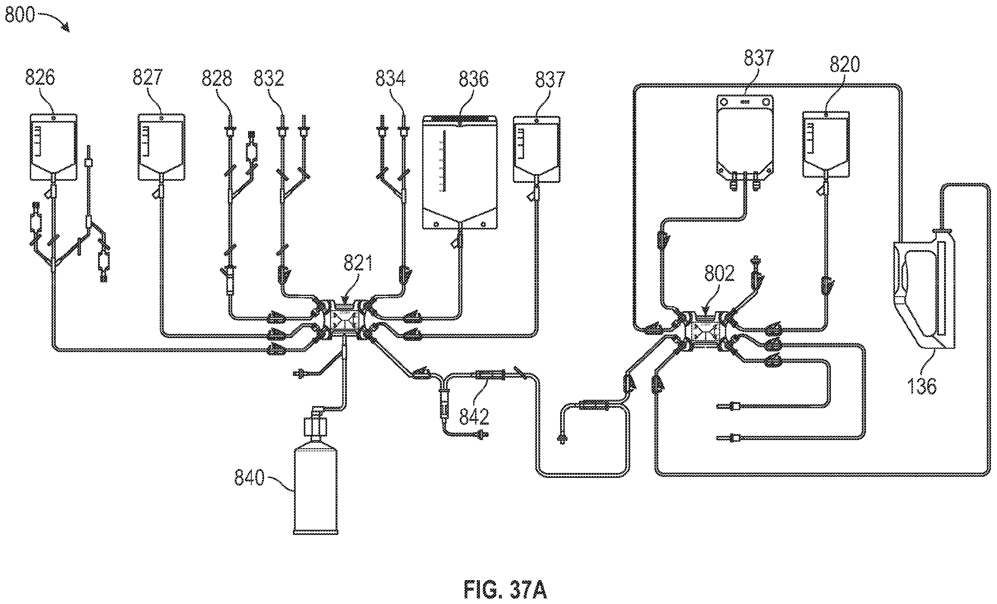

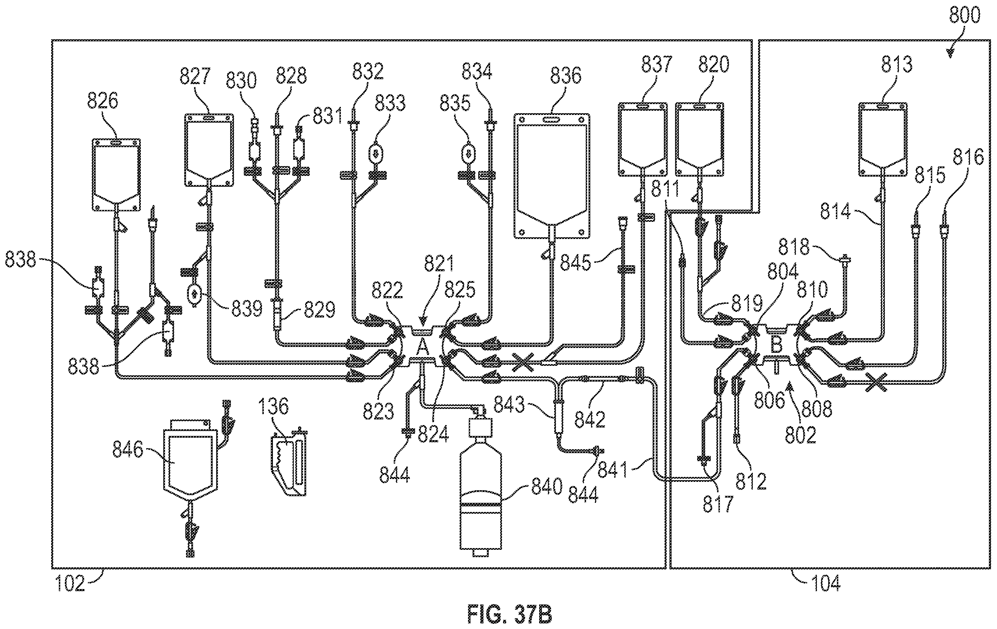

In an embodiment the isolation module 104 includes a drip chamber holder 113 to insert and hold drip chamber of a disposable bioprocessing kit (e.g., for washing, dosing preparation, formulation and/or isolation of cells), as described hereinafter. In an embodiment the drip chamber holder can accommodate different diameters or shapes to be compatible with different versions of the disposable kit drip chamber, e.g., the drip chamber 380 of the kit 350 shown in FIG. 36, and/or the drip chamber 829 of kit 800 shown in FIGS. 37A and 37B. In an embodiment, the drip chamber holder may include one or several spring plungers to improve the grip on the drip chamber when inserted.

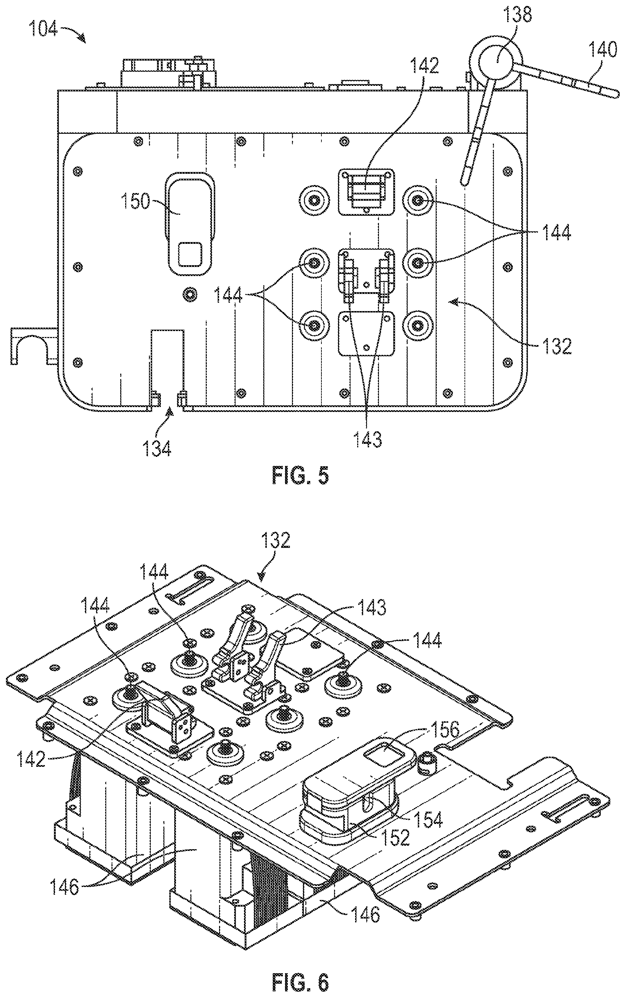

As best shown in FIG. 5-7, the stopcock manifold interface 132 includes one or more latches or clamps 142, 143 that can be selectively deployed to retain a cell processing kit in position on the interface 132, as described hereinafter. The interface 132 further includes an array of stopcock pins or splined output shafts 144 drivingly connected to at least one stopcock motor 146 housed within the base 130. In an embodiment, there are 6 output shafts configured to interface with a respective one stopcock of a 6 stopcock manifold of a disposable cell processing kit, although it is envisioned that more or fewer than 6 stopcock pins may be utilized without departing from the broader aspects of the disclosure, and depending on the particular configuration of the disposable kit. In an embodiment, each output shaft 144 has a dedicated motor 146. The motors 146 are configured to rotate the output shafts 144 to move stopcocks of the disposable cell processing kit received on the interface 132 between open and closed positions under control of a controller, as described hereinafter. Notably, the 6 stopcock interface shown in FIG. 4 is capable of interfacing with a 4 or 6 stopcock manifold of a disposable cell processing kit.

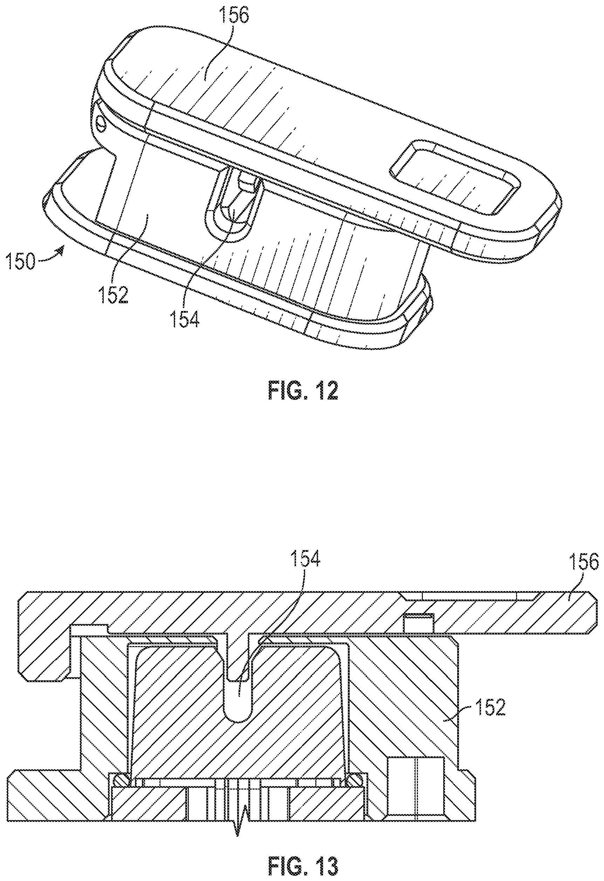

With specific reference to FIGS. 4-6, 12 and 13, the isolation module 104 may include a plurality of sensors for monitoring various operational parameters of the isolation module 104, as well as parameters or conditions of flow lines and/or fluid therein. For example, in an embodiment, the isolation module 104 may include a line pressure sensor assembly 148 having an interface beneath which a pressure sensor is positioned, and bubble sensor/detector assembly 150, both forming part of the stopcock manifold interface 132 for monitoring a pressure and the presence of bubbles, respectively, within one of more of the fluid flow lines connected to the module 104. As shown therein, the bubble sensor assembly 150 includes a housing 152 having an upward facing channel 154 or passage therein, with which the bubble sensor is associated, and a cover 156 pivotally connected to the housing 152. The channel 154 is sized and dimensioned to receive a length of tubing, and the cover 156 is selectively moveable between an open and closed position with respect to the housing 152 to capture and retain the length of tubing within the channel 154. In an embodiment, the housing 152 and cover 156 are formed from a material having poor electrical conductivity, such as anodized aluminum or plastic, such that any electrical current present will pass through the housing 130 of the isolation module 104 and not the bubble sensor 150 (which could adversely affect operation thereof). In an embodiment, the housing 130 includes an air inlet having an integrated filter through which air may be drawn into the housing 130 for cooling the internal components thereof during operation.

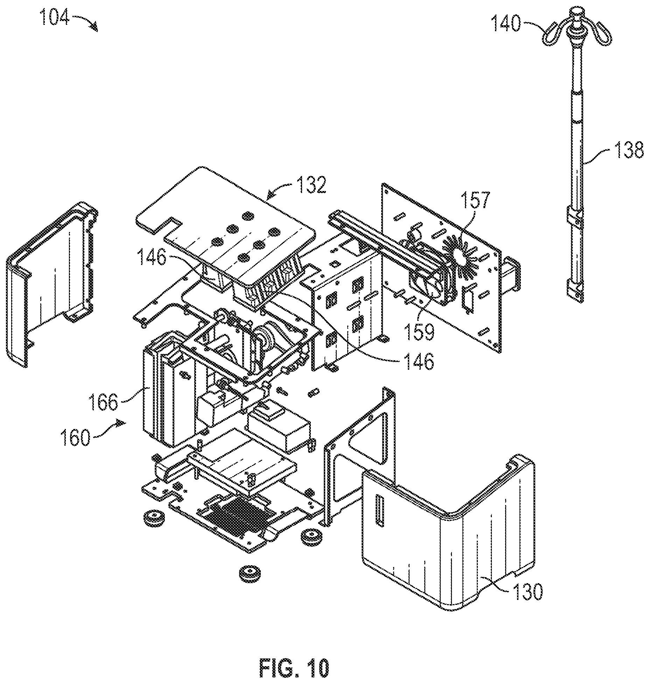

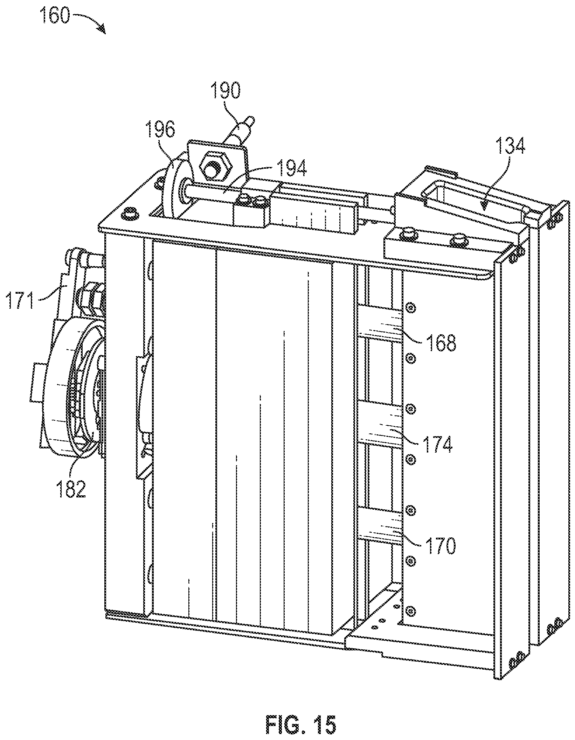

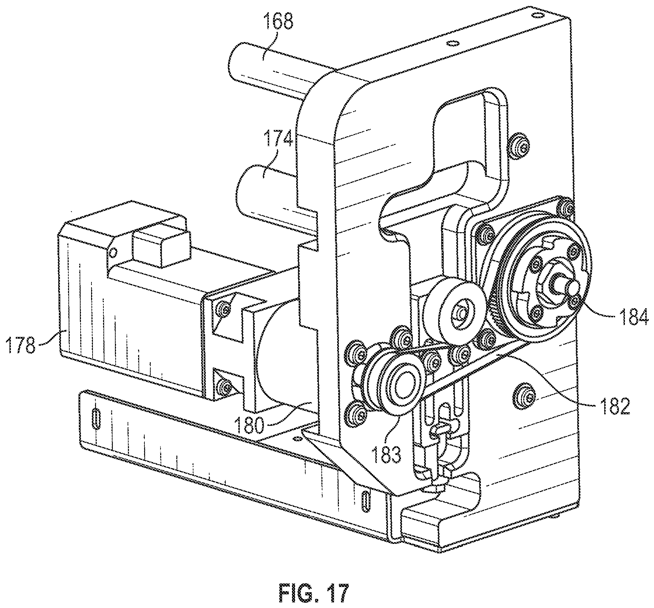

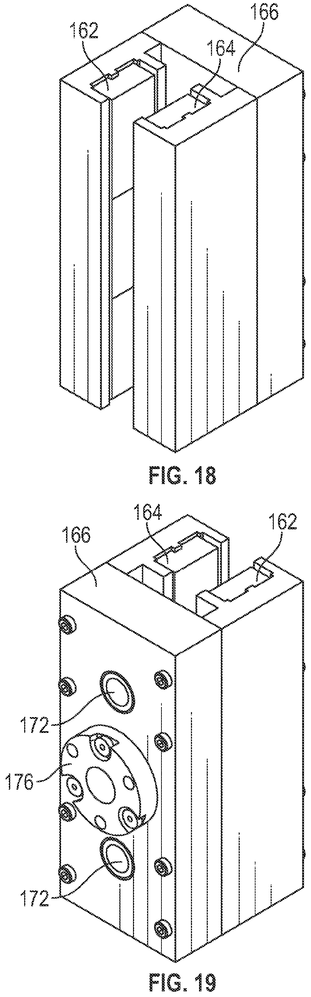

With reference to FIGS. 10, 11 and 14-19, the isolation module 104 additionally includes a magnetic field generator assembly 160 housed within the base housing 130. In an embodiment, the magnetic field generator assembly 160 includes a pair of opposed permanent magnets 162, 164 (having a space therebetween) mounted to a moveable carriage 166. While a pair of magnets 162, 164 are illustrated, it is contemplated that for a same final height, each illustrated magnet 162 and 164 can either be made of a single long magnet, or of a stack of several shorter magnets without departing from the broader aspects of the disclosure. As described in detail below, the carriage 166 is moveable between an extended position, where the magnets 162, 164 are positioned on opposing sides of the slot 134 for generating a magnetic field within the slot 134, and a retracted position where the magnets 162, 164 are moved rearwardly of the slot 134 so as to not generate a magnetic field (or to only generate a small or negligible magnetic field) within the slot 134. The carriage 166 is slidably connected to, and supported by, upper and lower shafts 168, 170 received by bushings or bearings 172 in the carriage assembly 166, and is operatively connected to a lead screw 174 that is received through a central bushing 176 of the carriage 166. The lead screw 174 is rotatable to slidably move the carriage 166 between its extended position and its retracted position, as disclosed in detail hereinafter.

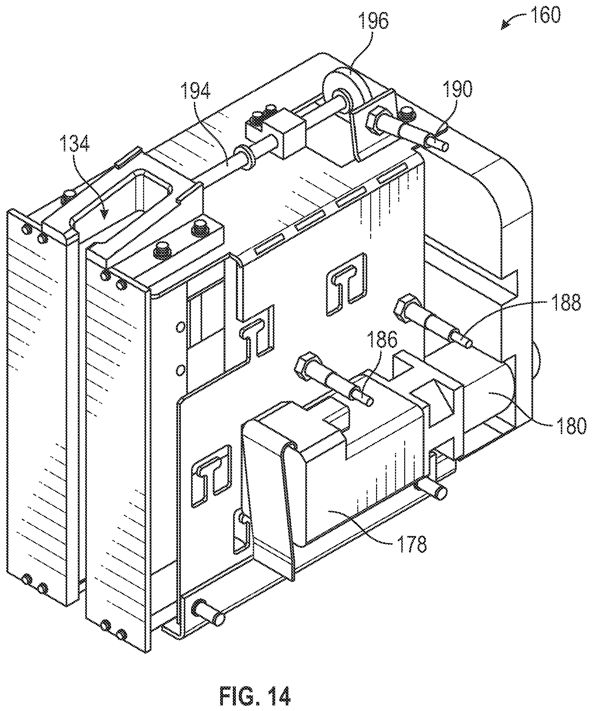

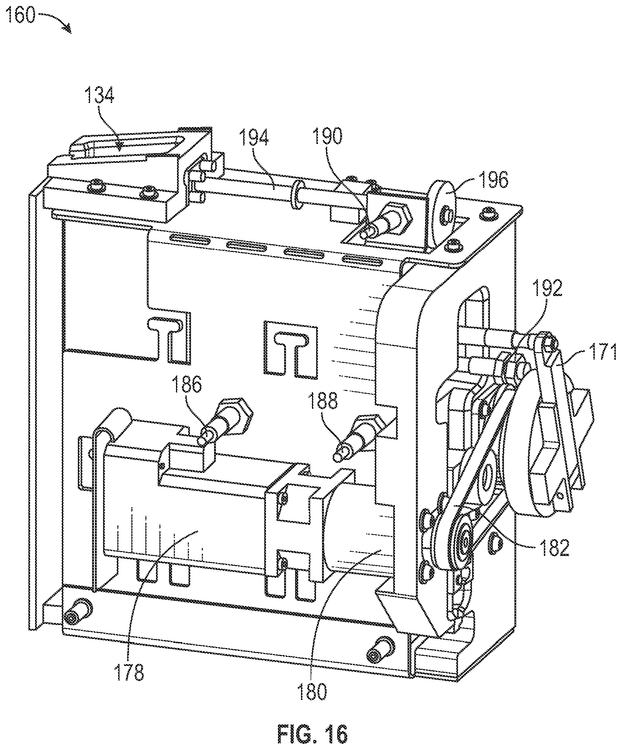

As best shown in FIGS. 14 and 16, the magnetic field generator assembly 160 includes a motor 178 that is drivingly connected to the lead screw 174 via a gearbox 180 and belt 182 (which links a timing pulley 183 of the gearbox 180 to a timing pulley 184 of the lead screw 174). The motor 178 is thus configured to rotate the lead screw 174 to extend or retract the carriage 166 and magnets 162, 164. As also shown therein, the magnetic field generator assembly 160 further includes an array of sensors that are utilized to detect movement of the carriage 166, the position of the carriage 166 (and thus magnets 162, 164), and the presence of the magnetic isolation holder 136 within the slot 134. For example, the magnetic field generator assembly 160 includes first and second sensors 186, 188 that are utilized to detect and confirm movement of the carriage 166, a third sensor 190 that is utilized to detect the presence of the magnetic cell isolation holder 136 within the slot 134 in the housing, and a crank sensor 192. In an embodiment, the sensors 186, 188, 190, 192 are inductive proximity sensors, although other types of sensors known in the art may also be utilized without departing from broader aspects of the disclosure. In connection with detection of the magnetic cell isolation holder 136, the magnetic field generator assembly 160 also includes a slidable locking pin 194 having a flange 196 (or washer) that is configured to engage a rear face of the carriage 166 adjacent to a top edge thereof. The locking pin 194 also includes a coil spring 198 that is configured to bias the locking pin 194 towards the front of the isolation module 104 (i.e., towards the slot 134), the purpose of which is hereinafter described.

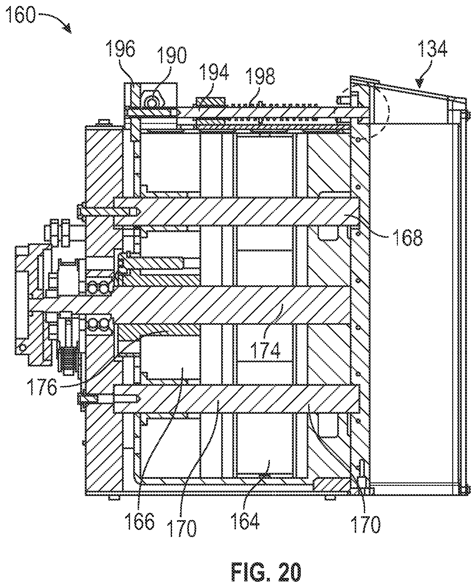

Turning now to FIGS. 20-25, operation of the magnetic field generator assembly 160 and positioning of the carriage 166 thereof will now be described. With reference to FIG. 20, detection of the presence or absence of the magnetic cell isolation holder 136 within the slot 134, is carried out using the second sensor 188 and the third sensor 190. At the beginning of the process, the carriage 166 is in its retracted position where it is sensed by sensor 188 and sensor 186. In this position, the locking pin 194 is in its retracted position (as it is prevented sliding forward due to engagement of the flange 196 with the rear of the carriage 166). In particular, the carriage 166 holds the locking pin 194 in its retracted position against the bias of the spring 198, freeing the slot 134 for the magnetic cell isolation holder 136 to be inserted.

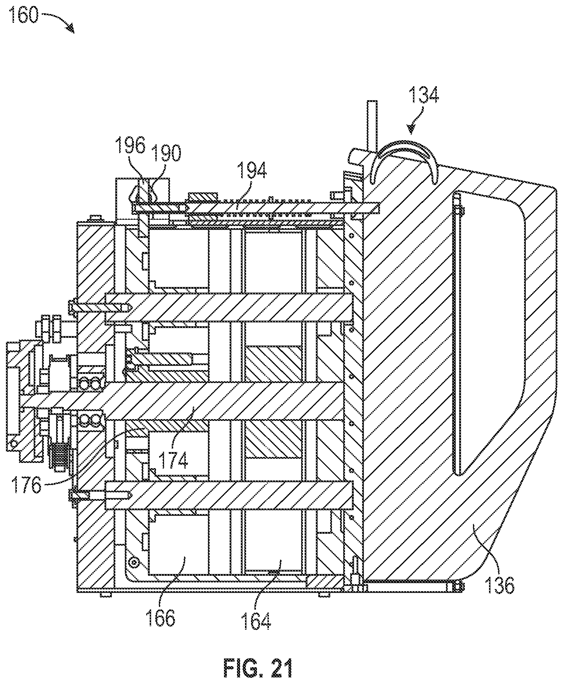

As shown in FIG. 21, the magnet cell isolation holder 136 is now inserted. When the motor 178 rotates the lead screw 168, the carriage 166 is driven forward towards the slot 134 and isolation holder 136. The locking pin 194 and flange 196 thereof move forward along with the carriage 166 due to the bias of the spring 198 which urges the locking pin 196 forward. As shown therein, as the flange 196 or disc of the locking pin 194 is urged forwardly, it is detected by sensor 190 (and the first and second sensors also continue to detect the presence of the carriage 166). In this position, the distal end of the locking pin 194 contacts the magnetic cell isolation holder 136 engaged with the slot 134.

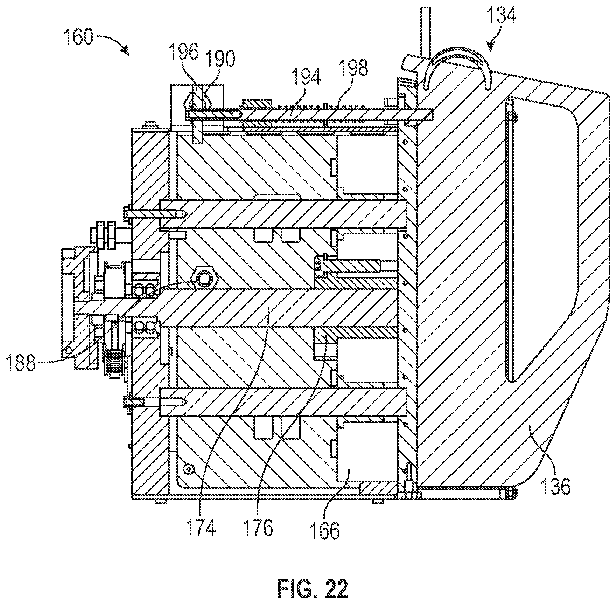

As shown in FIG. 22, the carriage 166 is then driven to its forward most position by the motor 178 and lead screw 168 until the opposed magnets 162, 164 are aligned with opposing sides of the slot with slot 134. As shown therein, the locking pin 194 is prevented from moving further forward due to its seated engagement with the inserted isolation holder 136 (i.e., it contacts a seat in the isolation holder 136), and so the flange 196 continues to be detected by the sensor 190. In this position, however, the carriage 166 is forward and clear of the sensors 186, 188, and so the presence of the carriage 166 is not detected by these sensors. As will be appreciated, therefore, detection of the flange 196 by the sensor 190 indicates that the isolation holder 136 is received in the slot 134, and the absence of a detection of the carriage 166 by either the first sensor 186 or second sensor 188 indicates that the carriage 166 and magnets 162, 164 thereof are in the forward, working position where a magnetic field can be generated within the slot 134.

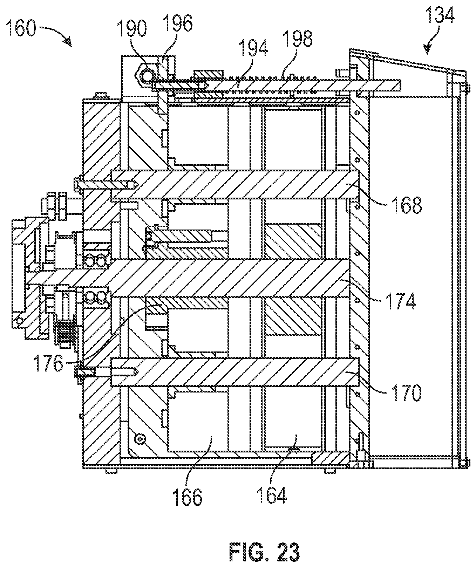

Turning now to FIG. 23, when the carriage 166 and magnets 162, 164 are moved forward towards the extended position, but the isolation holder 136 is not received within the slot 134 in the housing 130, the locking pin 194 is free to move forward with the carriage 166 under the bias of the spring 198 (i.e., its forward motion does not contact the seat in the isolation holder 136). The locking pin 136 thus slides forward until its end bottoms out and reaches the end of its motion range. In this position, the distal end of the locking pin 194 obstructs the slot 134, inhibiting insertion of the isolation holder 136, and the flange 196 is forward of the sensor 190 so that it is not detected thereby, indicating that the isolation holder 136 is not present. As shown in FIG. 23, the absence of the isolation holder 136 can be detected even when the carriage is not in its forward most position (i.e., sensor 186 detects the presence of the carriage 166, which sensor 188 does not).

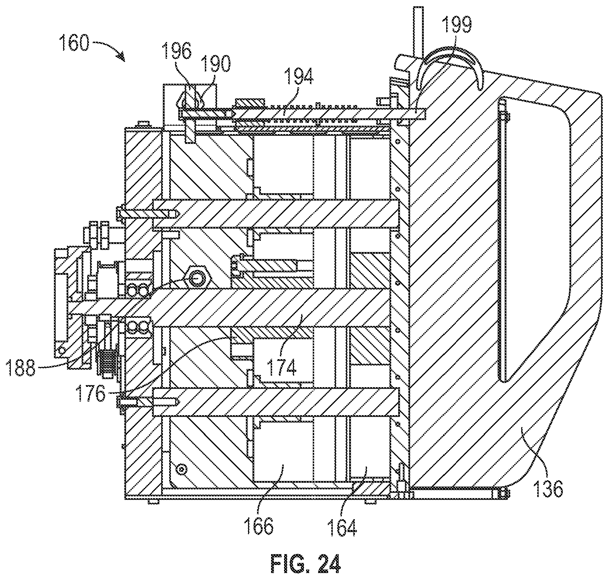

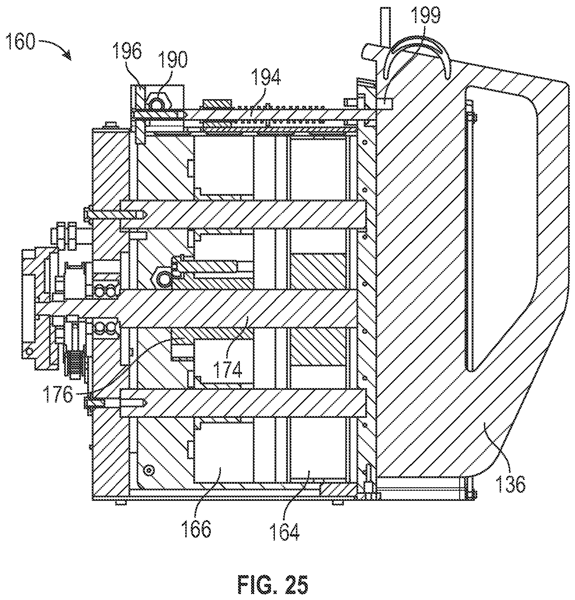

With reference to FIG. 24, and as indicated above, if the isolation holder 136 is inserted correctly within the slot 134, the locking pin 194 moves forward along with the carriage 166 until it is seated within a recess or seat within the isolation holder 136. In this position, the locking pin 194 prevents removal of the isolation holder 136 from the slot 134. As shown in FIG. 25, however, if the isolation holder 136 is not properly positioned within the slot 134, the seat 199 within the isolation holder 136 is misaligned with the distal end of the locking pin 194. This misalignment prevents the locking pin 194 from entering the recess/seat 199. Accordingly, the locking pin 194 is prevented from traveling far enough forward for the flange 196 to be aligned with the sensor 190. In this position, the sensor 188 does not detect the carriage 166, indicating that the carriage 166 has been moved forward. As the sensor 190 does not detect the flange 196 of the locking pin 194 in this position of the carriage 166, however, it indicates that the isolation holder 136 is not properly received within the slot 134. Once in the position shown in FIG. 24, with the locking pin 194 holding the isolation holder 136 in place within the slot 134, and with the magnets 162, 164 aligned with the opposing sides of the slot 134, a magnetic field may be generated to capture bead-bound cells within the isolation holder 136 in a manner known in the art and discussed in more detail hereinafter.

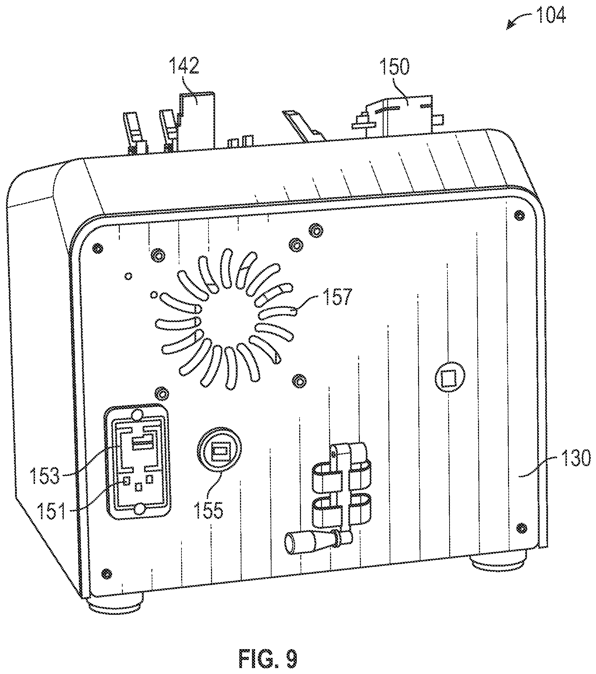

Referring once again to FIGS. 9, 11, 15 and 16, in an embodiment the isolation module 104 further includes a manual crank 171 that is operatively connected to the linear screw 174. The crank 171 is operable to manually move the carriage 166 and magnets 162, 164 to the retracted position in an emergency or in the event of a loss of electrical power. The crank 171 has a pivotable handle that remains closed when not in use, but which can be folded out when needed. A ball detent screwed into the handle maintains the handle in the closed position. In an embodiment, the crank 171 may include a pawl and ratchet mechanism such that when the crank is closed, a pin separates a pawl from the ratchet due to the force of a spring. In this position, the crank is free to rotate, as the pawl and ratchet are not in contact. To open the crank 171, an operator must unfold the crank arm lever, which presses the pawl against the rachet by the force of the spring and by retreat of the pin. Since the pawl and ratchet are now in contact, the crank 171 can be rotated to engage the lead screw 174 in a clockwise direction, which corresponds to rearward movement of the carriage 166. As alluded to above, sensor 192 is provided to detect an open position of the crank 171. In an embodiment, the crank 171 is configured so that rotation in the opposite direction is prevented, so that manual, forward movement of the carriage 166 is not possible (thereby preventing inadvertent or accidental activation of the magnetic circuit).

Referring back to FIG. 9, the rear of the isolation module 104 may include a connector 151 for connection to a supply of electrical power for powering the isolation module 104, a switch 153 for turning the isolation module 104 on and off, a communications connector 155 for communicatively connecting the isolation module 104 to a controller, and a plurality of openings 157 through which an internal fan 159 may exhaust air to keep the isolation module 104 at an optimal working temperature. In an embodiment, the communications connector 155 may be a USB connector, although other wired or wireless communication means known in the art may also be utilized. In an embodiment, the isolation module 104 is communicatively coupled to the processing apparatus 102 and controlled by the controller 110 thereof. In this respect, all the information obtained by the various sensors of the isolation module 104 (e.g., regarding the position and status of the magnetic field generator assembly 160, cell processing kit on the interface 132, and/or parameters of the fluid passing through various flow lines, etc.) is communicated to the controller of the processing apparatus 102 where it is analyzed and then utilized by the controller to control operation of the isolation module 104, generate alerts and the like. Accordingly, the isolation module 104 need not be outfitted with a separate processor and memory, which increases cost and complexity.

In connection with operation of the isolation module 104, the front of the isolation module 104, as shown in FIG. 4 may include an array of indicator lights for conveying to an operator a status/position of the magnetic field generator assembly. For example, the indicator lights 161 may include a green indicator light indicating the carriage 166 and magnets 162, 164 are in their retracted position, a blinking yellow indicator light indicating that the carriage 166 is moving, and a solid yellow indicator light indicating that the carriage and magnets are in their extended positions for magnetic retention of bead-bound cells passing through the isolation holder 136. In another embodiment, the front of the isolation module 104 may instead, or in addition, include pictograms including, for example, a first pictogram that, when illuminated, indicates that the isolation holder 136 can be inserted into the slot 134 in the isolation module 104, a second pictogram that, when illuminated indicates that the application/process has been successfully completed and the magnetic circuit is off (and that the isolation holder 136 can be removed from the isolation module 104), and a third pictogram in the form of a lock or other icon that, when illuminated, indicates that the isolation holder 136 is properly locked in place.

As discussed in detail hereinafter, the isolation module 104 provides for an expanded array of bioprocessing functions to be carried out in a single, easy to use system. These processes may include, for example, enrichment and magnetic isolation of cells, washing, as well as dosing preparation including cell harvesting and final formulation. As is known in the art, magnetic particle-based cell selection or isolation involves isolating certain cells from a cell mixture via targeted binding of cell surface molecules to antibodies or ligands of magnetic particles (e.g., beads). Once bound, the cells coupled to the magnetic particles are able to be separated from the unbound population of cells. For example, the cell mixture including the bound and unbound cells may be passed through a separation column positioned within a magnetic field generator (e.g., magnetic field generator assembly 160 of the isolation module 104) that captures the magnetic particles and, therefore, the associated bound cells. The unbound cells pass through the column without being captured. In embodiments, the magnetic cell isolation holder 136 and/or the isolation module 104 may be specifically configured for cell enrichment and isolation using various magnetic isolation bead types (including, for example, Miltenyi beads, Dynabeads and StemCell EasySep beads). Exemplary configurations of the isolation holder 136 are provided below.

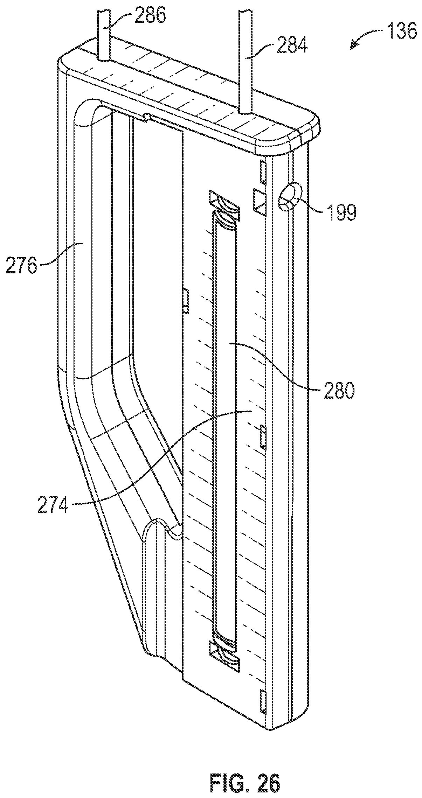

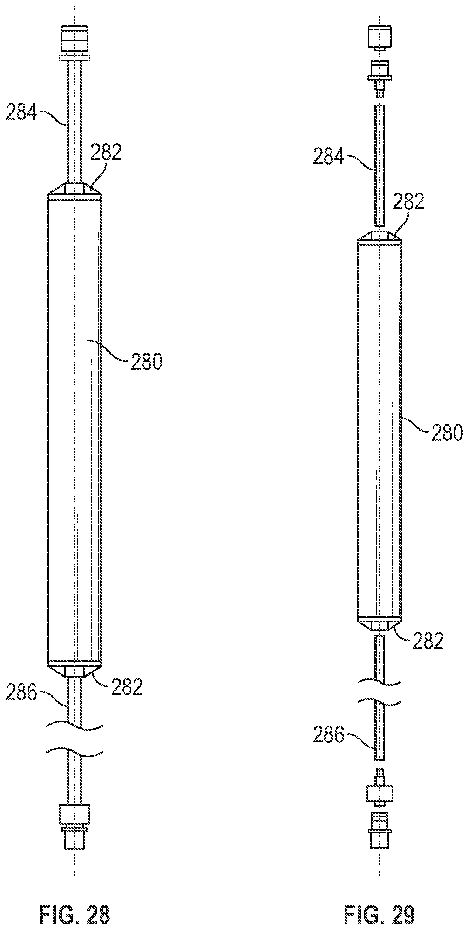

As indicated above, the magnetic cell isolation holder 136 may be designed and configured for use with a variety of different magnetic bead sizes and types. For example, in an embodiment, the magnetic cell isolation holder 136 may be specifically designed for use with nano-sized magnetic beads such as, for example, Miltenyi beads. With reference to FIGS. 26-29, in an embodiment, the magnetic cell isolation holder 136 of the isolation module 104 may include a body portion 274 that that receives and retains therein a vertical column 280, and a handle 276 connected to the body portion 174 allowing for easy manipulation by a user (e.g., to install and remove the isolation holder 136 from the slot 134 in the isolation module 104). In an embodiment, the body portion 274 and handle portion 276 are integral, and may be formed from molded halves 277, 278 that sandwich the column 280. As best shown in FIG. 26, the recess 199 for receiving the locking pin 194 of the magnetic field generator assembly 160 is formed in a forward face of the body portion 274. With reference to FIGS. 28 and 29, in one exemplary embodiment, the column shell may be a stock extruded aluminum shell, anodized and further machined as necessary for dimensional tolerances. The column 280 has a pair of identical endcaps 282 connected to the column 280 at opposed ends thereof, each including a female glue port for interfacing directly to lengths of PVC tubing 284, 286, an O-ring (for forming a fluidic seal) and a heat-sealed-on piece of mesh (useful in process to retain the beads before the encapsulant is added). In an embodiment, the column is filled with a magnetic retention element which, in an embodiment, in array of ferromagnetic spheres or beads, and an encapsulant. The encapsulant utilized may be a biocompatible epoxy. To apply the encapsulant, the column is filled with ferromagnetic spheres or beads, the encapsulant is added to fully wet the beads, and then the excess encapsulant is removed via centrifugation and the encapsulant is cured.

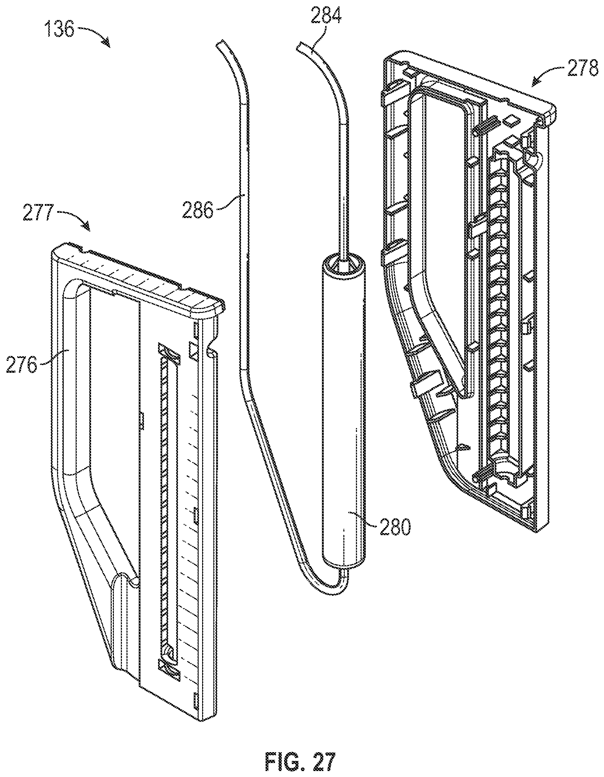

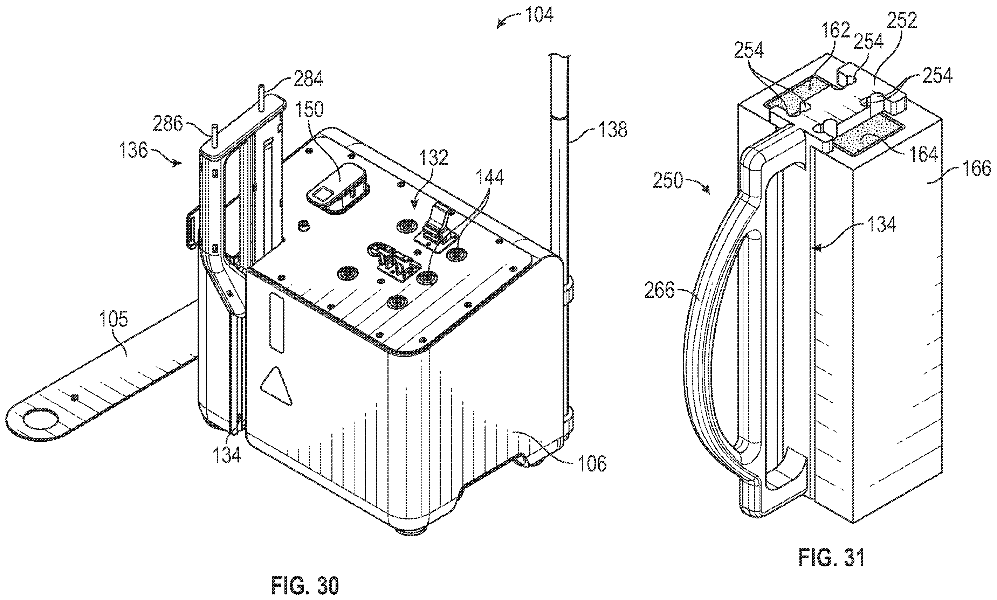

As best illustrated in FIGS. 26 and 27, the first length of PVC tubing 284 enters the upper end of the column 280 vertically from above, and forms an inlet flow passage for bead-bound cells into the column 280 when the isolation holder 136 is received within the slot 134 of the isolation module 104. The second length of PVC tubing 286 exits the lower end of the column 280, providing for an exit pathway of fluid from the column 280 while the bead-bound cells are retained within the column, as known in the art. The second length of PVC tubing 286, in an embodiment, is routed through the handle 276 where is exits the isolation holder 136 vertically. While note illustrated, the first and second lengths of tubing 284, 286 include connectors for integration of the column 280 with a flow pathway of a magnetic cell isolation kit or cassette received on the interface 132 of the isolation module, as described hereinafter. FIG. 30 illustrates installation of the magnetic cell isolation holder 136 into the slot 134 in the isolation module (i.e., by sliding it into the slot 134 from above). Removal of the magnetic cell isolation holder 136 is carried out by sliding the holder upwardly within the slot 134.

Turning now to FIGS. 31-35, various other exemplary configurations of the magnetic cell isolation holder 136 for use with the isolation module 104 are illustrated. As disclosed above, certain magnetic cell isolation techniques may incorporate nano-sized particles (e.g., beads of about 50 nm or less in diameter) while other techniques may use larger particles (e.g., beads of about 2 µm or more in diameter). For example, smaller particles may be desirable because smaller particle sizes may avoid receptor activation on the target cells. Further, downstream steps may skip particle removal, because the nano-sized particles may have little effect on downstream processing or cell function. However, the smaller nano-sized magnetic particles may be separated using a magnetic cell isolation procedure that involves the use of a magnetic field gradient intensifier to amplify an applied magnetic field gradient. In contrast, larger particles have a higher magnetic moment. Thus, isolation of certain larger particles may not involve a magnetic field gradient intensifier. However, with larger particles, the isolation column within the magnetic field generator may reach capacity before a sufficient number of bead-bound cells are captured. In particular, the bead-bound cells accumulate on the inside of the passage to a point where additional bead-bound cells to be captured are no longer in a region with a high enough gradient to overcome the viscous drag forces urging them through the passage. Accordingly, using larger particles may necessitate multiple capture and elution cycles to obtain a desired yield, which adds complexity to magnetic particle-based cell isolation techniques. As disclosed hereinafter, certain configurations of the magnetic cell isolation holder may obviate the need for multiple cycles to be carried out, namely, by passing the cell mixture through a non-linear flow path within the magnetic field, circulating the cell mixture through or within the magnetic field, and/or making multiple passes through the magnetic field. As used herein, non-linear means not in a straight line through the magnetic field. For example, the flow path may be spirally or helically shaped, or may have one or more curves or contours within the magnetic field.

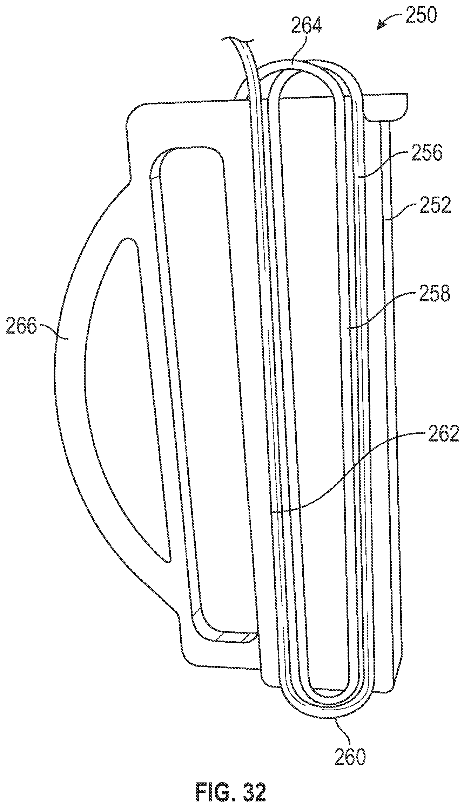

As shown in FIGS. 31-33, a magnetic cell isolation holder 250 for use with the isolation module 104 is shown coupled to the magnetic field generator assembly 160 (i.e., received between the magnets 162, 164 of the magnetic field generator assembly 160). As alluded to above, the magnetic field generator 160 is configured to generate a magnetic field within the slot 134 (also referred to herein as receiving area 134). The receiving area 134 and the magnetic field have a major axis (defining the longitudinal extent of the magnetic field) and a minor axis, whereby the gradient and field strength is substantially constant along lines parallel to the major axis (and may decrease at the extremities of the major axis). Looking at a cross-sectional area perpendicular to the major axis (see, e.g., FIG 32), the gradient is substantially constant along lines running into and out of the page.

The magnetic cell isolation holder 250 is configured for removable coupling with the magnetic field generator 160, e.g., received within receiving area/slot 134 of the magnetic field generator 160. As illustrated in FIGS. 31 and 32, in an embodiment, the magnetic cell isolation holder 250 includes a body 252, which may be formed from any suitable nonmagnetic material configured to accommodate the cell isolation and be coupled to the magnetic field generator 160. In an embodiment, the body 252 is generally rectangular in shape, having a longitudinal extent along the major axis of the magnetic field (in the vertical direction in FIG. 31) that is greater than a width or thickness of the body, and includes a plurality of channels or races 254 that extend along the body 252 for receiving and retaining a tube 256. The tube 256, for its part, may be configured to, under the magnetic field, retain cells bound to magnetic particles and permit unbound cells to pass through, as is known in the art. For example, the magnetic particles may be Dynabeads or SCT beads, although other magnetic particle/bead types may be utilized without departing from the broader aspects of the disclosure.

The tube 256 is routed along and/or through the body 252 via the races 254 and defines a flow passage for the flow of a fluid (e.g., a cell mixture). The races 254 and tube 256 are positioned such that the flow passage defined by the tube 256 is positioned within the magnetic field when the magnetic cell isolation holder 250 is coupled to the magnetic field generator 160 (i.e., received within slot 134). Moreover, the races 254, and thus the tube 356 and the flow passage defined thereby, are configured such that, when the magnetic cell isolation holder 250 is coupled to the magnetic field generator 160, a direction of fluid flow within the flow passage (i.e., through the tube) at a first location within the magnetic field is different than a direction of fluid flow within the flow passage at a second location within the magnetic field, as discussed hereinafter.

For example, as illustrated in FIGS. 31-33, in an embodiment, the body 252 may include eight generally vertical channels or races 254, two adjacent to each longitudinal corner of the body 252. The tube 256 is routed through the races 254 in a manner so as to form a plurality of serially and fluidly interconnected loops. Where the body contains eight races 254, four serial loops are formed by routing the tube 256 through the races 254. It is contemplated that the body 252 may be formed with more or fewer than eight races so as to accommodate more or fewer than four loops, as desired. The positioning of the tube 256 into loops provides for increased residence time of the cell mixture within the magnetic field (the total time the cell mixture passes through the high gradient magnetic field) without reducing the flow velocity (by reducing flow rate or enlarging the cross-sectional area of the flow passage), thus enabling better capture of the bead-bound cells as compared to a single, vertical pass through the magnetic field (at the same flow velocity and same longitudinal length of the magnetic field generator).

FIG. 32 more clearly shows the tube loops of the magnetic cell isolation holder 250. As shown therein, the plurality of loops of tube each include a first portion 258 extending substantially linearly along the longitudinal extent of the body 252, wherein the longitudinal extent of the body 252 aligns with the major axis of the magnets 162, 164 and magnetic field that has substantially constant gradients along lines parallel to the major axis of the magnet and thus parallel (and ultimately colinear) to the tubing pathways running along the longitudinal axis of the holder. The loops of tube further include a second portion 260 extending from the first portion 258 and forming a first return bend, a third portion 262 extending substantially linearly and parallel to the first portion 258, and a fourth portion 264 extending from the second portion and forming a second return bend. As indicated above, the loops are serially connected to one another such that the fourth portion/bend 264 of a first loop of the plurality of loops is fluidly connected to the first portion 258 of a second loop to provide fluid interconnection of the first loop with the second loop for circulation of the fluid between the loops within the magnetic field. Fluid in one of the loops, for example, first passes through the generally vertical first portion 258, enters the first return bend 260, and then passes into the generally vertical third portion 262. The fluid then passes into the fourth portion/bend 264 and into a next downstream loop. In an embodiment, the first return bend 260 and the second return bend 264 are bends of approximately 180 degrees such that fluid flow in the first portion 258 and the third portion 262 are generally parallel but in opposite directions, respectively. While not illustrated, the tube 256 has an inlet end for connection to, and for receiving a cell mixture from, a source (e.g., process bag), as well as an outlet end for selective connection to a waste bag and/or collection bag. The plurality of loops of tube 256 are intermediate the inlet end and outlet end. In some embodiments, the flow passage may have an even number of lengths (e.g., vertical portions) such that the inlet and outlet are on the same end of the magnetic cell isolation holder 250. In other embodiments, the flow passage may have an odd number of vertical portions so that the inlet and outlet are located on opposing ends of the magnetic cell isolation holder 250.

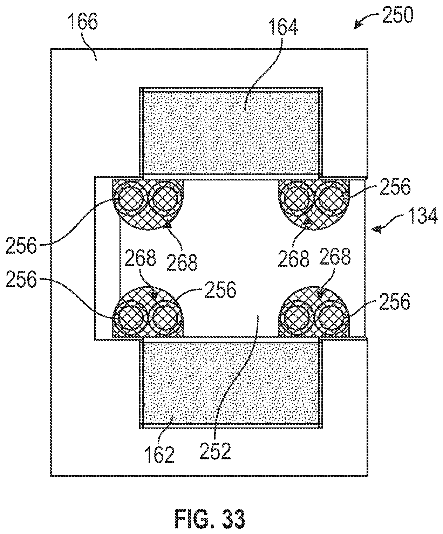

In an embodiment, the magnetic cell isolation holder 250 may include a handle 266 or finger grip portion enabling a user to grasp the magnetic cell isolation holder 250 to position it within, or remove it from, the receiving area 134. As best illustrated in FIG. 31, the magnetic cell isolation holder 250 is inserted between the magnetic field plates 162, 164 of the magnetic field generator 160. For example, the position of races 254, and thus the longitudinal passes 258, 262 of tube 256 within the magnetic field generator 160 may cover the location in the magnetic field with the highest magnetic field strength. In another example, the position of races 254, and thus the vertical passes 258, 262 of tube 256 within the magnetic field generator 160 may cover the location in the magnetic field with the highest magnetic field gradient, while meeting a magnetic field strength requirement of the magnetic particles. FIG. 33 illustrates the position of the vertical passes of tube 256 within the magnetic field when the magnetic cell isolation holder 250 is received in the slot 134 between the magnets 162, 164. As illustrated therein, the body 252 of the magnetic cell isolation holder 250, and the location of the races 254, as well as the magnets 162, 164, are configured and dimensioned such that the vertical passes of tube 256 are positioned within the high gradient regions 268 of the magnetic field generator 160 when the magnetic cell isolation holder 302 is coupled to the magnetic field generator 350.

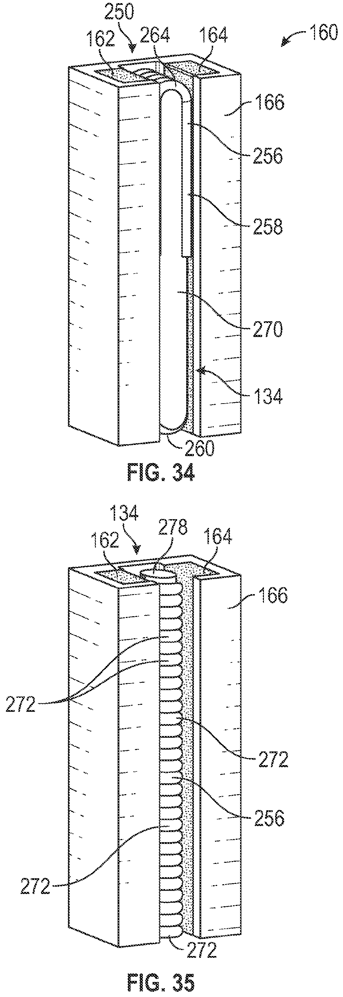

Turning now to FIG. 34, in an embodiment, the magnetic cell isolation holder 300 may include a ferromagnetic core 270 that extends substantially the entire length of the magnetic field, and which is encircled by the tube 256. In an embodiment, the ferromagnetic core 270 may be an integral part of the body 252 of the isolation holder 250, or it may be an additional component. The use of a ferromagnetic core 270 allows higher gradients to be produced over a longer length as compared to systems without a ferromagnetic core. In particular, the ferromagnetic core creates additional parallel high gradient regions along the longitudinal axis of magnetic plates, thereby allowing for a longer length of tubing to be routed within the same magnet field volume as compared to not having the ferromagnetic core. In an embodiment, the ferromagnetic core 270 may be formed from a variety of ferromagnetic materials such as, for example, iron.

FIG. 35 is a simplified illustration of another configuration for the magnetic cell isolation holder according to another disclosed embodiment. As illustrated therein, rather than the tube 256 being formed into a plurality of longitudinal loops, the tube 256 is wound or wrapped in a substantially spiral or helical configuration. As shown, the plurality of loops 272 extend in a direction substantially perpendicular to the longitudinal direction, such that a flow through each loop 272 is generally perpendicular to the longitudinal direction within the magnetic field (e.g., horizontal, rather than vertical). Similar to the configuration of the tube shown in FIGS. 31-31, the plurality of loops 272 within the magnetic field provide a longer length of travel for a fluid within the flow passage of the tube 256 as compared to a single column that extends linearly through the magnetic field. In an embodiment, the horizontal or spiral loops 272 of tube 256 may encircle a ferromagnetic core 278.

While FIGS. 31-35 illustrate the tube 306 arranged in substantially vertically- and horizontally-extending loops (i.e., parallel to or perpendicular to the longitudinal direction/major axis of the magnetic plates and receiving area), respectively, it is contemplated that the tube may be arranged in any configuration that provides a flow passage of increased length/distance within the magnetic field generated by the magnetic field generator as compared to a single, linear passage through the magnetic field. This may be accomplished through the use of multiple loops of any orientation/direction (so that the cell mixture makes multiple passes through the magnetic field), and/or through the use of a single or multiple non-linear passes through the magnetic field (e.g., the tubing has curved or arcuate portions within the magnetic field).

In an embodiment, the tubing may be arranged to form a plurality of loops wherein the tubing loops around the outside of the receiving area 134 (i.e., outside of the magnetic field) so that all flows within the magnetic field area are all running in the same direction (e.g., from top to bottom or bottom to top). Moreover, it is contemplated that all of the tube in the magnetic field may run in the same direction, and the system may include a manifold at the top and a manifold at the bottom to allow for parallel flow.

Still further, it is contemplated that the magnetic isolation holder may be configured with a flow passage/tube that diverts into multiple passages through the magnetic field, and which reconverge. Moreover, in an embodiment, the body 252 of the magnetic cell isolation holder 250 may be configured as a fluidic device having an integral flow passage(s) (i.e., without necessitating a separate tube 256). In particular, it is contemplated that the flow passage and/or ferromagnetic core may be manufactured entirely out of metal. This would allow one to take further advantage of the higher gradient regions of the magnetic field. In yet another embodiment, it is contemplated that the flow passages may be injection molded into an insert. It is contemplated that one could insert mold with a metal framework to add more gradient regions. Similarly, it is contemplated that one could additively manufacture/print the flow passages out of a suitable nonferrous material (e.g., plastic or the like).

While it has been hereinbefore disclosed that the magnetic field generator may be comprised of two opposed magnetic plates that form permanent magnets, the disclosure is not so limited in this regard. In particular, it is contemplated that the magnetic field generator may be an electromagnet that generates a field that is substantially similar to a field created by permanent magnets.

As disclosed above, the processing apparatus 102 and isolation module 104 are intended to be used in combination with one another to carry out, in an automated or semi-automated manner, a variety of functions, protocols and/or workflows associated with isolation, harvesting and final formulation of cellular products. In particular, the processing apparatus 102 and isolation module 104 can be controlled so as to carry out various operations associated with these processes in sequence, with minimal or no human intervention, according to a set of instructions executed by the controller (e.g., controller 110 or 310) of the processing apparatus 102 and stored in memory of the processing apparatus 102. In an embodiment, the processing apparatus 102 is configured and operable to carry out any of the protocols set forth in WIPO International Publication No. WO 2019/106207 and carried out by the apparatus 900 disclosed therein, with the isolation module 104 providing additional functionality and possible workflows, as described below. Indeed, the processing apparatus 102 and isolation module, as disclosed above and described in more detail below, provide for fluid management, centrifugation, temperature control, cell isolation, cell washing, cell concentration, cell preparation and formulation, for example.

In connection with the processing apparatus 102 and insolation module 104, disclosed embodiments provide a variety of single use, disposable/consumable kits that are designed for use with the processing apparatus 102 and/or isolation module 104 for assisting with the carrying out processes and/or workflows associated with isolation, harvesting and final formulation of cellular products. With reference to FIG. 36, a disposable washing kit 350 for use with the processing apparatus 102 is shown. The washing kit 350 is a single-use, disposable kit that is utilized in conjunction with the apparatus 102 to wash and concentrate a fresh or thawed input product after an optional, temperature-controlled initial dilution. As shown in FIG. 36, the kit 350 includes a cassette or manifold 352 having four stopcocks 354, 356, 358, 360, an input line 362 fluidly connected to stopcock 354, a final product/collection container or bag 364 fluidly connected to stopcock 356 via line 366, a washing solution line 368 and a resuspension solution line 370 fluidly connected to stopcock 358 via line 372, and a waste container or bag 374 fluidly connected to the stopcock 360 via line 376. As shown therein, the input line 362, washing solution line 368 and resuspension solution line 370 may be outfitted with end caps 378 the preserve the sterility of the lines during transport and storage, and which can be removed or cut off just prior to use so that bags containing a fresh/thawed input product, wash solution, and resuspension solution, respectively, can be connected to the lines via any means known in the art, such as sterile welding or the like.

As further shown in FIG. 36, the input line 362 includes an in-line drip chamber 380 having an integral filter. The kit 350 further includes a separation chamber 382 fluidly connected to the cassette 352 via flow line 384, and a branch tubing tail 386 fluidly connected to the cassette 352 via line 384. The tubing tail 386 and a second tubing tail connected to the stopcock 358 of the cassette are outfitted with hydrophobic filters 388. In an embodiment, the hydrophobic filters are 2 micrometer hydrophobic filters. In an embodiment, the kit 250 may be sterilized by means known in the art such as, for example, ethylene oxide sterilization, and sealed in a blister pack for transport to an end user and for storage.

In use, the manifold 352 is installed on the stopcock manifold interface 112 of the processing apparatus 102 such that respective motor output shafts of the interface 112 are engaged with a respective one of the four stopcocks 354, 356, 358, 360 for controlling a position of the stopcocks. The separation chamber 382 is received within the centrifugal processing chamber 108. The input line 362 is connected to a bag containing an input product to be washed, the washing solution line 372 connected to a bag containing washing solution, and the resuspension solution line 370 connected to a bag containing resuspension solution, and these bags, along with the waste bag 374 and collection bag 364, are suspended from the hooks 118 of the processing apparatus 102. The washing and optional concentration processes are then carried out according to a preprogrammed set of instructions stored in memory and utilized by controller 110 of processing apparatus 102. In an embodiment, a washing process utilizing processing apparatus 102 and disposable kit 350 includes, optionally, initial dilution of the input product, concentrating the input product (so as to reduce its volume), washing the input product, and then resuspending the input product and collecting the resuspended input product in the collection bag.

During the initial dilution step, parameters such as temperature, post-dilution mixing, dilution mix time and dilution mix rate can be input or retrieved from memory, and the washing solution from the bag connected to washing solution line 372 is utilized to perform the initial dilution. During the concentration/volume reduction step, parameters such as priming (or not) of the flow line(s) with input product, input bag rinsing during the last volume reduction cycle, input bag rinsing volume, and input bag manual mixing (during the middle of the input bag rinsing step) can be selected and/or entered, and/or enabled or disabled. Moreover, the number of washing cycles performed during the washing phase can be input and selected. Finally, during the resuspension phase, a prompt instructing the user to switch washing and resuspension clamps after the washing phase can be enabled or disabled, and the volume of the final product at the end of the resuspension phase can be input and/or selected. In an embodiment, the washing process carried out utilizing the processing apparatus 102 and kit 350 can be utilized to wash and concentrate an input product before and/or after activation, transduction and expansion.

In an embodiment, the processing apparatus 102, isolation module 104 and kit 350 allow for accurate, small final product volumes to be achieved during resuspension using an algorithm to control filling of resuspension media into the separation chamber 382, in order to avoid overshooting the volume. A method of resuspending the intermediate volume to achieve a desired final volume is carried out simultaneously with a rinsing of the separation chamber of cellular products, and includes, at a first step, extracting the contents of the separation chamber intermediate volume into the final bag line, at a second step, calculating of the number of rinsing cycles and associated filling volumes required to reach the final volume, at a third step, filling of the separation chamber 382 until 10 mL from the rinsing cycle volume final target is achieved, at a fourth step, incrementally filling of 1 mL volume increments with a 2 second pause between increments until the target rinsing cycle volume is reached, at a fifth step, extracting the rinsing volume toward the final/collection bag, and repeating steps three through five until the number of rinsing cycles is completed and the final volume of the output bag is reached. During extraction of the rinsing volume toward the final bag, air intake during the filling step is accounted and the filling volume of the next rinsing cycle subsequently adjusted to ensure the total final volume is effectively reaching the target value. It is contemplated that the general process steps disclosed above may be modified as desired such that, for example, the initial filling of the separation chamber is carried out to any desired volume, and then the separation chamber is incrementally filled with any desired smaller increment volumes, with a pause of a selected duration taking place between smaller volume increments (i.e., the volumes and pause durations specified above may be modified, as desired).

With reference to FIGS. 37A and 37B, a single-use, disposable magnetic cell isolation kit 800 for use with the processing apparatus 102 and isolation module 104 is shown (FIG. 37B more clearly showing placement of the various components on the processing apparatus 102 and isolation module 104, respectively). The magnetic cell isolation kit 800 and associated protocols enabled by the use of such kit, under control of the controller 110 of the processing apparatus 100, allows for initial dilution, volume reduction, washing, incubation, post-incubation wash, magnetic isolation and final resuspension of a cell population, as disclosed hereinafter. In an embodiment, the magnetic cell isolation kit 800 includes a cassette or manifold 802 having four stopcocks 804, 806, 808, 810. The kit 800 also includes a line 811 fluidly connected to the first stopcock 804 and configured for fluid connection to a fitting on the magnetic cell isolation holder 136, a line 812 fluidly connected to the second stopcock 806 and configured for fluid connection to a second fitting on the magnetic cell isolation holder 136, a collection bag 813 fluidly connected to the fourth stopcock 810 via line 814, a tubing tail 815 fluidly connected to the third stopcock 808 for fluid connection to a resuspension buffer bag (not shown) containing a suspension medium used to resuspend the positive fraction of cells after bead isolation, and a tubing tail 816 fluidly connected to the third stopcock 808 for fluid connection to a bag (not shown) containing a release buffer used to release the cells from the magnetic beads within the magnetic cell isolation holder 136. As further shown in FIGS. 37A and 37B, the magnetic cell isolation kit 800 additionally includes a pair of tubing tails 817, 818 fluidly connected to the second and fourth stopcocks 804, 810, respectively, and outfitted with hydrophobic filters of the type hereinbefore described. The kit 800 also includes a negative fraction bag 820 fluidly connected to the first stopcock 804 via a line 819. As shown in FIG. 37A, the manifold 802 is configured for installation on the manifold interface 132 of the isolation module 104.

With further reference to FIGS. 37A and 37B, the kit 800 further includes a second manifold 821 having four stopcocks 822, 823, 824, 825, and configured to be received on the manifold interface 112 of the processing apparatus 102. The kit 800 additionally includes a final collection/transfer bag 826 fluidly connected to the second stopcock 823, a process bag/incubation bag 827 likewise fluidly connected to the second stopcock 823, a line 828 fluidly connected to the first stopcock 822 and having an in-line drip chamber 829 with a 200 micrometer filter. The line 828 additionally includes a branch line 830 having a tubing tail, and a branch line 831 having a sampling pillow. As illustrated, the kit 800 further includes a line 832 fluidly connected to the first stopcock 822 for connection to a platelet-free buffer bag used for platelet depletion. Line 832 includes a branch line 833 having a filter. The kit 800 also includes line 834 fluidly connected to the fourth stopcock 825, and having a branch line 835 having a filter. The line 834 is configured for fluid connection to a bag containing isolation buffer used to perform washing cycles during bead incubation and optional post-incubation wash cycles for removing excess beads. As illustrated, the kit 800 includes a waste bag 836 fluidly connected to the fourth stopcock 825, and a spare bag 837 (that is not used during the isolation process) fluidly connected to the third stopcock 824. Certain of the lines are outfitted with sampling pillows 838 and/or filters 839, as illustrated. Still further, the kit 800 includes a separation chamber 840 configured to be received in the centrifugal processing chamber 108 of the processing apparatus 102. Line 841 interconnects the manifold 802 on the isolation module 104 with the manifold 821 on the processing apparatus 102 for fluid flow therebetween, and includes a section of peristaltic pump tubing 842 configured to engage the peristaltic pump assembly 111 of the processing apparatus 102, and a drip chamber 843. The kit 800 additionally includes further tubing tails having sterile air filters 844. A line 845 is also fluidly connected to the third stopcock 824 an opposite end of which is configured for fluid connection to the bottom port in a process bag 846, which also forms a part of disposable kit 800. In an embodiment, the kit 800 may be sterilized by means known in the art such as, for example, ethylene oxide sterilization, and sealed in a blister pack for transport to an end user and for storage.

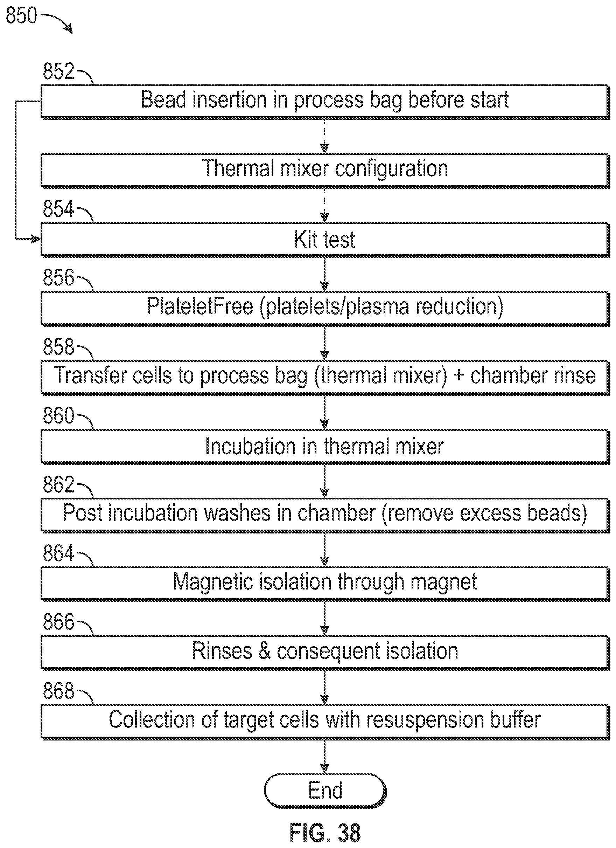

Turning now to FIG. 38 an exemplary protocol 850 for magnetic isolation of cells using magnet cell isolation kit 800, the processing apparatus 102 and the isolation module 104 is illustrated. As indicated above, the magnetic cell isolation kit 800, when utilized in conjunction with the processing apparatus 102 and isolation module 104, allows for initial dilution, volume reduction, washing, incubation, post-incubation wash, magnetic isolation and final resuspension of a cell population. In an embodiment, the protocol 850 illustrated in FIG. 38 performs an optional initial dilution of an apheresis product, concentrates cells, depletes platelets, isolates CD3+ cells (for example) using magnetic beads in the isolation holder 136, and resuspends the cells in a preselected solution for downstream use (e.g., for activation, transduction, and expansion and, ultimately, formulation and dosing preparation). As shown therein, at step 852, magnetic cell isolation beads (e.g., Miltenyi beads, Dynabeads and StemCell EasySep beads) are inserted into the process bag 846 before starting. A kit test may be carried out at step 854. An initial dilution is carried out in a further step. A volume reduction is then carried out at step 856, after which the cells are transferred to the process bag positioned with the thermal mixing chamber 114 of the processing apparatus 102, at step 858. The cells and beads are then incubated in the thermal mixing chamber 114 at step 860, and a post incubation wash is carried out at step 862 to remove excess beads. In an embodiment, the process bag containing the cells and beads is a three dimensional process bag, which provides improved thermal control as a result of the flat bottom surface of the bag (while the upper surface remains flexible). Another advantage of utilizing a three dimensional process bag is improved fluid transfers (e.g., during bag to bag isolation and rinsing steps, the bottom and top surfaces of the bag remain separated and are unlikely to trap or retain cells). During the incubation step, control of the volume for incubation (to a target specific cell density), control of temperature and control of mixing carrier movements is enabled.

After incubation and washing, magnetic isolation of the bead-bound cells is then carried out at stop 864 by inserting the magnetic cell isolation holder 136 into the slot 134 in the isolation module 104 and applying a magnetic field to retain the bead-bound cells within the column or flow passages of the magnetic cell isolation holder, as the case may be. Rinse and isolation is carried out at step 866, after which the target cells are collected with a resuspension buffer, at step 868. In an embodiment, step 868 may include replacement of isolation buffer with media, carrying out elution cycles in 3 steps: (1) detach the major amount of cells from the column and collect volume, (2) perform elution cycles with fresh media and collect volume, and (3) rinse tubing/bag and collect volume. An optional volume reduction step may also be carried out prior to resuspension of the target cells. In an embodiment, air plugs may be utilized to assist in dislodging bead-bound cells from the isolation holder/column, as more specifically disclosed in WIPO International Publication No. WO 2019/106207.

In an embodiment, the processing apparatus 102, isolation module 104, and magnetic cell isolation kit 800 may be utilized to cycle the bead-bound population of cells back and forth through the magnetic field to isolate/capture the bead-bound cells (rather than making a single pass through the magnetic field). For example, a population of bead-bound cells, post incubation, may be pumped (via pump 111) from a first bag, through the magnetic cell isolation holder 136 positioned within the magnetic field within the slot 134 in the isolation module 104, to a second bag. As the cell mixture passes through the magnetic field generated by the magnetic field generator, bead-bound cells are retained/captured in the portion of the fluid pathway that extends through the magnetic cell isolation holder 136 and positioned between the opposed plates of the magnet of the magnetic field generator, in the manner described above, while the unbound population of cells, bead-bound cells that were not captured, and other contents of the cell mixture pass through the magnetic field generator and into a second bag on the other side of the magnetic field generator. The pump 111 of the processing apparatus 102 is then operated in reverse to pump the cell mixture from the second bag, through the magnetic cell isolation holder 136, and back to the first bag. As the cell mixture again passes through the magnetic field generated by the magnetic field generator, additional bead-bound cells are retained/captured in the portion of the fluid pathway of the magnetic cell isolation holder 136 that is positioned within the magnetic field. This process (bag to bag cycling/transfer of the cell mixture) may be repeated until a sufficient number of bead-bound cells are retained in the fluid pathway (or the magnetic cell isolation holder thereof). As will be appreciated, transfer of the cell mixture back and forth between the first bag and the second bag causes the cell mixture to pass through the magnetic field multiple times, increasing the capture efficiency of the system.

The back and forth cycling of the cell mixture between bags on opposing sides of the magnetic field generator as described above essentially has the same effect as increasing the distance of travel of the cell mixture within the magnetic field with multiple loops, passes, or by using a non-linear flowpath through the magnetic field, as disclosed above in connection with FIGS. 31-35. In particular, by cycling the cell mixture back and forth, the total 'distance' that the cell mixture travels within the magnetic field is increased as compared to a single linear pass through the magnetic field. This ensures that bead-bound cells that are not retained on a first pass through the magnetic field can be captured in a subsequent pass before collection. In connection with the above, therefore, it is contemplated that the fluid pathway in the area of the magnetic field generator (e.g., the flow passage within the magnetic cell isolation holder) may take the form of any of the embodiments hereinbefore described. For example, the flow passage within the magnetic field may include a plurality of loops, passes, spirals, contours, turns etc., to increase the residence distance within the magnetic field. In particular, it is contemplated that the flow passage configurations shown in FIGS. 31-35 may be used in connection with the isolation sequence (bag to bag cycling) described above. In other embodiments, a straight path though the magnetic field may be utilized to capture the bead-bound cells.

As also alluded to above, the kit 800 enables and allows for the collection of both the positive and negative fractions resulting from the isolation (in the collection bag 826 and negative fraction bag 820, respectively). In particular, rather than flowing the negative fraction to waste, it can be collected in the negative fraction bag 820 for other potential uses. While the embodiments disclosed above discuss the collection of bead-bound cells using magnetic isolation, the kit 800 additionally allows for negative selection whereby a desired cell population is not labeled with magnetic beads, while other cells are labeled with such beads, such that the undesired cell population is captured in the magnetic cell isolation holder and the desired, unlabeled cell population is allowed to pass through the isolation holder and collected after the bead bound cell population is captured in the magnetic cell isolation holder.

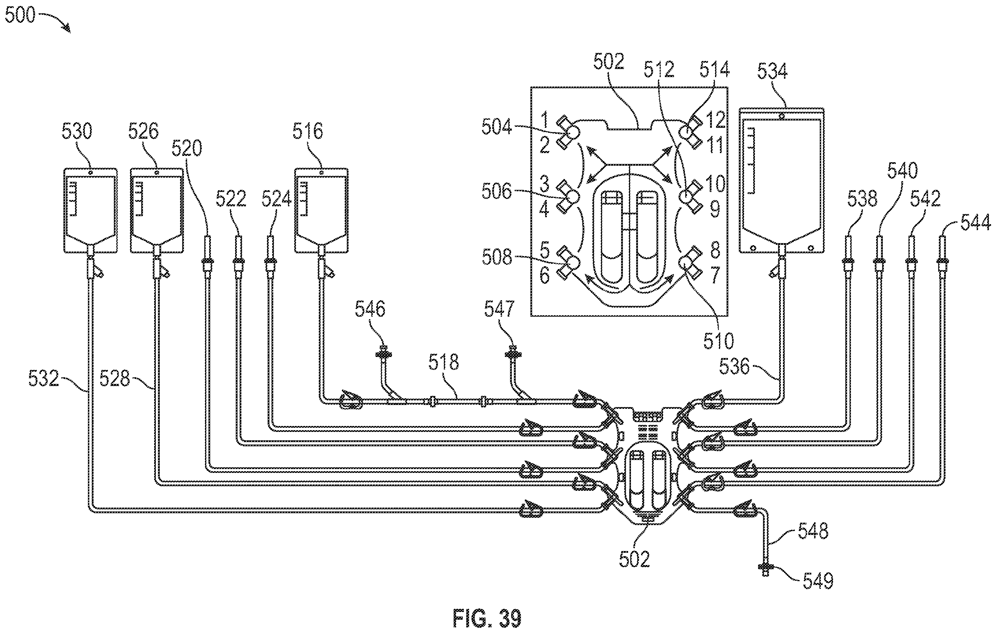

Referring now to FIG. 39, a single-use, disposable dosing preparation kit 500 for use with the processing apparatus 102 and isolation module 104 is shown. The dosing preparation kit 500 and associated protocols enabled by the use of such kit, under control of the controller 110 of the processing apparatus 100, allow for the automation of volume splitting, dilution, mixing, cryopreparation and dosing of cellular products, as described hereinafter. The dosing preparation kit 500 includes a cassette or manifold 502 having six stopcocks 504, 506, 508, 510, 512, 514, a process bag 516 fluidly connected to the stopcock 504 via peristaltic pump tubing 518, a plurality of tubing lines 520, 522, 524 fluidly connected to the cassette 502 for fluid connection (e.g. sterile welding) to one or more media bags (not shown), a final formulation/collection bag 526 fluidly connected to stopcock 508 via line 528, and a bag 530 (for containing initial/intermedia product from which a final dose/formulation is produced) fluidly connected to stopcock 508 via line 532. In an embodiment, the process bag 516 is a three-dimensional process bag. As further shown therein, the kit 500 further includes a waste bag 534 fluidly connected to stopcock 514 via line 536, and a plurality of cryobag connection lines 538, 540, 542, 544 fluidly connected to stopcocks 510, 512, 514 (for connection to a plurality of cryobags utilizing sterile welding or other connection means). Lastly, line 518 is outfitted with a pair of hydrophobic filters 546, 547 on opposing sides of the peristaltic pump tubing section, and the kit 500 further includes an air inlet line 548 fluidly connected to stopcock 510 and having a hydrophobic filter 549. In an embodiment, the hydrophobic filters are 2 micrometer hydrophobic filters. In an embodiment, the kit 500 may be sterilized by means known in the art such as, for example, ethylene oxide sterilization, and sealed in a blister pack for transport to an end user and for storage.

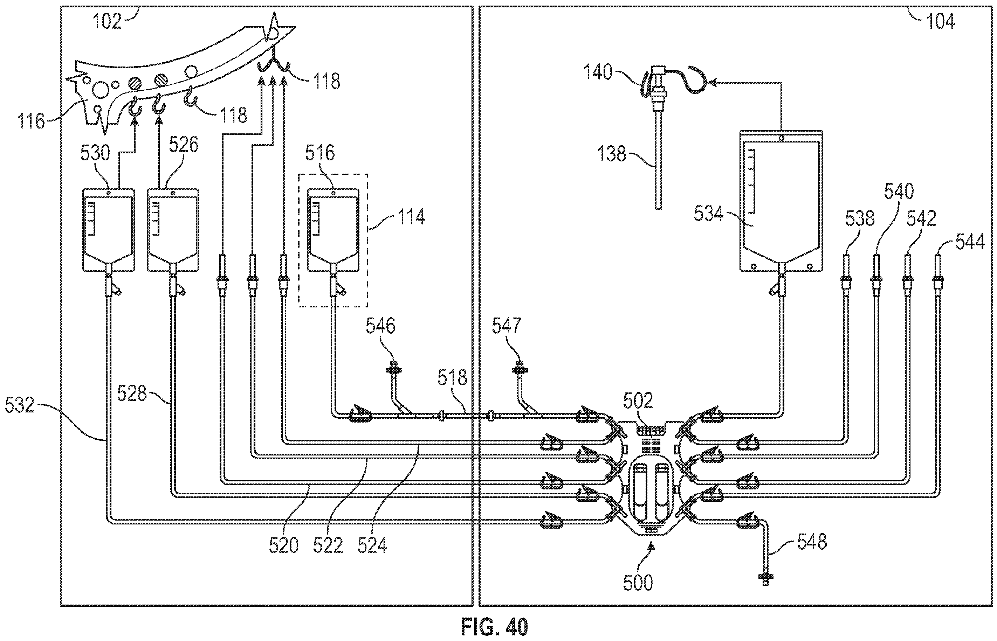

FIG. 40 illustrates integration/installation of the dosing preparation kit 500 on the processing apparatus 102 and isolation module 104. As illustrated therein, on the isolation module side, the stopcock manifold/cassette 502 is installed on the stopcock manifold interface 132 of the isolation module 104 such that respective motor output shafts 144 of the motors 146 are engaged with a respective one of the six stopcocks 504, 506, 508, 510, 512, 514 for controlling a position of the stopcocks. The waste bag 534 is suspended from one of the hooks 140 on the pole 138 of the isolation module, and one or more of lines 538, 540, 542, 544 are sterile welded (or connected via other means) to corresponding cryobags, which are then suspended from one of more of the hooks 140 on the pole 138 of the isolation module 104. Finally, the tubing tail with air filter 547 is connected to the line pressure sensor 148 of the isolation module 104, and a portion of the line connecting the 3D process bag 516 to the stopcock manifold 502 is engaged with the bubble sensor assembly 150 of the isolation module 104.

On the processing apparatus side, the initial product bag 530 and final formulation bag 526 are suspended from a single hook 118 of the hanger assembly 116 of the processing apparatus 102 (which has an integrated load cell or weight sensor for sensing a weight of the bag(s) suspended therefrom). Media bags (not shown) are sterile welded (or connected via other means) to media lines 520, 522, 524 and suspended from another hook 118 of the hanger assembly 116 of the processing apparatus 102 (which, likewise, has an integrated load cell or weight sensor for sensing a weight of the bag(s) suspended therefrom). The 3D process bag 516 of the kit is placed inside the thermal mixing chamber 114 of the processing apparatus 102. Finally, the section of peristaltic tubing 518 that fluidly interconnects the process bag 516 with the stopcock manifold 502 is engaged with the peristaltic pump assembly 111 of the processing apparatus 102, and the tubing tail with air filter 546 is connected to a pressure sensor (not shown) of the processing apparatus 102.

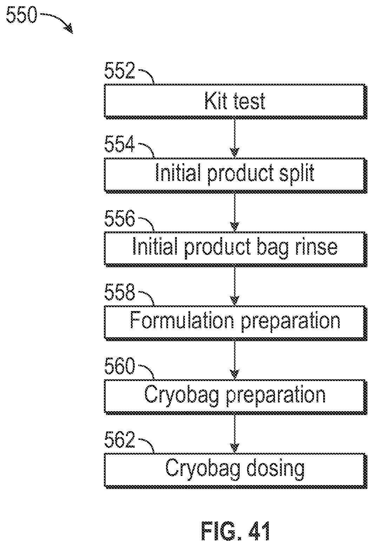

Turning to FIG. 41, a method 550 for preparing a dose of a cellular product using the processing apparatus 102, isolation module 104 and dosing preparation kit 500, is illustrated. As indicated above, the dosing protocol is carried out in an automated manner by the controller 110 of the processing module 102, controlling both the processing module 102 and isolation module 104 through the data connection therebetween. The method 550 includes, at step 552, testing and priming the kit 500 which, in an embodiment, may include evacuating air from the 3D process bag 516, cryobags and cryobag lines 538, 540, 542, 544 to minimize air in the bags at the end of the process, priming the 3D mixing bag 534 (to equalize the amount of air inside the 3D process bag 534), priming the media bag lines 520, 522, 524, and calibrating the pump 111 by flowing medium from the media bag connected to line 520 (to calibrate the pump speed with the exact weight pulled off the bags on the load cells/hooks). Next, at step 554, the initial product in bag 530 is split. In an embodiment, this involves transferring the entire input product from bag 530 to the process bag 516 positioned within the thermal mixing chamber 114 of the processing apparatus 102 and mixing the input product in the thermal mixing chamber 114 for a preselected or preset duration. A preset or preselected volume of product is then transferred from the process bag 516 to formulation bag 526. A remaining volume of product is transferred from the process bag 516 back to the initial input bag 530. In an embodiment, at step 556, the 3D process bag 516 is then rinsed with medium from a media bag connected to line 520, and the rinse volume is pumped to the input bag 530. In an embodiment, the rinse volume and rinse mix time can be selected by a user. As further shown therein, at step 558, formulation preparation is then carried out. In an embodiment, this includes transferring a predetermined volume of medium from the media bag connected to line 520 to the process bag 516 within the thermal mixer 114, and transferring such medium to the formulation bag 526.

If selected/desired, cryobag preparation and dosing can then be carried out at steps 560 and 562, respectively, to formulate additional bags (which may be cryobags for cryopreservation purposes). In such case, at step 560, a selected volume of split product from the input bag 530 is then transferred to the process bag 516 within the thermal mixer 114 (with excess split product remaining in the input bag 530). A predetermined volume of medium from the media bag connected to line 524 and/or media bag connected to line 522 is pumped to the process bag 516 within the thermal mixer 114, where temperature conditioning then takes place at a predetermined/preselected temperature (for a period of time calculated by the controller 110 needed to condition down to the target temperature). Next, medium from a media bag connected to line 520 is transferred to the process bag 516 within the thermal mixer (after a prompt), and the volume within the process bag 516 is mixed with such medium. Cryobag dosing is carried out by transferring a preselected volume to a first cryobag connected to line 538, a second cryobag connected to line 540, a third cryobag connected to line 542 and/or a fourth cryobag connected to line 544, as desired. Precise control of the volume transferred is enabled by ensuring an accurate peristaltic pump flow rate and controlling the flow timing. The peristaltic pump flow rate setpoint is calibrated during the initial priming step to account for possible deviations from the nominal baseline of the peristaltic pump tubing 518 and/or peristaltic pump 111. This protocol therefore allows for the formulation/production of one formulation bag 526, and up to four cryobags (connected to lines, 538, 540, 542 and 544, respectively). Accordingly, one to five doses/bags of a user-selected volume of up to four components (initial product plus three media) are enabled by such disclosed system and method.

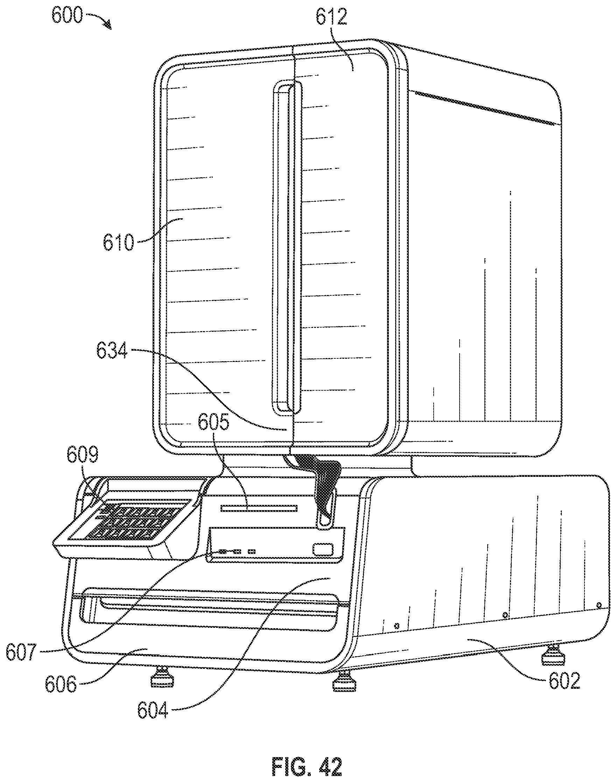

Turning now to FIGS. 42-45, an exemplary embodiment of a second module 600 (also referred to herein as bioprocessing apparatus 600) for the activation, transduction and expansion of cells (e.g., cells enriched and isolated using the first module 100) is illustrated. The second module 600 may be, for example, an apparatus/system configured to carry out the workflows and methods described above in connection with second module 200, and may be configured to operate similar to module 200 disclosed in WIPO International Publication No. WO 2019/106207. As shown therein, in an embodiment the second module 600 includes a housing 602, a latchable process drawer 604 slidably received within the housing 602, and a latchablewaste bag drawer 606 located beneath the process drawer 604 and likewise slidably received within the housing 602. Both the process drawer 604 and the waste bag drawer 606 are moveable between a closed position and an open position for inserting and removing various components of the second module 600, as disclosed hereinafter. As discussed in detail below, the process drawer 604 is configured to receive a disposable cell processing kit having one or more culture/bioreactor vessels therein. In an embodiment, a rear of the housing 602 includes a power connection port or cable, one or more communications ports (e.g., a RJ45 and RS485 ports), at least one inlet for receiving a supply of carbon dioxide, air oxygen, and/or nitrogen, etc., one or more outlet/exhaust ports, and/or a plurality (e.g., three) USB ports. The drawer 604 may also include a status indicator light 605, a plurality of USB or other ports 607 for the transfer of data, and an input terminal 609.

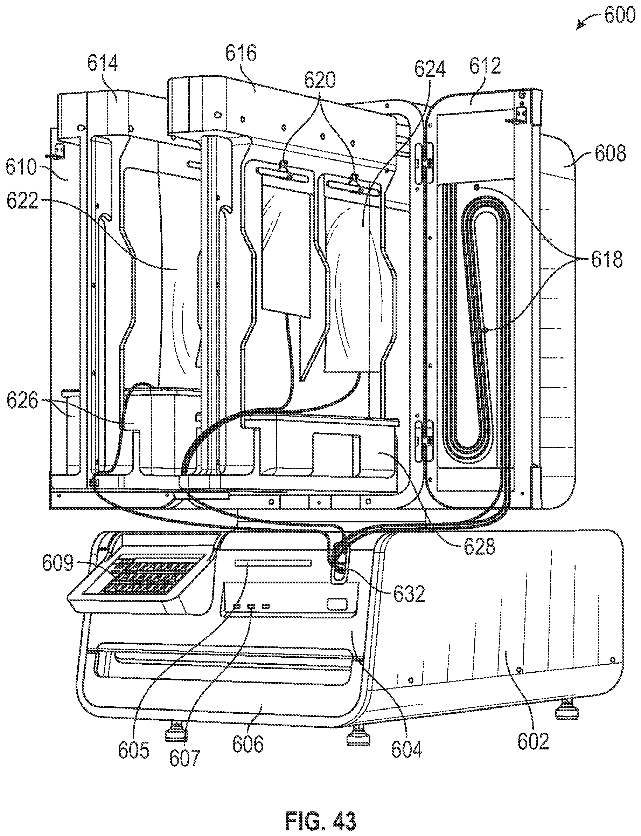

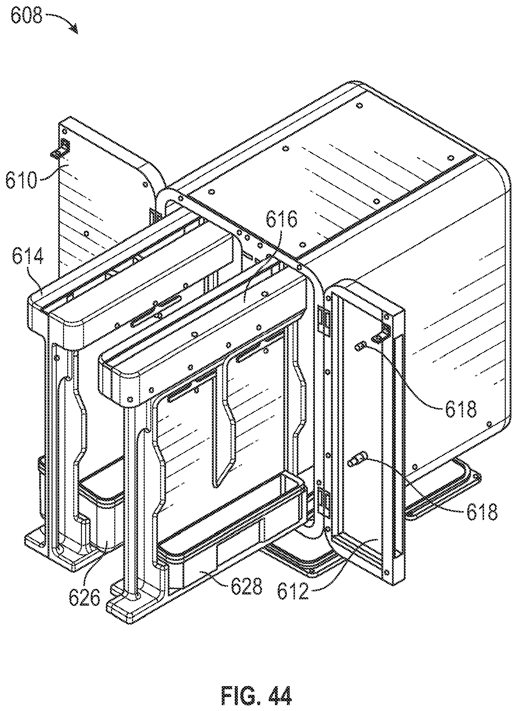

The second module 600 also includes a cabinet 608 positioned in stacked vertical relation to the housing 602 (e.g., mounted atop of the housing 602). The cabinet 608 includes a pair of latchable doors 610, 612 hingedly mounted about a vertical axis, which are configured to be moved between a closed position (preventing access to an interior of the cabinet 608) and an open position (allowing access to the interior of the cabinet 608). The cabinet 608 and doors 610, 612 may also include an interlock mechanism (e.g., a pneumatic latch or pin) that is utilized to maintain the doors 610, 612 in the closed position when a bioprocessing operation is in progress. In an embodiment, the cabinet 608 further includes a plurality of vertically-oriented storage drawers 614, 616 slidably received within the cabinet 608. While two vertical storage drawers 614, 616 are illustrated in FIGS. 43 and 44, more or fewer than two drawers may be present. In an embodiment, the storage drawers 614, 616 are slidably mounted on upper and/or lower tracks within the cabinet 608, allowing the drawers 614, 616 to be easily moved between a stowed position where the drawers are received within the cabinet 608 and the doors 610, 612 may be closed, and an extended position (shown in FIGS. 43 and 44) where the drawers 614, 616 extend from the cabinet 608, allowing for easy access to components and accessories mounted to the left and right vertical sides of the drawers 614, 616.

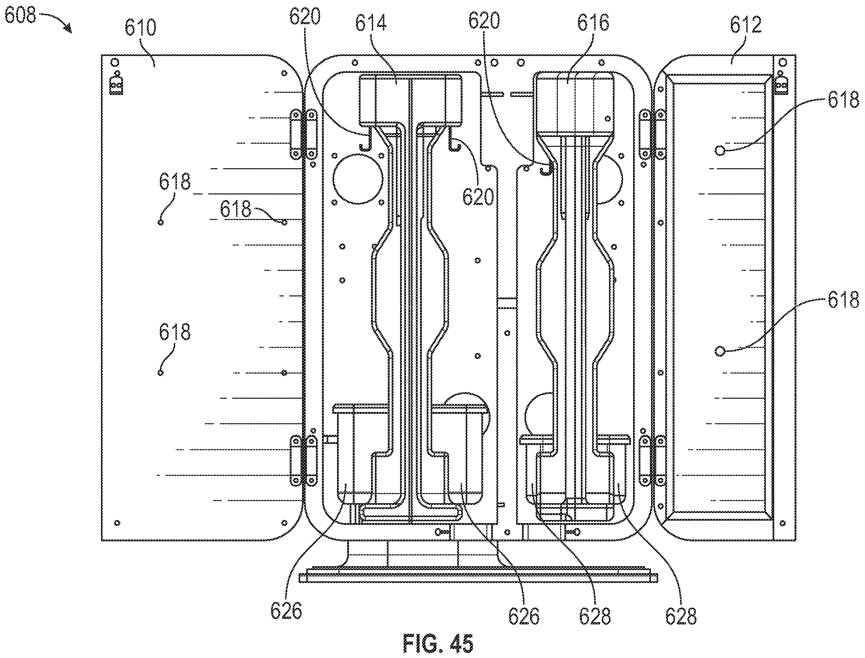

As best shown in FIG. 45 the interior faces of the doors 610, 612 contain a mechanism (e.g., a specific array of pegs or pins 618) for releasably connecting a tubing organizer card and/or sampling card to the doors 610, 612, as described below. For example, in an embodiment, the left door 610 may include an array of pegs for retaining a sampling card of a disposable kit, while right door 612 may include an array of pegs for retaining a tubing organizer card of the disposable kit. In an embodiment, both the tubing organizer card and sampling card may be mounted to the right door 612. As shown in FIGS. 43 and 45, one or both of the vertical storage drawers 614, 616, on one or each of the faces thereof, may include hooks 620 for receiving media, reagent and/or other fluid/solution bags for use in a variety of bioprocessing operations carried out by the apparatus 600. The hooks 620 may each be operatively connected to or integrated with a load cell for monitoring a weight of the bag(s) connected thereto. In one embodiment, the first vertical drawer 614 is configured to receive one or more media bags 622, while the second vertical drawer 616 is configured to receive one or more reagent bags 624. In this respect, the first vertical drawer 614 may be referred to as a media tray or compartment, while the second vertical drawer 616 may be referred to as a reagent tray or compartment. The first vertical drawer 614 is equipped with media drip trays 626 on opposed faces thereof, for catching leaks or drips from the media bags suspended from hooks 620, while the second vertical drawer 616 is equipped with reagent drip trays 628 on opposed faces thereof, for catching leaks or drips from the reagent bags 624 suspended from hooks 620. In an embodiment, the drip trays 626, 628 are removable form the drawers 614, 616, respectively.

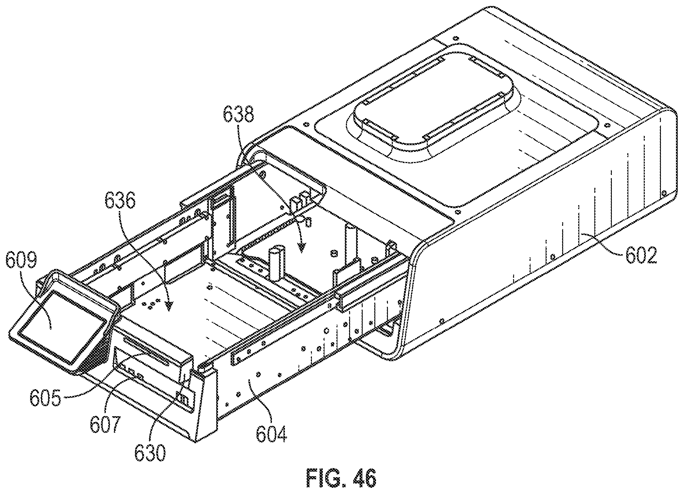

In an embodiment, one or more of the vertical drawers 614, 616 may be housed within a refrigerated compartment that forms part of the cabinet 608, for maintaining a fluid or solution contained in one of the bags 622, 624 at a predetermined temperature. Similar to housing 604, the cabinet 608 may likewise include a status indicator light 634. While FIGS. 42-45 illustrate the waste bag drawer 606 as being part of the lower housing 602, it is contemplated that the waste bag drawer may, alternatively, be housed within cabinet 608 (e.g., as a horizontally-oriented drawer, or as a vertically-mounted drawer). As best shown in FIGS. 43 and 46, the process drawer 604 includes an upwardly-facing slot 630 that is configured to receive an anchor comb 632 that facilitates the routing of tubing from the cabinet 608 into the process drawer 604. In an embodiment, the entire apparatus 600 is sized and dimensioned so as to be supported by a table or benchtop and so that the process drawer 604 and cabinet 608 can be easily accessed by a user. Control of the apparatus 600 and its functions is carried out by an on-board controller (e.g., controller 210), as disclosed hereinafter.

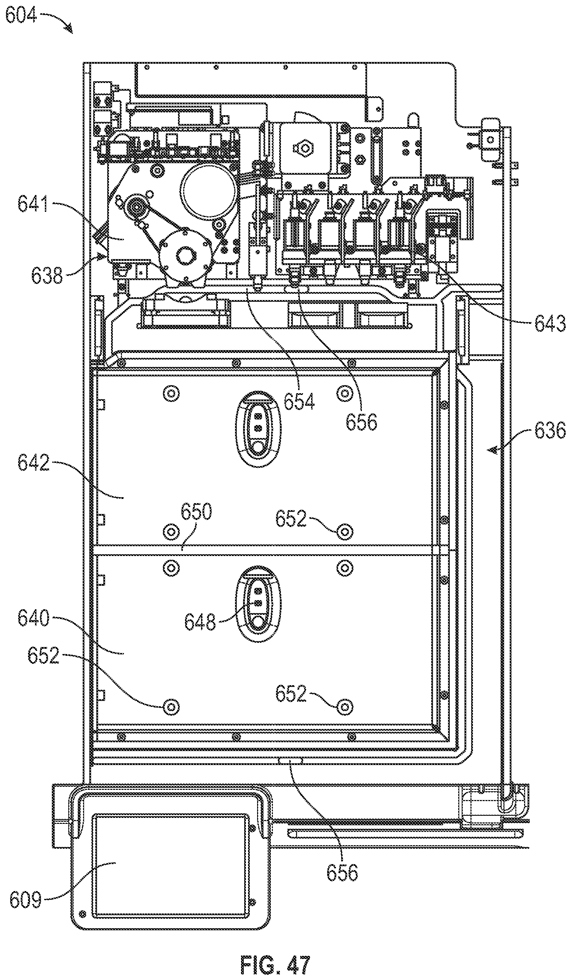

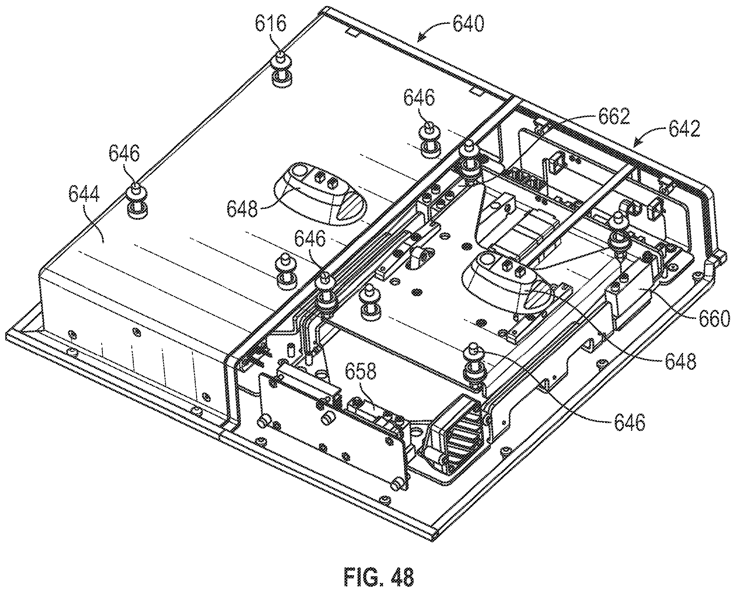

Turning now to FIGS. 46 and 47, detailed views of the process drawer 604 are illustrated. As best shown in FIGS. 46 and 47, the process drawer 604 includes a first interior space 636 configured to receive a disposable bioprocessing kit, and a second interior space 638 positioned rearward of the first interior space 436, within which functional components of the apparatus 606 are mounted. For example, in an embodiment, the second interior space 638 houses a peristaltic pump assembly 641, a pinch valve array or linear actuator array 643 (for controlling a flow of fluid through an array of fluid flow lines), and other components and devices necessary to carry out the functions of the apparatus 600. In an embodiment, the peristaltic pump assembly 641, and other components and devices may be configured as disclosed in WIPO International Publication No. WO 2019/106207. As shown in FIG. 47, within the first interior space 636 are mounted first and second platform rocker assemblies 640, 642 which are configured to support thereon culture vessels (also referred to herein as bioreactor vessels) of a disposable bioprocessing kit in the manner disclosed hereinafter. The platform rocker assemblies 640, 642 each have a cover 644 through which a plurality of culture vessel support or mounting posts 646 extend, for supporting a culture vessel of the disposable kit. In an embodiment, each platform rocker assembly 640, 642 includes four support posts 646, as more clearly shown in FIG. 48. As also shown therein, a sensor assembly 648 associated with each platform rocker assembly 640, 642 is provided to detect the presence of a culture vessel and/or measure a temperature within the culture vessel. In other embodiments, the sensor assembly 648 may be used to measure various additional parameters (e.g., temperature, carbon dioxide concentration, oxygen concentration, etc.) of a culture within a culture vessel received atop each platform rocker assembly 640, 642 and/or for determining if the culture vessels are properly positioned and seated on the rocker assemblies. As discussed below, each platform rocker assembly 640, 642 includes a plurality of load cells 658, 660, 662 for sensing the weight/mass of a culture vessel support by the mounting posts 646.

Referring once again to FIG. 47, the process drawer 604 contains a number of features that are configured to contain leaks and to prevent or inhibit any fluid from collecting within the process drawer 604. For example, the process drawer 604 includes a seal element 650 that forms a fluid-tight seal between each platform rocker assembly 640, 642 and the bottom of the process drawer 604 (which extends around the periphery of each rocker assembly), as well as between the rocker assemblies 640, 642, themselves. In addition, each culture vessel support post 646 is outfitted with a seal element in the form of a flexible bellows 652 that forms a seal between the support posts 646 and the covers 444. The seal element 650 and bellows 652 prevent any fluid from entering the space beneath the covers 644 of the platform rocket assemblies 640, 642. Still further, the bottom of the process drawer 604 is formed with a peripheral channel 654 that collects fluid that has spilled or leaked. Drain holes 656 in the channel 654 provide a means of egress for the fluid that collects in the channel 654 of the process drawer 604. The drain holes 656 are in fluid communication with the waste drawer 606 beneath the process drawer 604, so that any fluid that spills or leaks into the process drawer 604 is drained directly into the waste drawer 606 to prevent harm to the electromechanics in the process drawer 604.

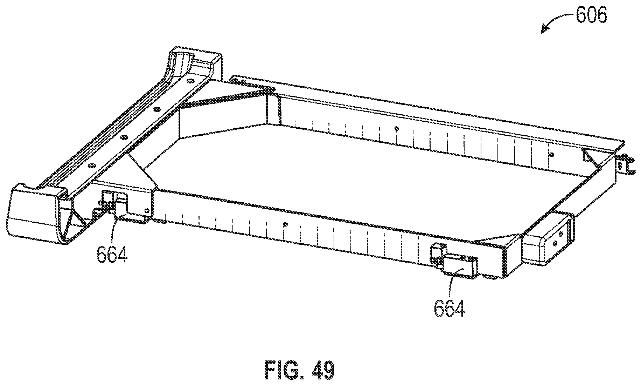

FIG. 49 illustrates a configuration of the waste drawer 606 which, as shown therein, includes a plurality of load cells 664. In an embodiment, there are four load cells positioned adjacent to the four corners of the waste drawer 606. As indicated above, the waste drawer is slidably received in the housing 602 beneath the process drawer 604, and is configured to receive a waste bag. In an embodiment, the tubing that connects to the waste bag is routed from the process drawer along a groove behind the front panel of the process drawer so as to escape the process drawer and then route freely down to the waste drawer. Further, as indicated above, the waste drawer 606 is configured to directly receive fluid that has leaked into the process drawer via drain holes 656 in the process drawer 604.

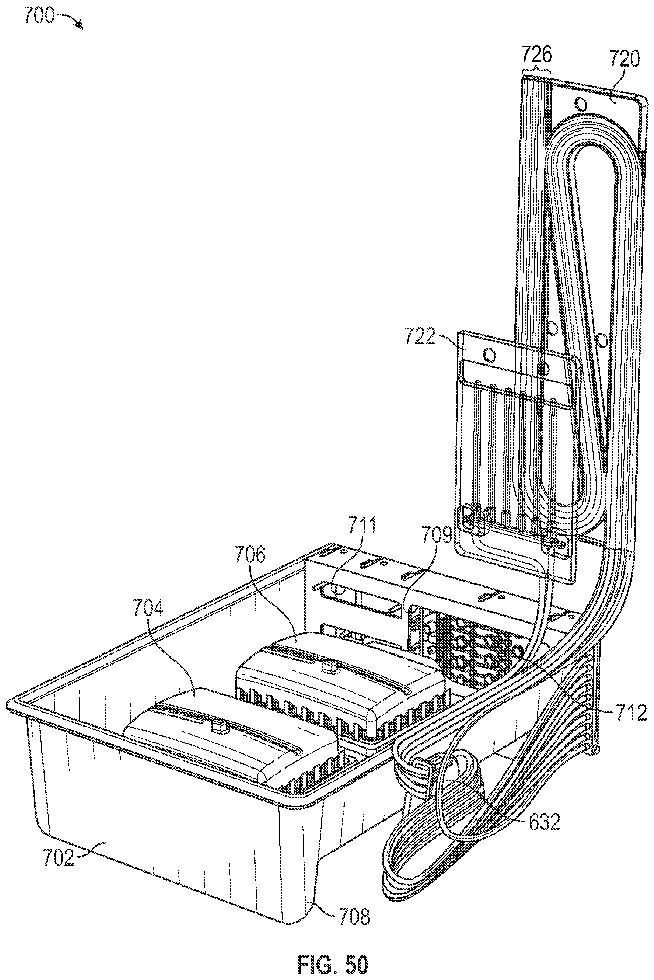

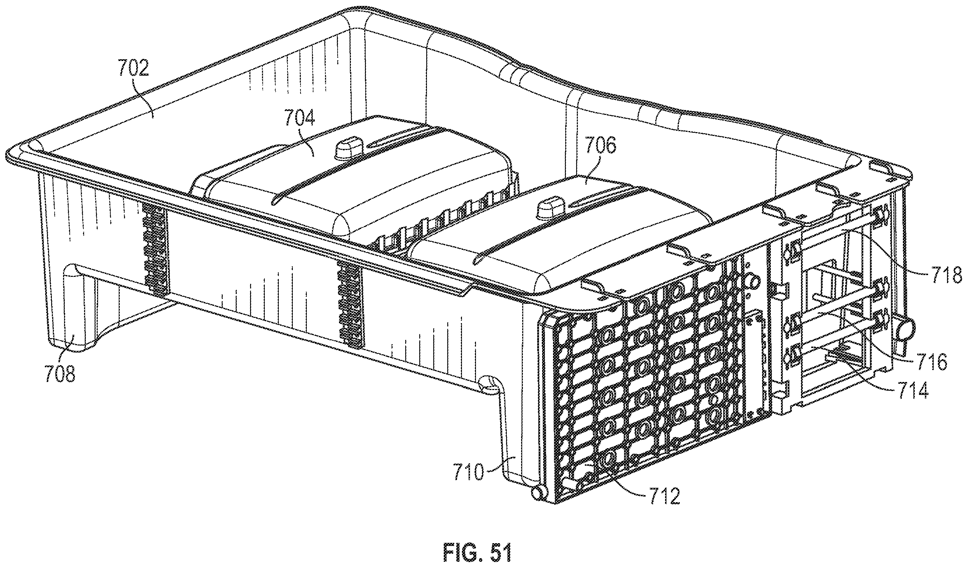

Referring now to FIG. 50, a single-use, disposable bioprocessing kit 700 for use with the bioprocessing apparatus 600 is illustrated. The bioprocessing kit 700 includes a generally rectangular tray 702 sized and dimensioned to be received in the first interior space 636 of the process drawer 604 and a pair of culture vessels 704, 706 received within the tray 702. The tray 702 has a pair of openings or windows beneath the culture vessels 704, 706 and supports the culture vessels 704, 706 in an elevated position such that the culture vessels 704, 706 are lifted from the tray 702 when engaged with the support posts 646 of the platform rocker assemblies 640, 642 when the tray 702 is positioned within the first interior space 636 of the process drawer 604. As shown in FIGS. 50 and 51, the tray 702 includes a pair of legs 708, 710 located at the front and rear of thereof that support the tray 702 on the bottom of the process drawer 604. The legs 708, 710 are hollow and form a low point of the tray 702. Accordingly, in the event of leaks or spills within the tray 702 (as opposed to in the process drawer 604), the fluid will collect and be contained in the bottom of the legs 708, 710.

With further reference to FIGS. 50 and 51, the tray further 702 further includes first and second windows 709, 711 in the rear of the tray 702 within which are positioned a valve manifold 712 and up to three segments 714, 716, 718 of peristaltic pump tubing for engagement with the peristaltic pump assembly 641 mounted in the process drawer 604 rearward of the tray 702. The valve manifold 712 may be, for example, a fluidic vessel as disclosed in U.S. Patent Application Publication No. 2020/0238282, which is configured to interface with a plurality of linear actuators having plungers of linear actuator array 643, which is likewise mounted in the process drawer 604 rearward of the tray 702. Alternatively, the valve manifold 712 may be formed from a plurality of fluid flow lines configured to be acted upon by a plurality of pinch valves of a pinch valve array, as disclosed in WIPO International Publication No. WO 2019/106207. The valve manifold 712 is fluidly interconnected with the culture vessels 704, 706, the media bags and reagent bags in the cabinet 608, the waste bag in the waste drawer 606, and sampling lines to form a fluidic network or architecture as disclosed in, or similar to that disclosed in, the '207 publication. FIG. 50 illustrates connection of the various tubes with the valve manifold 712.

As further illustrated in FIG. 50, therefore, the disposable kit 700 further includes a tubing organizer card 720 that retains a plurality of tubing tails 726 that are fluidly connected to the valve manifold 712 and which are configured for connection to the various media and reagent bags housed within the cabinet 608, and a sampling card 722 that retains a plurality of sampling tubing tails that are, likewise, fluidly connected to the valve manifold 712. Lastly, the disposable kit 700 also includes the anchor comb 632 which is received in the slot 630 in the process drawer 604 and which facilitates the routing of tubing from the cabinet 608 (e.g., from the tubing organizer 720 and sampling card 722 into the process drawer 604 and to the valve manifold 712. As discussed hereinafter, the anchor comb 632, tubing organizer 720 and sampling card 722 provide a means to organize all of the tubing tails during and after installation of the kit 700 and connection of the various media, reagent and other bags/containers. In an embodiment, the disposable kit 700, including all of the elements described above in connection with FIG. 50, may be sterilized by means known in the art such as, for example, ethylene oxide sterilization or gamma sterilization, and sealed in a blister pack for transport to an end user and for storage.

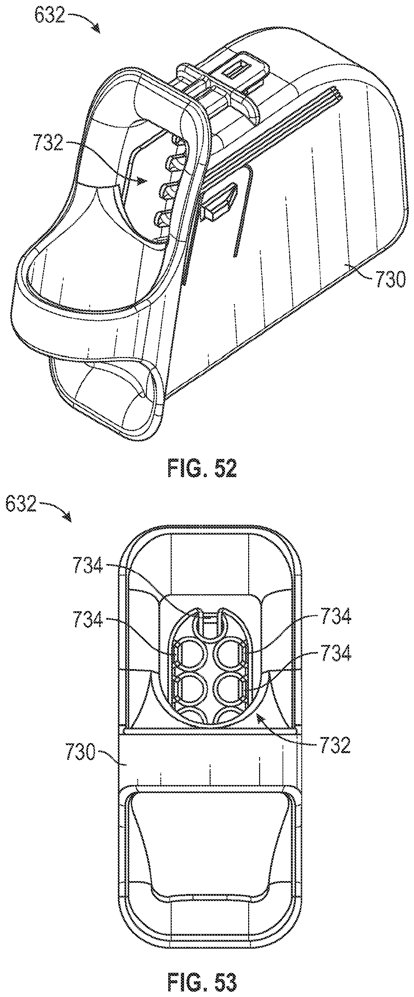

As illustrated in FIGS. 52 and 53, the anchor comb 632 includes a body portion 730 having a passage 732 therethrough. Within the passage 732 are a plurality of tubing retention elements 734 which function to retain and maintain lengths of tubing in an organized fashion. As disclosed above, during installation, the anchor comb 632 is received within the slot 630 of the process drawer 604 and facilitates routing of the various passes of tubing from the cabinet 608 into the process drawer 604 where they are fluidly connected to the valve manifold 712.

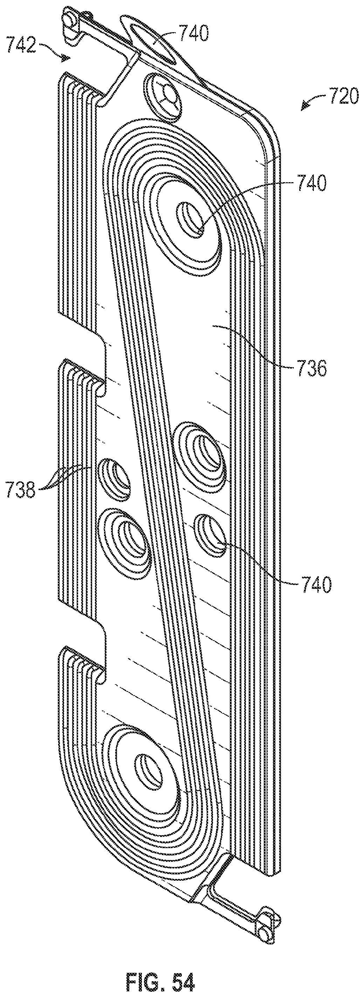

With reference to FIG. 54, a detailed view of the tubing organizer 720 according to a disclosed embodiment is illustrated. The tubing organizer 720 includes a generally rigid plate body 736 and a plurality of tubing retention channels 738 molded into or otherwise connected to the rigid plate body 738 and being configured to receive and retain a corresponding plurality of tubing tails 726 therein. In an embodiment, the channels 738 extend from a lower, right hand corner of the plate body 736 upward along the right hand side thereof, turn back upon themselves and extend generally downwardly at an angle toward the lower, right hand corner of the plate body 738, turn back upon themselves once again, and extend from a lower, left hand corner of the plate body 736 upward along the left hand side thereof. Tubing tails 726 received in these channels 738 thus follow the same tortuous path. This serpentine configuration of the channels 738 thus maximizes the length of the tubing tails 726 that are able to be retained by the tubing organizer, allowing for a fair degree of play to facilitate connection of the tubing tails 726 to the various bags and/or containers contained within the cabinet 608 of the bioprocessing apparatus 600. The tubing organizer 720 thus maintains the tubing tails 726 in an organized and easy to access manner which helps minimize set up time.

As also shown in FIG. 54, the plate body 736 includes features that allow the tubing organizer 720 to be removable mounted or hung from the inside of the door 612 of the cabinet 608, as shown in FIG. 43. Such features may include, for example, mounting and/or locating apertures 740 through which pegs 618 or hooks on the door 612 are received. In use, once the tubing organizer 720 is attached to the interior face of the door 612, a user may easily grasp an end of a tubing tail 726 that extends into a clearance or relieved area 742 of the plate body 736 and remove it from its seated position with its corresponding channel 738. The tubing tail 726 may then be connected to a media bag, reagent bag or other vessel contained within the cabinet 608 by means of aseptic techniques such as, for example, sterile tube welding. This process may be repeated until all fluid connections between the bags housed in the cabinet 608 and the valve manifold 712 housed in the drawer 604 are made.

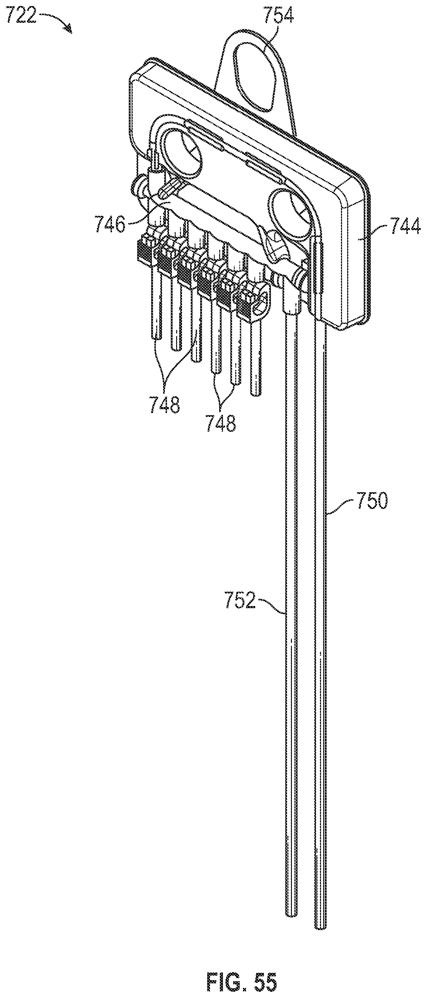

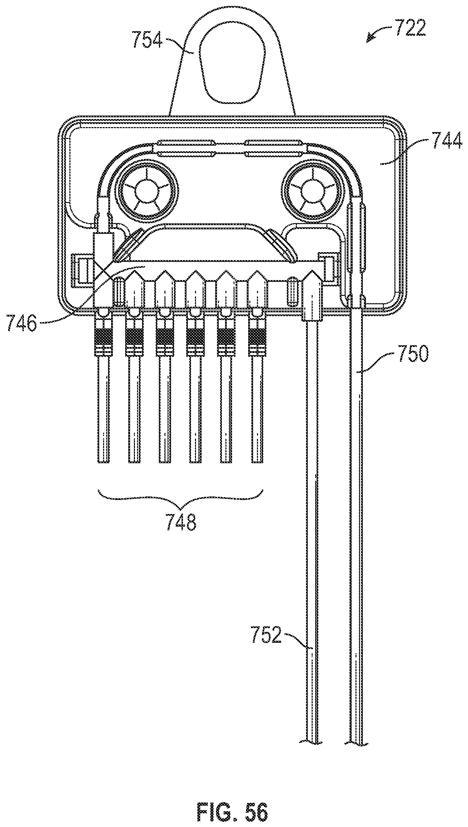

With reference to FIGS. 55 and 56, detailed views of the sampling card 722 according to a disclosed embodiment is illustrated. As shown therein, the sampling card/apparatus 722 includes a body portion 744 having a manifold 746 and a plurality of sampling tubing tails 748 fluidly connected to the manifold 746. The sampling card 722 also includes a feed line 750 fluidly connected to a first end of the manifold 746, and a return line 752 fluidly connected to a second end of the manifold 746. Similar to the tubing organizer 720, the body portion 744 of the sampling card 722 includes features that allow the sampling card 722 to be removable mounted or hung from the inside of the door 612 of the cabinet 608. Such features may include, for example, mounting and/or locating apertures 754 through which pegs 618 or hooks on the door 612 are received. In use, once the sampling card 722 is attached to the interior face of the door 612, a user may draw a sample from one of the culture vessels 704, 706 using one of the sampling tubing tails 748 that are easily accessible on the sampling card 722. Accordingly, samples may be easily drawn during a bioprocessing operation, without necessitating opening of the process drawer 604 and without having to pause operations.

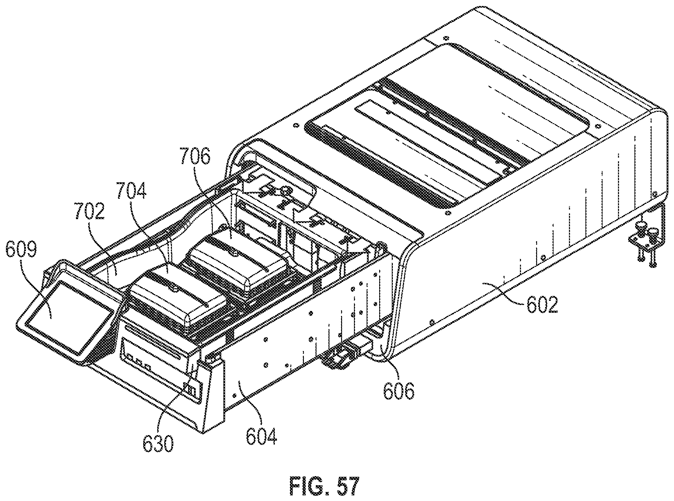

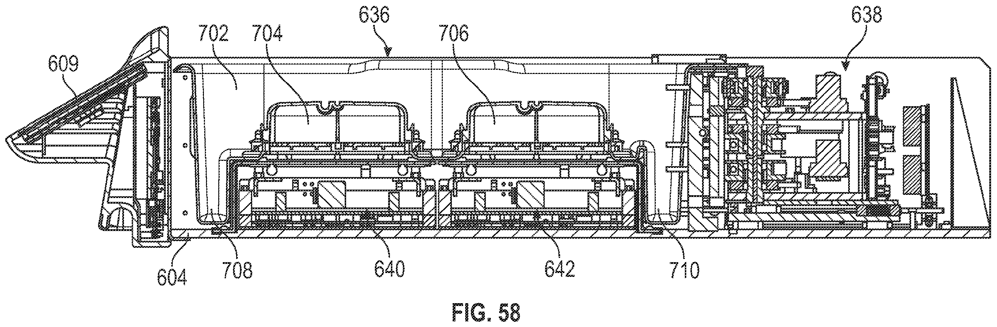

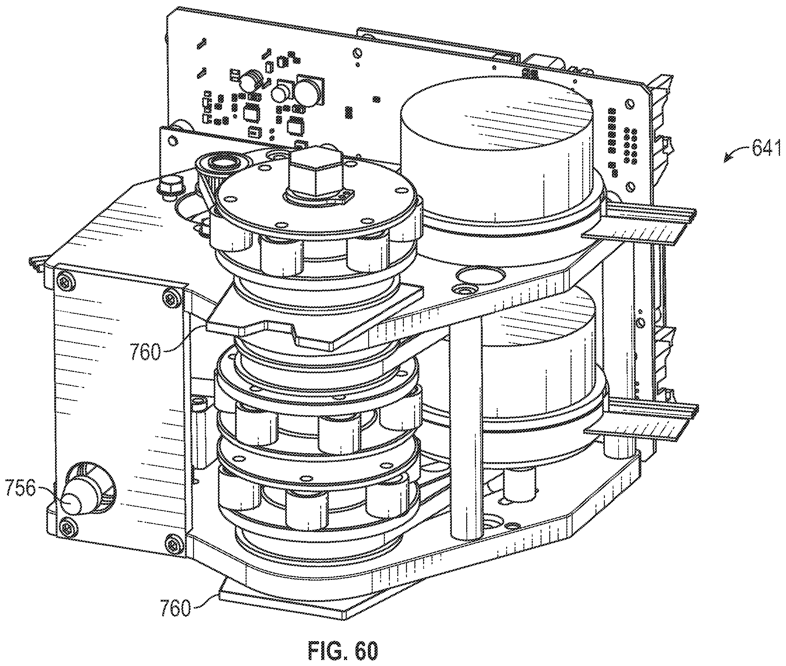

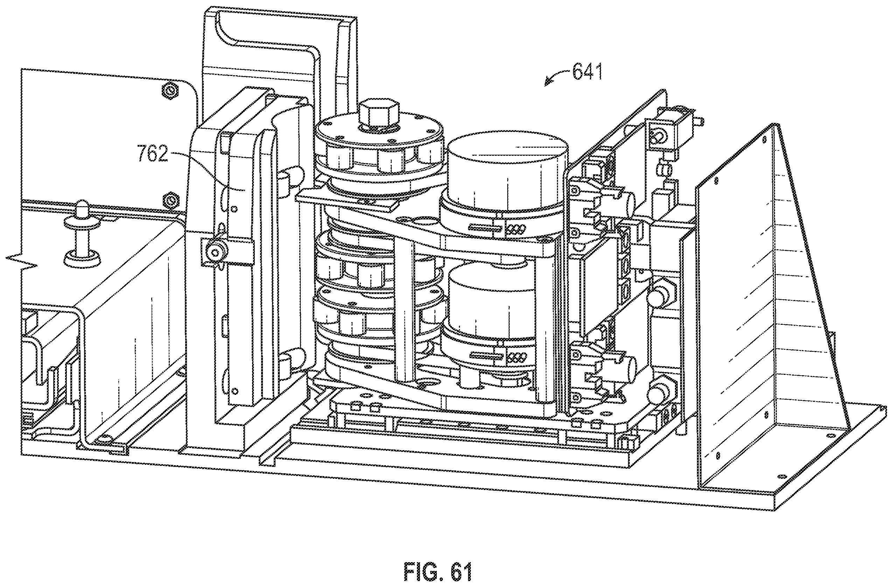

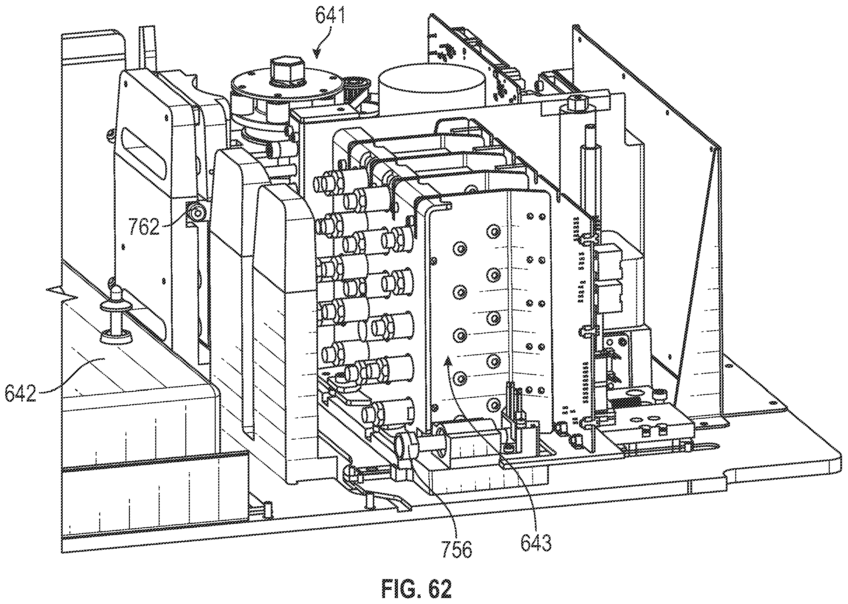

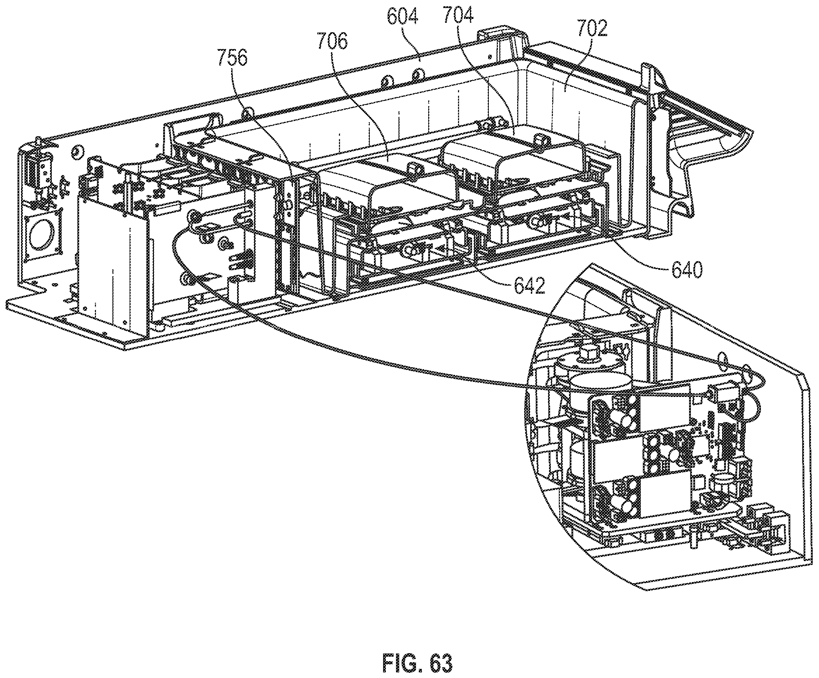

Turning now to FIGS. 57-63 installation and seating of the tray 702 within the process drawer 604 of the bioprocessing apparatus 600 are illustrated. As shown therein, the tray 702 is received within the first interior space 636 the process drawer 604 by opening the process drawer 604 and lowering the tray 702 into the process drawer 604 from above, such that the culture vessels 704, 706 of the disposable kit 700 are in front-to-back-relation within the process drawer 604. In this position, the valve manifold 712 is positioned just forward of, and aligned with, the linear actuator array 643, and the three segments 714, 716, 718 of peristaltic pump tubing are positioned just forward of, and aligned with, the peristaltic pump assembly 641. As indicated above, as the tray 702 is lowered into the process drawer 604, the culture vessels 704, 706 are received on support/mounting posts 646 of the respective platform rocker assemblies 640 such that the culture vessels 704, 706 are lifted from their seated engagement with tray 702 and instead supported by the support posts 646.

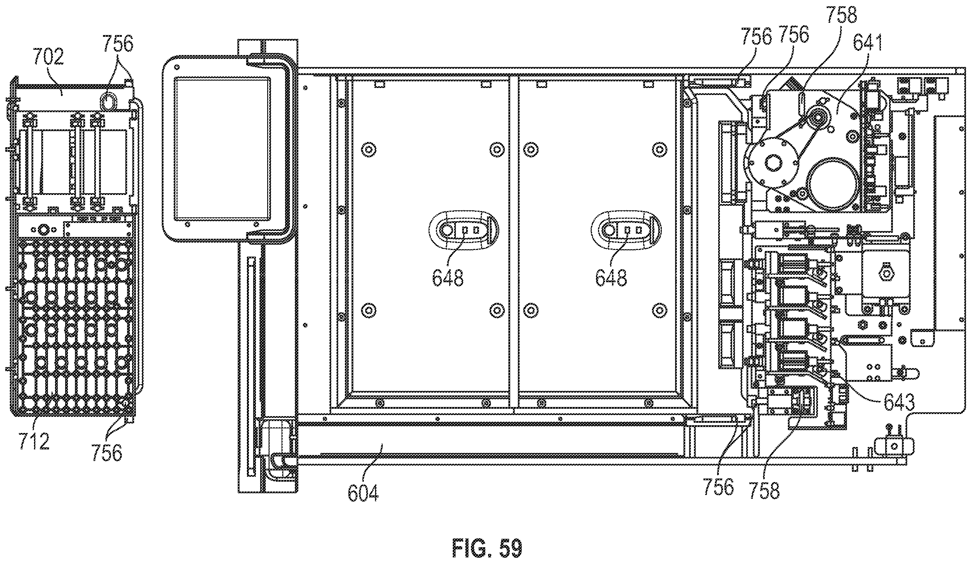

As most clearly shown in FIGS. 59-62, the tray 702 of the disposable kit 700 and the process drawer 604 have a number of cooperating features that facilitate proper positioning of the tray 702 within the process drawer 604, and allow for verification of proper positioning. For example, as shown in FIG. 59, the tray 702 and process drawer 604 include a plurality of engagement features/surfaces 756 that cooperate with one another when the tray 702 is properly positioned within the process drawer 604. The process drawer 604, in the second interior space 636, includes a number of sensors 758 associated with the engagement features 756 of the drawer that can detect when the cooperating engagement features 756 on the tray 702 and process drawer 604 are engaged with one another, indicating proper positioning of the tray 702. In an embodiment, the engagement features 756 associated with the tray 702 are located on the backbone of the tray, as best shown in FIG. 59, while the corresponding engagement features 756 associated with the process drawer 604 (and sensors 758) are located adjacent to the linear actuator array 643 and peristaltic pump assembly 741, respectively. In an embodiment, the engagement features 756 associated with the process drawer 604 are pins of sensors 758. In addition to detecting the proper alignment and positioning of the tray 702 within the process drawer 604, as indicated above, the platform rocker assemblies 640, 642 include sensors 648 that are configured to detect proper positioning of the culture vessels 704, 706.

Moreover, in addition to the engagement features and sensors disclosed above, the peristaltic pump assembly 641 also includes upper and lower engagement structures 760, as well as a pivoting pump shoe 762, that facilitate proper engagement of the peristaltic pump assembly 641 with the backbone of the tray 602. These features also minimize tolerance stack-up issues with respect to the engagement and actuation of the peristaltic pump assembly 741 and liner actuator array 742 with the segments 714, 716, 718 of peristaltic pump tubing and valve manifold 712, respectively.

In an embodiment, the peristaltic pump assembly 641 and solenoid actuators of the valve manifold 712 are configured to move towards and physically engage with the corresponding features of the disposable kit when the disposable kit is positioned in the process drawer and the drawer is closed. In particular, with specific reference to FIG. 59, the module 600 includes a motorized engagement mechanism that physically moves the assembly containing the peristaltic pump assembly 641 and solenoid array 643 towards the corresponding features in the disposable kit 700 (segments 714, 716, 718 of peristaltic pump tubing and valve manifold 712) with a fixed distance of movement limited by a feature that prevents further movement. Disengagement simply involves operating this motorized engagement mechanism in reverse.

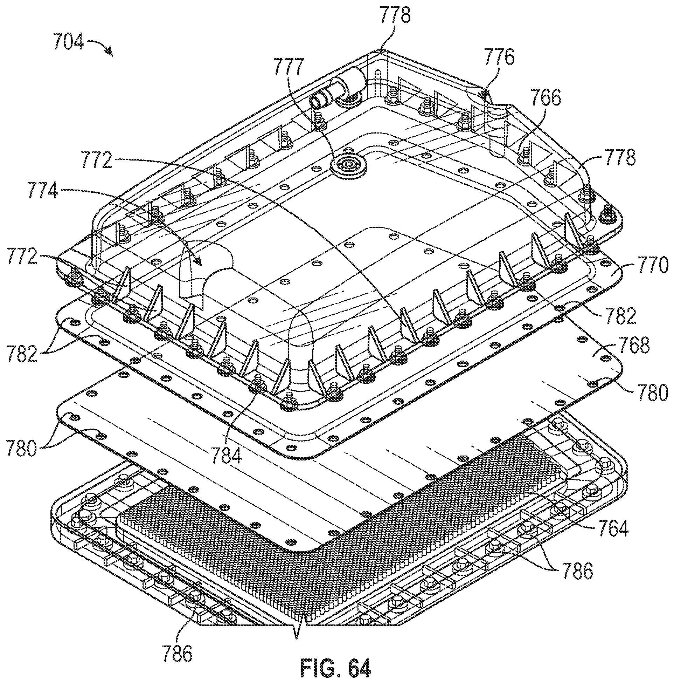

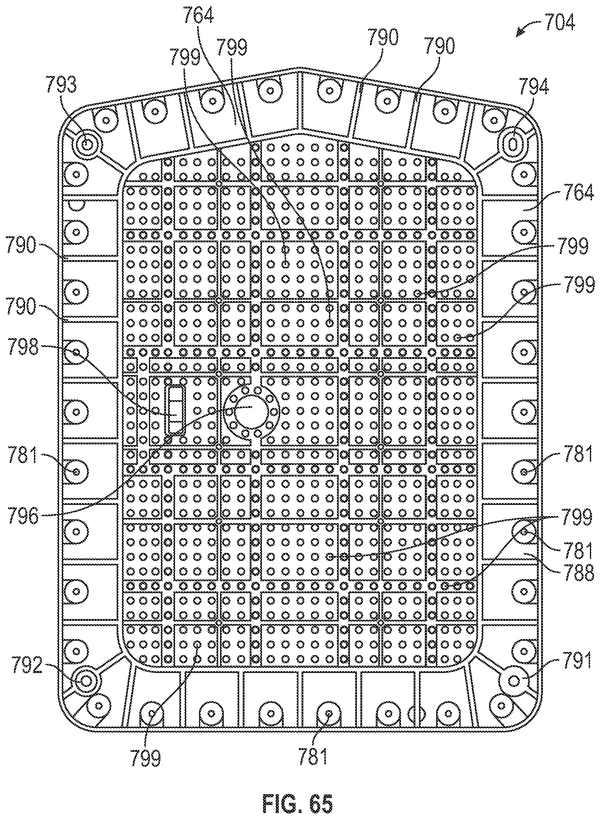

Referring now to FIGS. 64 and 65, the configuration of the bioreactor/culture vessels 704, 706 of the disposable bioprocessing kit 700 are shown. For ease of illustration, only culture vessel 704 is illustrated (culture vessel 706 being an exact duplicate). As shown therein, in an embodiment, the culture vessel 704 includes a base 764, a lid 766 connected to the base 764, a gas-permeable, liquid impermeable membrane 768 sandwiched between the base 764 and the lid 766, and a gasket 770 sandwiched between the membrane 768 and the lid 766. In an embodiment, the base 764 and the lid 766 are formed from polycarbonate, although other materials known in the art may also be utilized without departing from the broader aspects of the disclosed embodiment. As shown in FIG. 64, the lid 766 includes a plurality of bolstering supports 772 or gussets that reinforce the lid 766 and provide increased strength and durability. The lid 766 also includes inlet and outlet ports 774, 776 to which tubing may be connected. As illustrated therein, the inlet and outlet ports 774, 776 are molded into the lid such that the tubing extends, at least initially, vertically from the lid 776. This configuration of the ports 774, 776 facilitates set up, as the tubing can be more easily connected to the culture vessel 704 from above. As further illustrated, a vent port 777 is provided in the top of the lid 766. In an embodiment, the lid 766 includes rounded corners (e.g., corners 778), which eliminate/prevent any stagnant zones.

With further reference to FIG. 64, the membrane 768 may be formed from a suitable gas permeable material, e.g., silicone and/or polystyrene, or a porous material with a pore size that does not allow the passage of water or microbes, although other materials known in the art may also be utilized without departing from the broader aspects of the disclosed embodiment. The membrane 768 includes a plurality of location/retention holes 780 along the periphery of the membrane 768, the purpose of which will be described hereinafter. The gasket 770, for its part, may be formed from a variety of materials know in the art such as, for example, silicone, and includes a corresponding plurality of location/retention holes 782 located along the periphery of the gasket 770 and aligned with the holes 780 in the membrane 768.

In an embodiment, the lid 766 and base 764 are connected to one another via heat staking along the periphery of the lid 766 and base 764. In an embodiment, the heat stakes 781 extend through each of the location/retention holes 780, 782 of the membrane 768 and gasket 770, respectively, and function to anchor the membrane 768 and gasket 770 between the base 764 and lid 766. In an embodiment, the lid 766 may be configured with heat stake pins 784 that extend downwardly from the underside thereof, such that during assembly, the heat stake pins 784 extend through the corresponding location/retention holes 780, 782 of the membrane 768 and gasket 770, respectively, and are received in corresponding holes 786 in the periphery of the base 764 and heat staked to the base 764. In an embodiment, the lid 766 is joined to the base 764 using about 20 to about 40 heat stakes and, more preferably, approximately 34 heat stakes. While the embodiments described herein utilize heat staking to connected the lid to the base, it is contemplated that other connection means may also be utilized, such as fasteners, snap-fit connections and the like, without departing from the broader aspects of the disclosure.

In an embodiment, the upper surface of the base 764 has a textured surface that allows air flow and eliminates the need for a mesh (which has been customary on prior designs). As shown in FIG. 65 a flange region 788 of the base 764 includes a plurality of ribs 790 that provide for increased rigidity and strength and a more robust interconnection with lid 766 (which additionally provides more reliable and robust anchoring of the membrane 768 and gasket 770). The corners of the underside of the base 764 each include a pin well 791, 792, 793, 794 configured to receive therein a mounting/support post 646 of the platform rocker assembly 640 or 642 that supports the culture vessel 704. In an embodiment, one of the pin wells (e.g., well 794) is oblong in shape, which provides for improve positional tolerancing when installing the culture vessel 704 atop the platform rocker assembly 640. The base 764 is further provided with an IR sensor window 796 for measuring the temperature of the gas or fluid(s) within the culture vessel 704 using sensors positioned beneath the culture vessel 704 within the process drawer 604, and a sensor well 798 that is utilized by the sensor 648 of the platform rocker assembly 640 or 642 to determine if the culture vessel 704 is present within the process drawer and/or properly positioned therein. Finally, as illustrated in FIG. 65, the base 764 includes an array of small openings 799 that provide fluid communication between an atmosphere within the process drawer 604 and the underside of the membrane 768 for gas transfer during bioprocessing. In an embodiment, there are several hundred small openings 799 in the base 764.

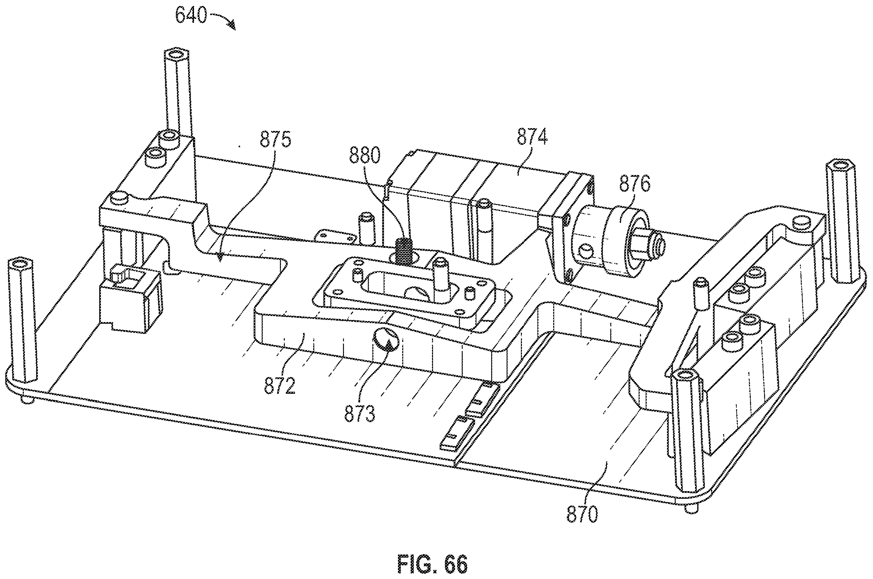

As indicated above, the culture vessels 704, 706 are configured to be received on the platform rocker assemblies 640, 642 when the tray 702 is received in the process drawer 604. Various rocking mechanisms known in the art may be utilized to provide for mixing of the fluid within the culture vessels 704, 706 to support bioprocessing operations therein, including the mechanism disclosed in WIPO International Publication No. WO 2019/106207. FIGS. 66-68 illustrate a configuration of the platform rocker assemblies 640, 642 according to the invention (rocker assembly 640 being depicted for simplicity and ease of understanding). As shown therein, platform rocker assembly 640 includes a base 870, a fulcrum 872 defining a central pivot axis 873 received on the base 870, a motor 874 mounted to the base 870 and having an eccentric roller 876 driven by the motor 874, a rocking plate 878 received atop the fulcrum 872 and in contact with the eccentric roller 876 and being pivotable about the fulcrum axis 873, and a compression spring 880 configured to maintain the rocking plate 878 in contact with the eccentric roller 876. In an embodiment, the fulcrum 872 and motor 874 are connected to the base 872 via a frame 875. In an embodiment, the eccentric roller 876 is a circular roller configured to rotate along an eccentric pathway. In yet other embodiments, in place of a circular roller moving along an eccentric path, a cam-shaped roller may be employed.

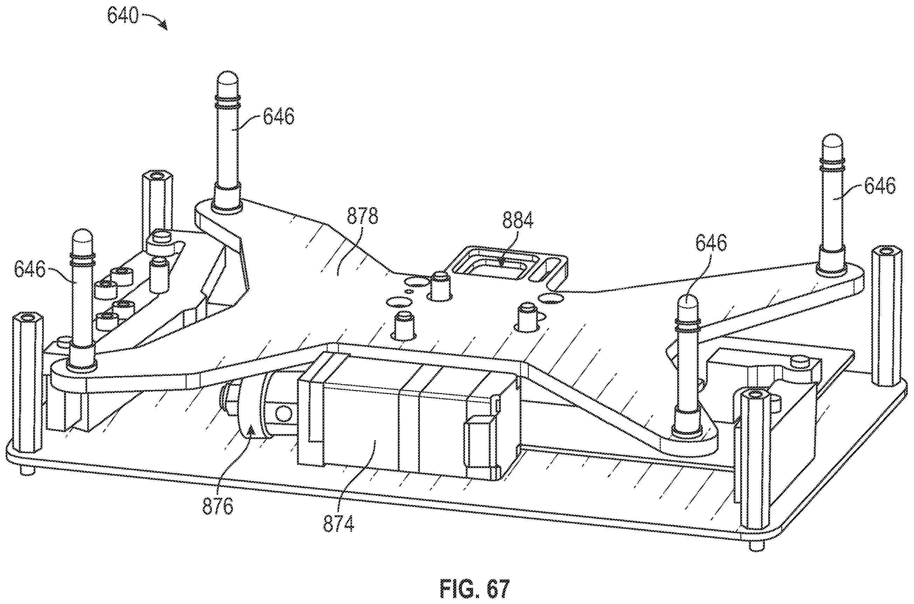

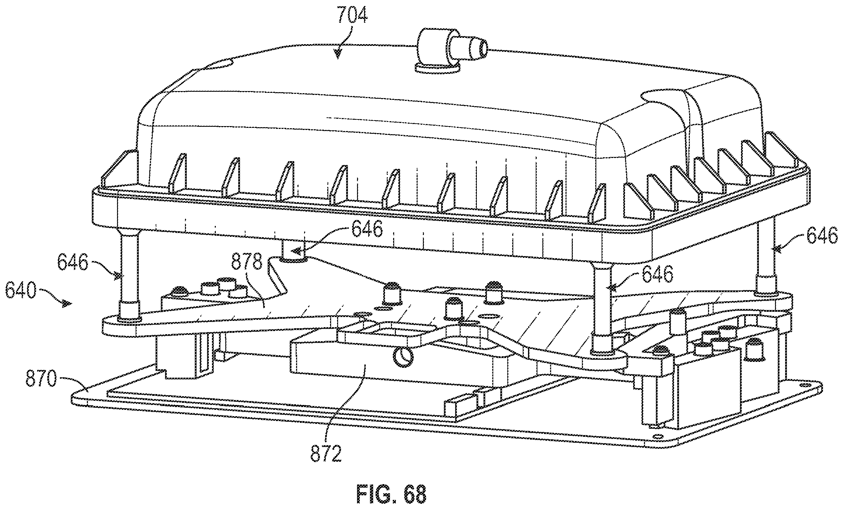

As illustrated in FIGS. 67 and 68, the rocking plate 878 includes four support posts 646 that are received by the pin wells 791, 792, 793, 794 in the base 764 of the culture vessel 704. The motor 874 is controllable (e.g., under control of the controller 210 of the second module 200 (i.e., apparatus 600)) to drive the eccentric roller 876 to transmit a force against or remove a force from an underside of the rocking plate 878 depending on the position of the eccentric roller 876 to tilt the rocking plate 878 and culture vessel 704 received thereon upward and/or downward. While the motor 874 may be controllable by the master controller, the platform rocker assemblies 640, 642 may, alternatively, have a dedicated controller positioned on the base plate 872 beneath the rocking plate 878. As a result of force (or lack thereof)from the eccentric roller 876, the rocking plate 878 and culture vessel 704 supported thereon pivots about the fulcrum axis 873 of the fulcrum 872.

In an embodiment, each of the support posts 646 may be configured with a load cell for measurement of the mass of the culture vessel 704. Alternatively, or in addition, the base 870 of the rocker assembly 640 may include a plurality (e.g., three) load cells 882 that extend through the rocking plate 878 and engage the underside of the culture vessel 704 for measuring a mass of the culture vessel 704. As further shown in FIG. 67, the rocking plate 878 may be outfitted with a tilt sensor 884 that is configured to measure a degree of tilt of the rocking plate 878 (and thus culture vessel 704) for use by the controller in carrying out the rocking/mixing process.

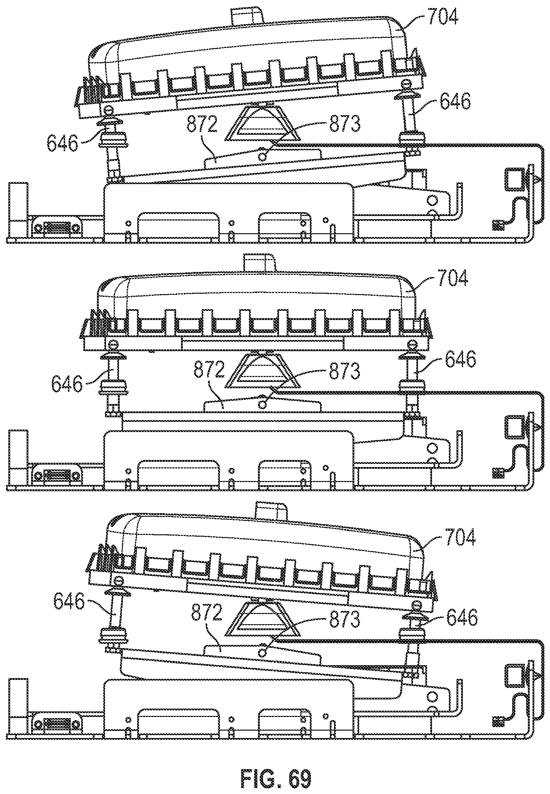

As indicated above, when the motor 874 is actuated, the eccentric circular roller 876 transmits a force against the bottom surface of the rocking plate 878, causing it move upwards or downwards depending on direction of rotation of the motor 874. When in constant operation, the circular profile of the eccentric circular roller 876 imparts a continuous sinusoidal rocking profile to the contents of the culture vessel 704. This rocking motion is illustrated in FIG. 69. The monitoring of rocking plate 878 using the tilt sensor 884 allows for closed loop control over the angle of tilt, homing and drain operations, as well detection of fault event conditions. The use of the support posts 646 to support the culture vessel 704 on the rocking plate 878 enables the entire bottom of the culture vessel 704 to remain unobstructed, which allows for better aeration, heat transfer and other functionalities, as discussed hereinafter. The use of the eccentric circular roller 876 allows for the tilting mechanism to be compact/low profile, and provides a low friction and highly reliable interface with the rocking plate 878. As will be appreciated, mammalian cells, in particular, are highly sensitive to the shear forces induced by small scale eddies on highly turbulent fluidic regimes. Therefore, strong vibrations, shocks or other mechanical stimulus leading to excessive turbulence, foam formation or spilling are potentially harmful. Accordingly, the continuous sinusoidal rocking profile of the platform rocker assemblies 640, 642 minimizes the presence of such small-scale eddies by removing any high frequency mechanical stimulus, providing for safer and more gentle mixing conditions, which is especially beneficial to mammalian cell cultures.

As indicated above, aeration and heat transfer through the base 764 and membrane 768 of the culture vessels 704, 706 is important for a variety of bioprocessing operations. Typically, certain cell cultures, e.g., mammalian cell cultures, must be surrounded by a sterile, homogeneous incubation atmosphere at the right temperature and CO₂ concentration for cell growth. The way such physio-chemical conditions are provided is dependent on the application, the cell type specificities and how well are they adapted to grow in suspension or adherence. In some cases, processes may require the cells to grow on a monolayer on top of a gas permeable membrane. In this case, heat and mass transfer takes place by passive diffusion based on the local gradients across the immediate regions at both side of the membrane. Embodiments of the invention optimize such phenomena by inducing a turbulent interaction between the gas permeable membrane 768 on the bottom of the culture vessel 704, 706 and an incubation atmosphere recirculation flow.

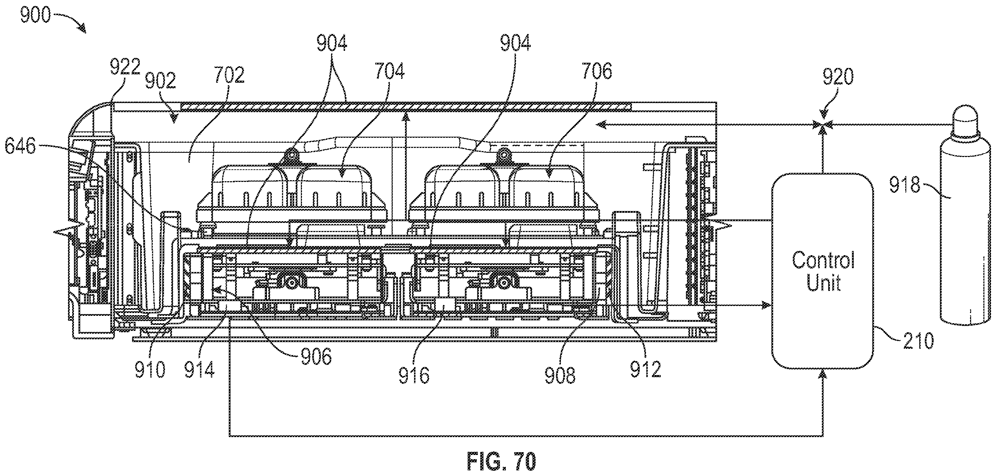

FIGS. 70-72 present cross-sectional views a portion of the process drawer 604 of the bioprocessing apparatus 600, with the tray 702 and culture vessels 704, 706 of the disposable bioprocessing kit 700 positioned therein. The process drawer 604 forms an incubation chamber 902 within which the tray 702 and culture vessels 704, 706 are positioned, as disclosed above. As shown therein, the culture vessels 704, 706 are supported by support posts 646 of the platform rocker assemblies 640, 642. Within the process drawer 604 are heating elements/devices 904 (e.g., positioned above and beneath each culture vessel). For example, the heaters 904 may be positioned beneath each culture vessel 704, 706, as well as adjacent to a top of the process drawer 604, for heating the incubation chamber 902 and culture vessels 704, 706. The process drawer 604 also includes a pair of fans or blowers 906, 908 within the cover 644 of the rocker assemblies 640, 642 adjacent to the front and back walls thereof. As further shown therein, the cover 644 may include a pair of opposed louvers or air passages 910, 912 near which the blowers 906, 908 are positioned, allowing for air to exit the space within the cover 644 (defining an incubation atmosphere recirculation chamber 915) adjacent to the rear of the process drawer 604 and reenter the recirculation chamber 915 from the front of the process drawer 604. A temperature sensor 914 and carbon dioxide sensor 916 are also positioned in at least one location along a recirculation air flow path, as discussed below, for measuring a temperature of the recirculation air flow and carbon dioxide concentration of the recirculation air flow. As additionally shown therein, a supply 918 of carbon dioxide is in selective fluid communication with the process drawer 604 (e.g., via the carbon dioxide inlet port on the rear of the housing 602 of the bioprocessing apparatus 600) and valve 920. The process drawer 604 also includes a gas port 922 which allows fluid communication between the interior of the process drawer 604 and the ambient air (obviating the need to have a dedicated, separate oxygen supply). The components described above form a system 900 for liquid to atmosphere direct mass transfer of the bioprocess system 600, the operation of which will be hereinafter described.

With further reference to FIG. 70, the temperature sensor 914 and carbon dioxide sensor 916 are electrically connected or otherwise in communication with a controller (e.g., master controller 210 of apparatus 600, although a dedicated controller for executing a recirculation air flow process is also envisioned) for receiving information regarding the temperature and carbon dioxide concentration of a recirculation air flow. The controller 210 is also electrically connected or otherwise in communication with the valve 920, the fans 906, 908 and heaters 904 for controlling operation thereof in response to the sensor readings and specified setpoints.

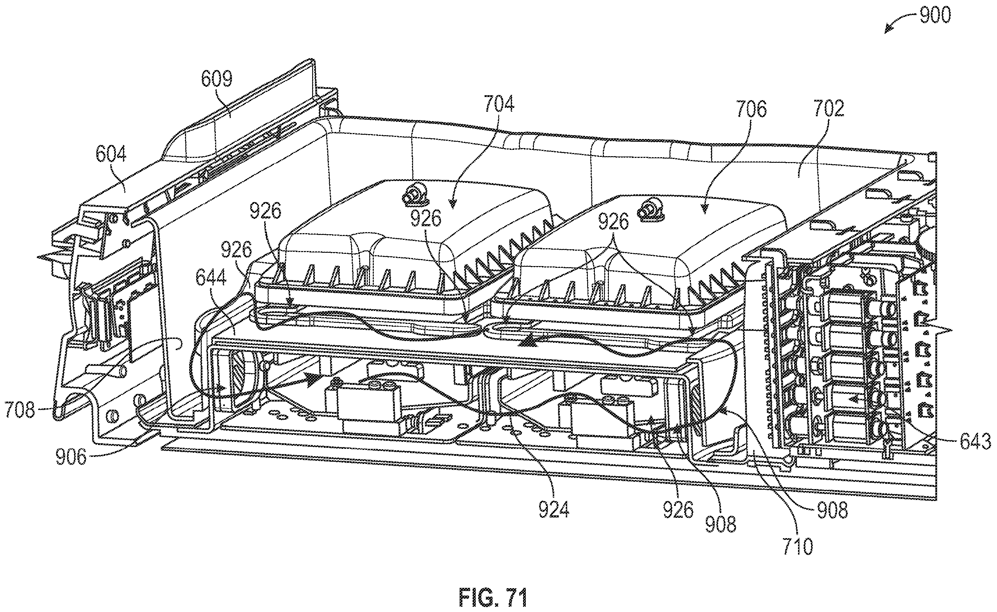

Referring now to FIG. 71 the controller 210 is operable to control the fans 906, 908 to produce a recirculation air flow 924. As discussed below, the tray 702 and process drawer 604 each include a variety of ducting features 926 that ensure that the recirculation air flow 924 exits the recirculation chamber 915 through the louver 912 adjacent to a rear of the process drawer 604, travels upwardly to a level of the culture vessels 704, 706, travels generally horizontally across the bottoms of the culture vessels 704, 706, travels downwardly near the front of the process drawer 604, and reenters the recirculation chamber 915 through louver 910. In this respect, the fan 908 pushes the recirculation air flow 924 outwardly from the recirculation chamber 915, while fan 906 draws the recirculation air flow 924 into the recirculation chamber 915.

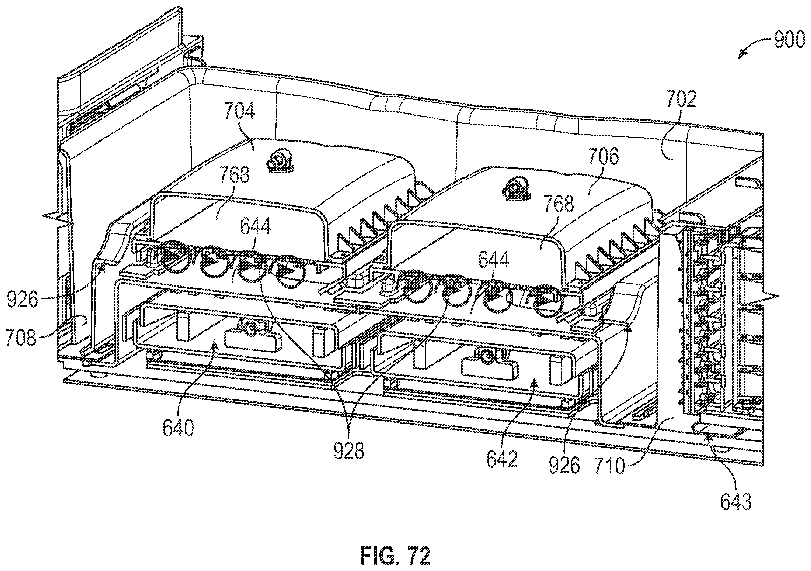

With reference to FIG. 72, the fan 908 pushes the incubation atmosphere through the incubation atmosphere recirculation chamber 915 and the ducting features 926 direct the recirculation air flow 924 across the bottom of the culture vessels 704, 706. In doing so, the ducting features and the configuration of the underside of the base 764 of the culture vessels 704, 706 induce the formation of local turbulences 928, which help to keep a constant oxygen and carbon dioxide supply in contact with the gas permeable membrane 768 of the culture vessels 704, 706.

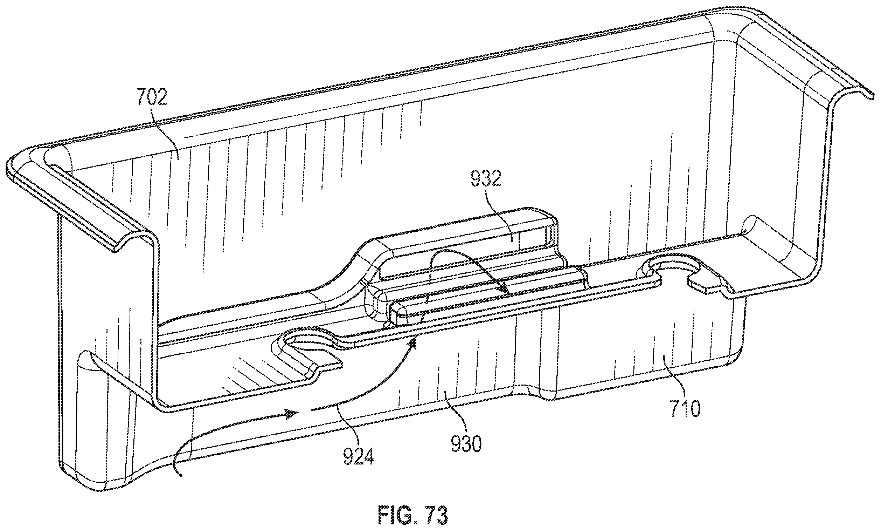

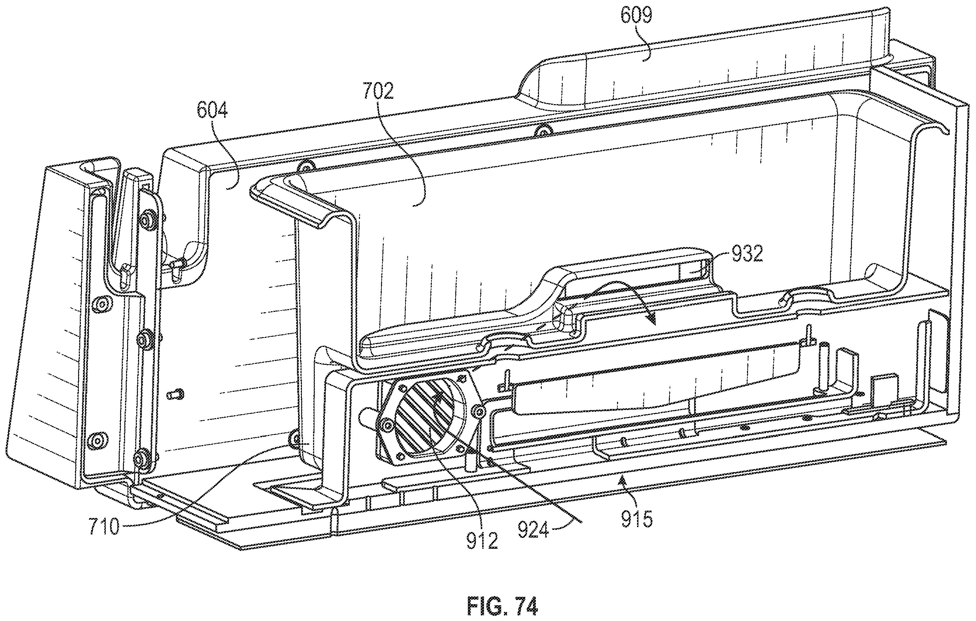

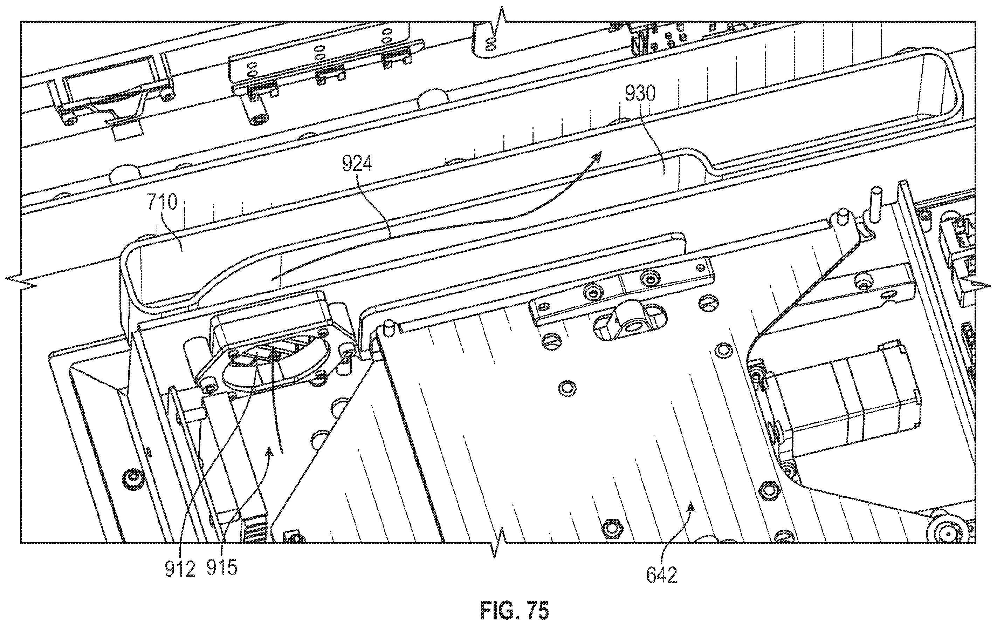

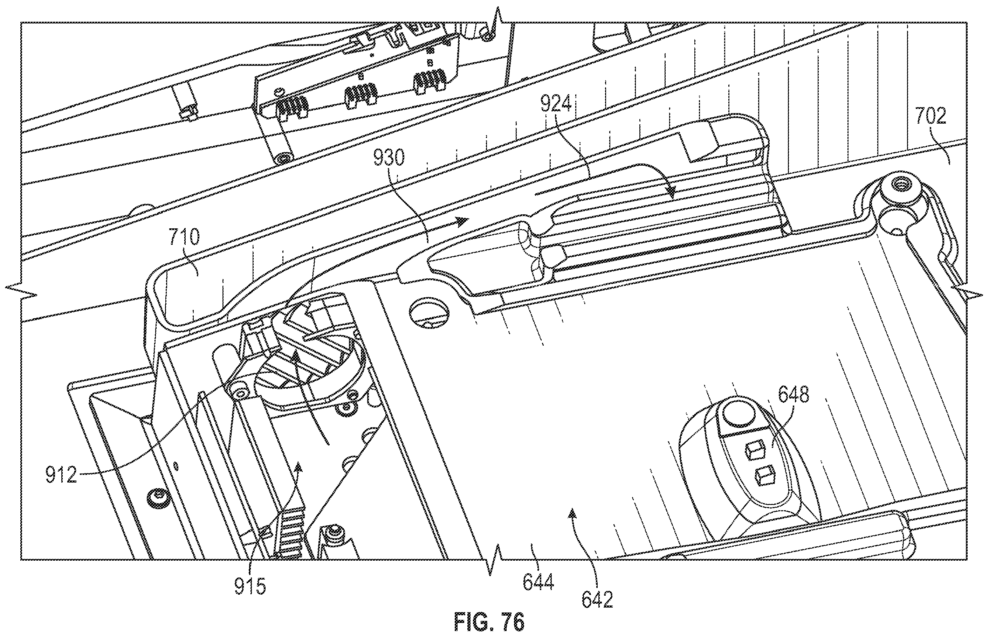

FIGS. 73-76 more clearly illustrate the ducting features of the system 900 that allow for the recirculation air flow 924 to be directed from the recirculation chamber 915, across the bottom of the culture vessels 704, 706, and back into the recirculation chamber 915 under influence from the fans 906, 908. As shown therein, the interior facing sides of the legs 708, 710 of the tray 702 are formed with indentations or recessed areas 930 which allow the recirculation air 924 exiting/entering the recirculation chamber 915 to travel upwardly or downwardly along the interior face of the legs 708, 710, as the case may be. FIGS. 73-76 illustrate, specifically, how the recirculation air 924 existing the recirculation chamber 915 is directed upwardly by the recessed area 930 of leg 710 of the tray 702. The recessed areas 930 of the legs 708, 710 and the exterior surface of the recirculation chamber therefore form vertical air passages for the flow of recirculation air 924. As the air exiting louver 912 travels upwardly within the recessed area 930 of the leg 710, it is impeded at a point where the leg 710 meets the bottom of the tray 702. As best shown in FIGS. 73 and 74, the tray 702 includes a pair of lateral vent openings 930 at a height that generally corresponds to a vertical height of the bottom of the culture vessels 704, 706. The vent openings 932 thus redirect the recirculation air flow 924 laterally through such openings 932 and towards the culture vessels 704, 706, where the recirculation air flow 924 interacts with the bottom geometry of the culture vessels 704, 706 and their corresponding gas permeable membranes, leading to the formation of local turbulences 928. The recirculation air flow 924 moves across the bottoms of the culture vessels 704, 706 where it enters the opposing vent opening, travels downwardly within the recessed area 930 of the leg 708, and reenters the recirculation chamber 915 through louver 910.

As disclosed above, the formation of local turbulences 928 in the recirculation air flow 924 help to keep a constant oxygen and carbon dioxide supply in contact with the gas permeable membrane 768 of the culture vessels 704, 706. At the same time, the overall recirculation air flow 924, along with the control action provided by the control unit 210, the temperature sensor 914, carbon dioxide sensor 916, heaters 904 and carbon dioxide control valve 920 allow for the homogenization of the volume inside the incubation chamber 902. The system 900 therefore provides for heat and mass transfer optimization. As will be appreciated, the constant availability of oxygen just some tens of microns away from cells monolayer supports higher cells concentrations and minimizes the physio-chemical gradients across the surface of the membrane 768 of the culture vessels 704, 706.

As hereinbefore described, the apparatus 600 includes a number of sensors and monitoring devices for monitoring a bioprocessing operation as it is carried out, including monitoring various parameters of a cell culture within the culture vessels 704, 706. This may include, for example, periodically drawing a sample from the culture vessels 704, 706 using the sampling tubing tails 748 of the sampling card 722 and/or using sensors to sense various parameters of the culture within the vessels. For example, sensor assembly 648 houses the IR sensor for temperature measurement and for detecting the presence of the culture vessel within the process drawer. The window 796 in the base 764 of the culture vessel 704, 706 enables IR-based temperature measurement of the membrane in the culture vessels, and thus the liquid temperature within the culture vessels.

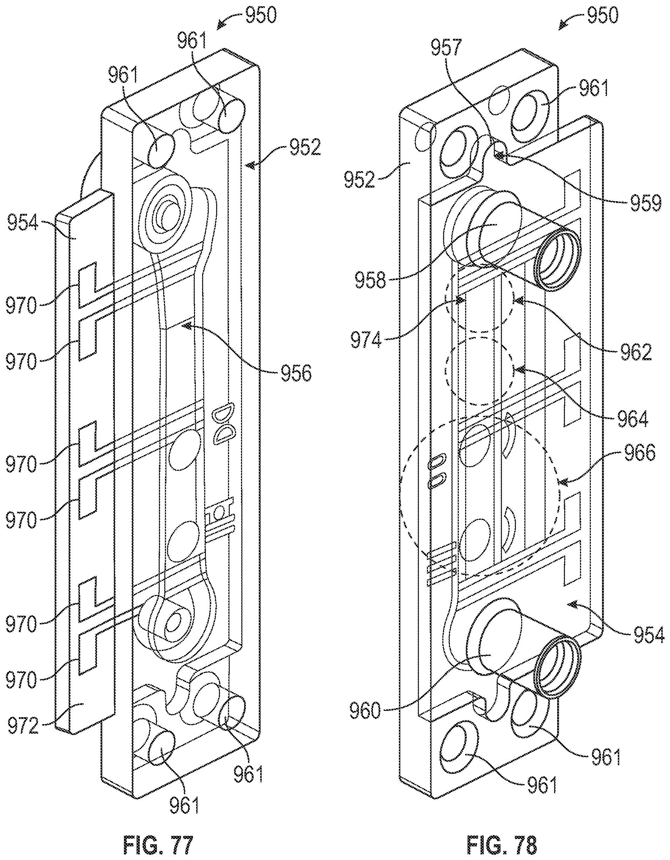

With reference to FIGS. 77-84, in an embodiment, the apparatus 600 may additionally include a flow-through sensing chamber 950 (also referred to herein as flow-through sensing apparatus 950) which may be utilized to measure or monitor various parameters of a fluid within the apparatus 600 (e.g., the culture(s) within the culture vessels 704, 706) utilizing a variety of different sensing/measuring devices, and without withdrawing any fluid from the system. As best shown in FIGS. 77-80, the flow-through sensing chamber 950 includes a first plate 952, a second plate 954 connected in facing relation to the first plate 952, and a fluidic channel 956 intermediate the first plate 952 and the second plate 954. In an embodiment the fluidic channel 956 is formed from a relieved area on an interior face of at least one or both of the first plate 952 and/or the second plate 954. In an embodiment, the fluidic channel 956 may be between about 0.1mm to about 1mm in height. The chamber 950 further includes a first port 958 in fluid communication with the fluidic channel 956 for facilitating the flow of a fluid into the chamber 950 and the fluidic channel 956 thereof, and a second port 960 in fluid communication with the fluidic channel 956 for facilitating the flow of the fluid out of the chamber 950 and the fluidic channel 956 thereof. In an embodiment, the ports 958, 960 are in fluid communication with opposing ends of the fluidic channel 956.

As shown in FIG.78, in an embodiment, the plates 952, 954 may have features that facilitate alignment and coupling of the plates with one another. For example, one of the plates (e.g., plate 952) may have a pair of notches 957 that receive corresponding tabs 959 of the other of the plates (e.g., plate 954). As discussed below the back plate/first plate 952 includes a plurality of mounting and positioning holes 961 that extend therethrough, which facilitate mounting of the chamber 950 to the tray 702 of the disposable kit 700. In an embodiment, the first/back plate 952 and second/front plate 954 are generally rectangular in shape, are transparent, and are manufactured from biocompatible plastic, glass or a combination of plastic and glass, although the invention is not intended to be so limited in this regard.

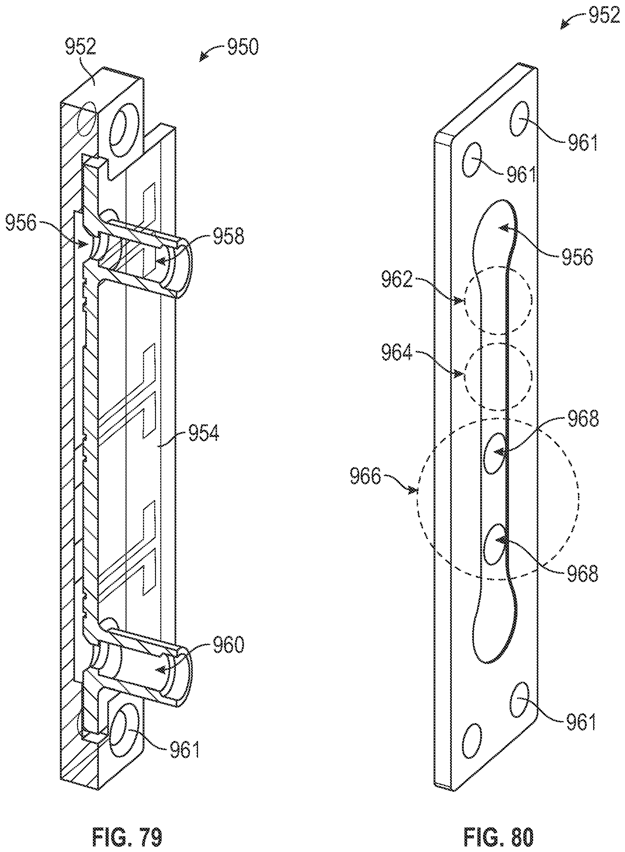

As best shown in FIGS. 78 and 79, the fluidic channel 956 includes a plurality of segments or sensing locations 962, 964, 966 which permit or facilitate interrogation of the fluid or monitoring of the fluid within the fluidic channel 956 with a plurality of sensing devices and techniques. In an embodiment, the fluid within the fluidic channel 956 may be interrogated with a variety of different sensing devices associated with a respective one of the plurality of sensing locations 962, 964, 966. In an embodiment, the segment 966 has one or more sensors 968 located within the fluidic channel 956 and which are configured to remain in continuous contact with the fluid passing through the fluidic channel 956. As shown in FIG. 77, the second plate 954 includes a plurality of electrodes 970 that extend into the fluidic channel 956 and are accessible from a laterally-extending flange 972 of the second plate 954. In an embodiment, the electrodes 970 are gold-plated electrodes.

As indicated above, the flow-through sensing chamber 950 permits interrogation of a fluid within the fluidic channel 956 utilizing a variety of different sensing devices, for measuring a variety of different parameters of the fluid. For example, in an embodiment, the sensing location 962 may be configured as a reflected light interrogation segment, configured with a gold plated mirror 974 behind the fluidic channel 956 that reflects light emitted by a sensing device. The sensing location 962 may therefore be suitable for a variety of techniques for monitoring/sensing of biological variables such as, for example, optical density sensing, turbidimetry, digital holographic microscopy, light dynamic scattering and/or optical interferometry, etc. In an embodiment, sensing location 964 may be configured as a transmitted and backscattered light interrogation segment that allows for interrogation of the fluid within the fluidic channel 956 using a transmitted or backscattered light sensing instrument. Sensing location 966, for its part, may be configured as a fluorescence sensor interrogation segment having a variety of sensors 968 in contact with the fluid within the fluidic channel 956 allows for monitoring or sensing of a variety of parameters of the fluid such as, for example, dissolved oxygen, pH, carbon dioxide, analytes, etc.). The electrodes 970 face rearwardly (opposite the ports 958, 960) and are configured to be contacted by spring-biased pins of one or more measuring devices suitable for a variety of electrochemical measurement techniques such as, for example, electrical impedance spectroscopy, galvanometry, amperometry and/or polarography, etc.

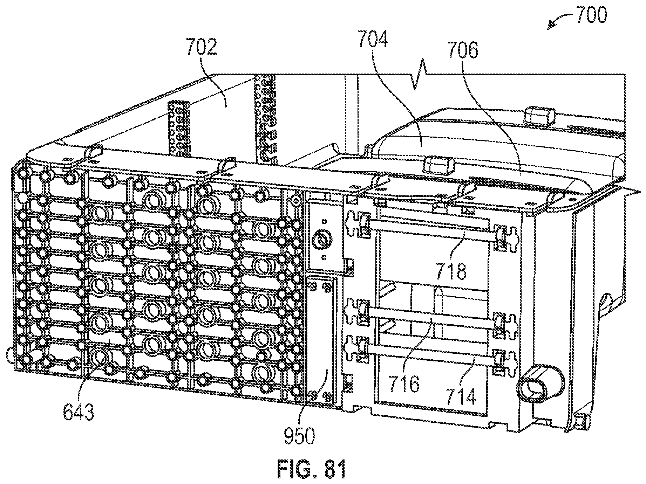

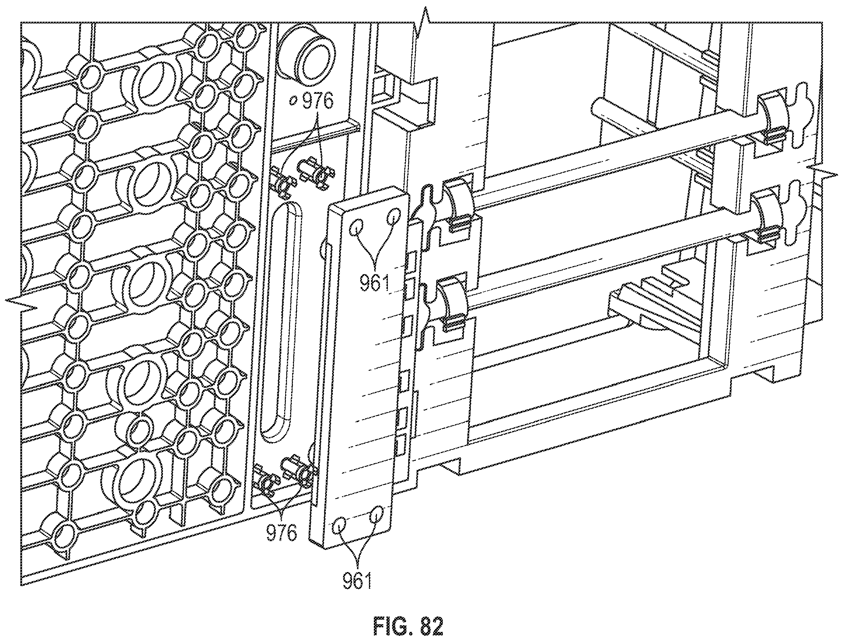

FIGS. 81 and 82 illustrate the positioning of the flow-through sensing chamber 950 on the backbone of the tray 702 of the disposable bioprocessing kit 700. As indicated above, the chamber 950 may be connected to the tray 702 by receiving snap pins 976 located on the backbone of the tray 700 within the corresponding mounting apertures 961 of the chamber 950. As shown therein, in an embodiment, the chamber 950 may be mounted to the tray 700 intermediate the valve manifold 712 and the peristaltic pump tubing segments 714, 716, 718. While pins 976 are illustrated as being utilized to mount the chamber 950 to the tray 700, it is contemplated that other connection means such as, for example, clipping, clamping, fasteners, snap-fitting, press-fitting and the like may also be utilized without departing from the broader aspects of the disclosure. In an embodiment, the chamber 950 may form part of the disposable bioprocessing kit 700.

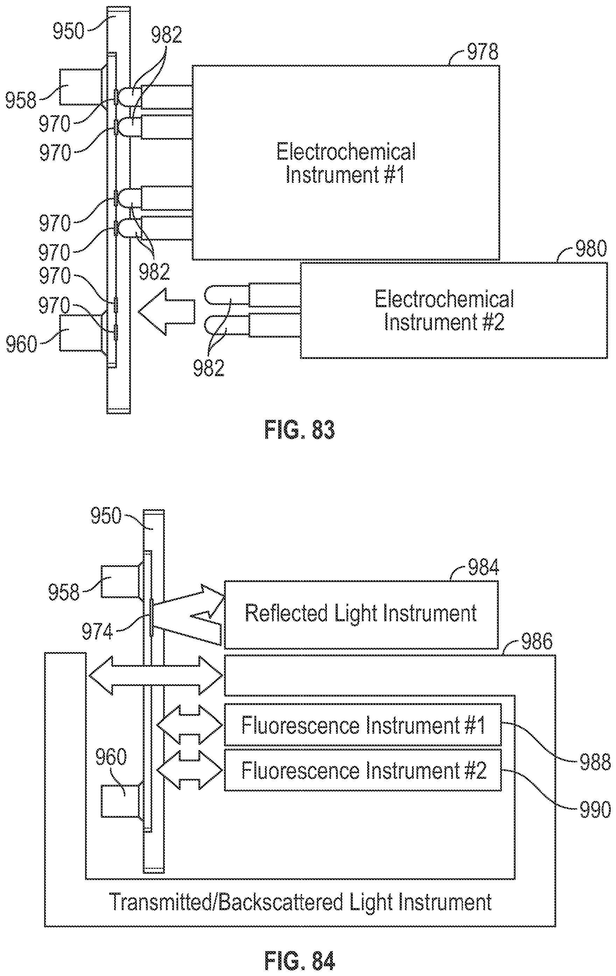

FIGS. 83 and 84 present schematic illustrations of the flow-through sensing chamber 950 and various sensing instruments/devices for monitoring various parameters of the fluid within the fluidic channel 956. As shown in FIG. 83, for example, first and second electrochemical sensing instruments 978, 980 on-board the apparatus 600 may interface with the electrodes 970 via spring-biased pins 982. As shown in FIG. 84, a reflected light instrument 984 may be positioned and configured to interrogate the fluid within the first sensing location, first and second fluorescence instruments 986, 988 positioned and configured to interrogate the fluid with the second sensing location 964, and a transmitted/backscattered light instrument 990 positioned and configured to interrogate the fluid within the third sensing location 966.

Disclosed embodiments therefore provide for an in-line sensing chamber 950 that provides for a variety of optical and electrical measurements of fluid within the fluidic channel 956 of the chamber 950, obviating any need to directly interrogate either of the culture vessels 704, 706. In use, when it is desired to monitor or measure various parameters of the culture within either of the culture vessels 704, 706, the fluid is pumped through the sensing chamber 950 using the peristaltic pump assembly 641, where is can be interrogated by a suite of sensor instruments/devices. That is, the chamber 950 disclosed herein facilitates the use of electrochemical and optical sensing techniques on a single fluidic channel allowing for multiparametric monitorization of the physio-chemical growth conditions, cell species metabolic activity (lactate acid, glucose, etc.) and viable cell density and total cell count measurements of the cell culture within the culture vessels 704, 706.

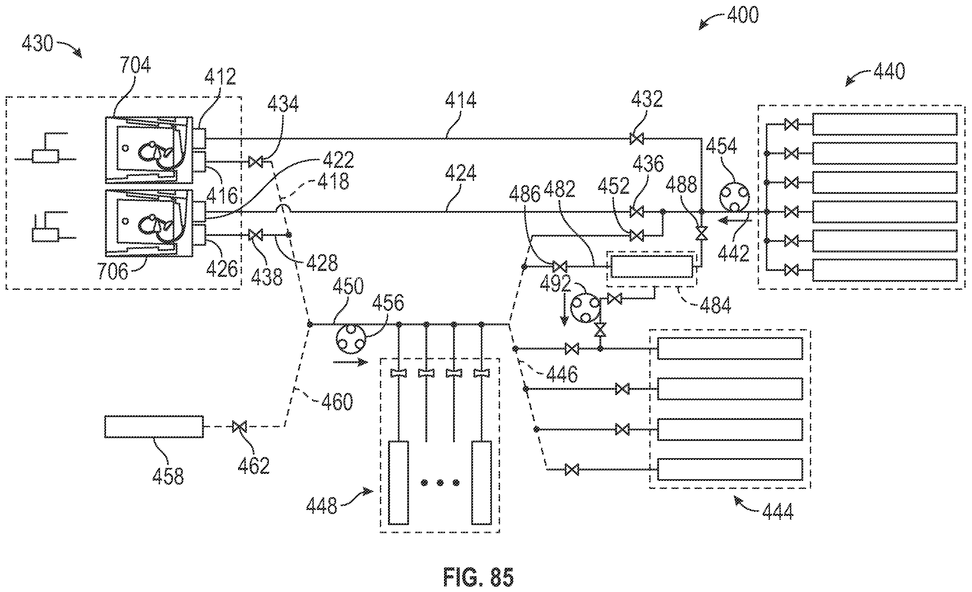

Having disclosed in detail the components of the bioprocessing apparatus 600 (also referred to as second module 200), the fluid flow architecture or system 200 embodiment within the apparatus 600 is illustrated with reference to FIGS. 85-89. As disclosed above, and as described in more detail hereinafter, the configuration of the bioprocessing apparatus 600 and kit 700, along with the fluid flow architecture 200 provided thereby, allows for cell activation, genetic modification and expansion of cellular products, and ancillary or related protocols, workflows and methods, in an automated and functionally closed manner. In an embodiment, the flow architecture or system 400 may be configured or arranged as disclosed in FIGS. 3-7 of WIPO International Publication No. WO 2019/106207, although other configurations are also possible. As illustrated in FIG. 85, the system 400 includes a first bioreactor vessel (e.g. culture vessel 704) and a second bioreactor vessel 420 (e.g., culture vessel 706). The first bioreactor vessel includes at least a first port 412 and a first bioreactor line 414 in fluid communication with the first port 412, and a second port 416 and a second bioreactor line 418 in fluid communication with the second port 416. Similarly, the second bioreactor vessel includes at least a first port 422 and a first bioreactor line 424 in fluid communication with the first port 422, and a second port 426 and a second bioreactor line 428 in fluid communication with the second port 426. Together, the first bioreactor vessel 410 and second bioreactor vessel 420 form a bioreactor array 430. While the system 400 is shown as having two bioreactor vessels, disclosed embodiments may include a single bioreactor or more than two bioreactor vessels.

The first and second bioreactor lines 414, 418, 424, 428 of the first and second bioreactor vessels 410, 420 each include a respective valve for controlling a flow of fluid therethrough, as discussed hereinafter. In particular, the first bioreactor line 414 of the first bioreactor vessel 410 includes a first bioreactor line valve 432, while the second bioreactor line 418 of the first bioreactor vessel 410 includes a second bioreactor line valve 424. Similarly, the first bioreactor line 424 of the second bioreactor vessel 420 includes a first bioreactor line valve 436, while the second bioreactor line 428 of the second bioreactor vessel 420 includes a second bioreactor line valve 438.

With further reference to FIG. 85, the system 400 also includes a first fluid assembly 440 having a first fluid assembly line 442, a second fluid assembly 444 having a second fluid assembly line 446, and a sampling assembly 448. An interconnect line 450 having an interconnect line valve 452 provides for fluid communication between the first fluid assembly 440 and the second fluid assembly 444. As shown in FIG. 85, the interconnect line 450 also provides for fluid communication between the second bioreactor line 418 and first bioreactor line 414 of the first bioreactor vessel 410, allowing for circulation of a fluid along a first circulation loop of the first bioreactor vessel. Similarly, the interconnect line also provides for fluid communication between the second bioreactor line 428 and first bioreactor line 424 of the second bioreactor vessel 420, allowing for circulation of a fluid along a second circulation loop of the second bioreactor vessel. Moreover, the interconnect line 450 further provides for fluid communication between the second port 416 and second bioreactor line 418 of the first bioreactor vessel 410, and the first port 422 and first bioreactor line 424 of the second bioreactor vessel 420, allowing for the transfer of contents of the first bioreactor vessel 410 to the second bioreactor vessel 420, as discussed hereinafter. As illustrated in FIG. 85, the interconnect line 450, in an embodiment, extends from the second bioreactor lines 418, 428 to the intersection of the first bioreactor line 414 of the first bioreactor vessel 410 and the first fluid assembly line 442.

As illustrated by FIG. 85, the first and second fluid assemblies 440, 450 are disposed along the interconnect line 450. Additionally, in an embodiment, the first fluid assembly is in fluid communication with the first port 412 of the first bioreactor vessel 410 and the first port of the second bioreactor vessel 420 through the first bioreactor line 414 of the first bioreactor vessel and the first bioreactor line 424 of the second bioreactor vessel 420, respectively. The second fluid assembly 444 is in fluid communication with the second port 416 of the first bioreactor vessel 410 and the second port 426 of the second bioreactor vessel 420 via the interconnect line 450.

A first pump 454 of the peristaltic pump assembly 641 capable of providing for bidirectional fluid flow is disposed along the first fluid assembly line 442, and a second pump or circulation line pump 456 of the peristaltic pump assembly 641capable of providing for bidirectional fluid flow is disposed along the interconnect line 450, the function and purpose of which will be discussed below. As also shown in FIG. 85, a sterile air source 458 is connected to the interconnect line 450 through a sterile air source line 460. A valve 462 positioned along the sterile air source line 460 provides for selective fluid communication between the sterile air source 458 and the interconnect line 450. While FIG. 85 shows the sterile air source 458 connected to the interconnect line 450, in other embodiments the sterile air source may be connected to the first fluid assembly 440, the second fluid assembly 444, or the fluid flow path intermediate the second bioreactor line valve and the first bioreactor line valve of either the first bioreactor or the second bioreactor, without departing from the broader aspects of the disclosure.

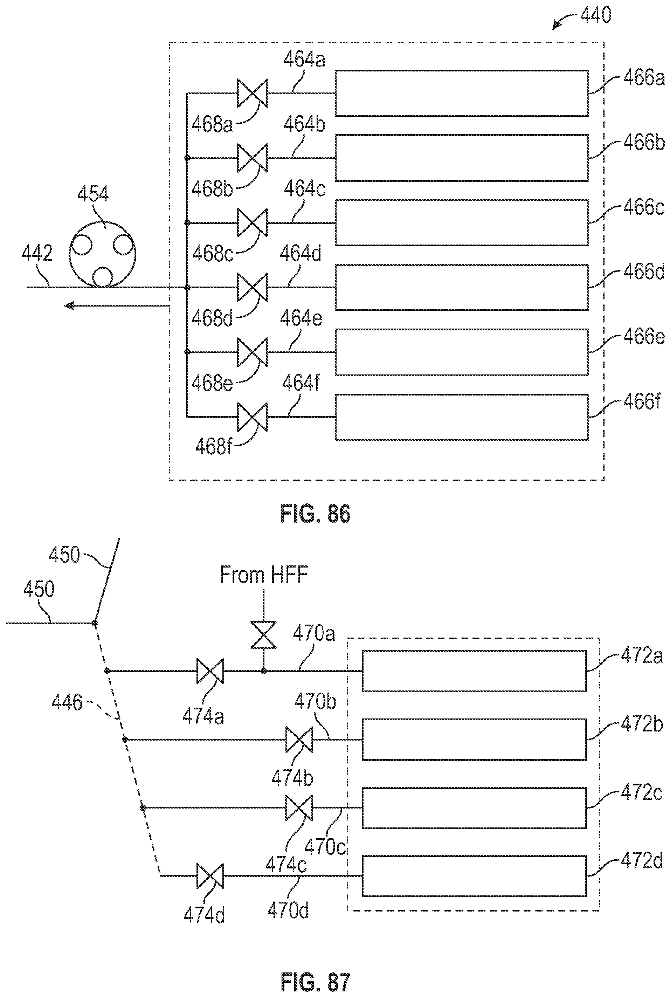

With additional reference now to FIGS. 86-88, detailed views of the first fluid assembly 440, second fluid assembly 444 and sampling assembly 448 are shown. With specific reference to FIG. 86, the first fluid assembly 440 includes a plurality of tubing tails 464*a*-*f*, each of which is configured for selective/removable connection to one of a plurality of first reservoirs 466*a*-*f.* Each tubing tail 464*a*-*f* of the first fluid assembly 440 includes a tubing tail valve 468*a*-*f* for selectively controlling a flow of fluid to or from a respective one of the plurality of first reservoirs 466*a-f* of the first fluid assembly 440. While FIG. 86 specifically shows that the first fluid assembly 440 includes six fluid reservoirs, more or fewer reservoirs may be utilized to provide for the input or collection of various processing fluids, as desired. It is contemplated that each tubing tail 464*a-f* may be individually connected to a reservoir 466*a-f,* respectively, at a time required during operation of fluid assembly 440, as described below.

With specific reference to FIG. 87, the second fluid assembly 444 includes a plurality of tubing tails 470*a-d,* each of which is configured for selective/removable connection to one of a plurality of second reservoirs 472*a-d* Each tubing tail 470*a-d* of the second fluid assembly 444 includes a tubing tail valve 474*a-e* for selectively controlling a flow of fluid to or from a respective one of the plurality of second reservoirs 472*a-d* of the first fluid assembly 444. While FIG. 87 specifically shows that the second fluid assembly 444 includes four fluid reservoirs, more or fewer reservoirs may be utilized to provide for the input or collection of various processing fluids, as desired. In an embodiment, at least one of the second reservoirs, e.g., second reservoir 472d is a collection reservoir housed within the cabinet 608 of the apparatus 600 for collecting an expanded population of cells, as discussed hereinafter. In an embodiment, the second reservoir 472*a* is a waste reservoir or bag housed within the waste drawer 606 of the apparatus 600, the purpose of which is discussed below.

In an embodiment, the first reservoirs 466*a-f* and the second reservoirs 472*a-d* are single use/disposable, flexible bags housed within the cabinet 608 of the apparatus 600 and fluidly connected to the manifold 712 via tubing tails of the tubing organizer 720. In an embodiment, the bags are substantially two-dimensional bags having opposing panels welded or secured together about their perimeters and supporting connecting conduit for connection to its respective tail, as is known in the art.

In an embodiment, the reservoirs/bags may be connected to the tubing tails of the first and second tubing assembly using a sterile welding device. In an embodiment, a welding device can be positioned next to the apparatus 600, and the welding device utilized to splice-weld one of the tubing tails to tail to the tube on the bag (while maintaining sterility). Thus the operator can provide the bag at the time it is needed (e.g., by grabbing a tubing tail from the tubing organizer 720 and inserting its free end into the welding device, laying the bag tube's free end adjacent to the end of the tubing tail, cutting the tubes with a fresh razor blade, and heating the cut ends as the razor is pulled away while the two tube ends are forced together while still melted so that they resolidify together). Conversely, a bag can be removed by heat sealing the line from the bag and cutting at the heat seal to separate the two closed lines. Accordingly, the reservoirs/bags may be individually connected when desired, and the present disclosure does not require that all reservoirs/bags must be connected at the beginning of a protocol, as an operator will have access to the appropriate tubing tails during the entire process to connect a reservoir/bag in time for its use. Indeed, while it is possible that all reservoirs/bags are pre-connected, the disclosure does not require pre-connection, and one benefit of the second module 200 is that it allows the operator to access the fluid assemblies/lines during operations so that spent bags may be connected in a sterile manner, and disconnected so that other bags can be sterilely connected during a protocol, as discussed below.

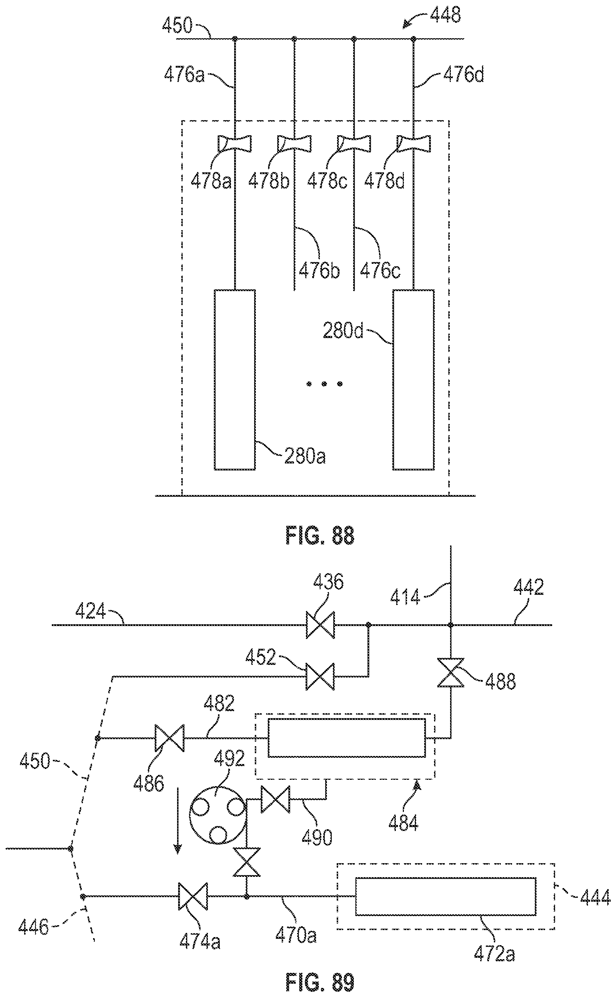

As illustrated in FIG. 88, the sampling assembly 448 includes one or more sampling lines, e.g., sampling lines 476*a-*476*d* (which may be sampling tubing tails 748 of the sampling card 722), fluidly connected to the interconnect line 450. Each of the sample lines 476*a-*476*d* may include a sample line valve 478*a-d* that is selectively actuatable to allow fluid to flow from the interconnect line 450 through the sample lines 476*a-*476*d.* As also shown therein, a distal end of each sampling line 476*a-*476*d* is configured for selective connection to a sample collection device (e.g., sample collection devices 280*a* and 280*d)* for collection of the fluid from the interconnect line 450. The sample collection devices may take the form of any sampling device known in the art such as, for example, a syringe, dip tube, bag, etc. While FIG. 88 illustrates the sampling assembly 448 being connected to the interconnect line, in other embodiments the sampling assembly may be fluidly coupled to the first fluid assembly 440, the second fluid assembly 444 a fluid flow path intermediate the second bioreactor line valve 434 and the first bioreactor line valves 432 of the first bioreactor vessel 410, and/or a fluid flow path intermediate the second bioreactor line valve 438 and the first bioreactor line valve 436 of the second bioreactor vessel 420. The sampling assembly 448 provides for fully functionally-closed sampling of a fluid at one or more points in the system 400, as desired.

Referring back to FIG. 85, in an embodiment, the system 400 may also include a filtration line 482 that is connected at two points along the interconnect line 450 and defines a filtration loop along the interconnect line 450. A filter 484 is positioned along the filtration line 482 for removing permeate waste from a fluid passing through the filtration line 482. As shown therein, the filtration line 482 includes an upstream filtration line valve 486 and a downstream filtration line valve 488 positioned on the upstream and downstream side of the filter 484, respectively. A waste line 490 provides fluid communication between the filter 484 and the second fluid assembly 444 and, in particular, with tubing tail 470*a* of the second fluid assembly 444, which is connected to the waste reservoir 472*a.* In this respect, the waste line 490 conveys waste removed from the fluid passing through the filtration line 482 by the filter 484 to the waste reservoir 472*a.* As illustrated in FIG. 85, the filtration line 482 surrounds the interconnect line valve 452 so that a flow of fluid through the interconnect line 450 can be forced through the filtration line 482, as discussed hereinafter. A permeate pump 492 positioned along the waste line 490 is operable to pump the waste removed by the filter to the waste reservoir 472*a.* In an embodiment, the filter 484 is desirably an elongate hollow fiber filter, although other tangential-flow or cross-flow filtration means known in the art such as, for example, a flat sheet membrane filter, may also be utilized without departing from the broader aspects of the disclosure.

In an embodiment, the valves of the first fluid assembly 440 and second fluid assembly 444, as well as the bioreactor line valves (i.e., valves 432, 434, 436, 438, sterile line valve 462, interconnect line valve 452 and filtration line valves 486, 488 are formed by engagement of one of the linear actuators of the linear actuator array 643 with the valve manifold 712 to block or allow a particular flow of fluid therethrough. In an embodiment, operation of the valves and pumps disclosed above (i.e., the linear actuators of the linear actuator array 643 and the three peristaltic pumps 454, 456, 492 of the peristaltic pump assembly 641) is automatically carried out according to a programmed protocol so as to enable proper operation of module 200/apparatus 600. It is contemplated that second controller 210 on board the second module 200/apparatus 600 may direct the operation of these valves (linear actuators) and pumps.

As indicated above, the bioprocessing apparatus 600, in combination with disposable bioprocessing kit 700 is configured to carry out activation, transduction and expansion phases of cell processing. In an embodiment, the activation phase contains six steps, each of which includes a plurality of user-controllable/selectable parameters and is executed by the controller 210. During the activation phase, two pre-seeding reagents and two post-seeding regents can be used. The cellular input to the activation phase are cells which are ready to undergo activation. Following activation it is possible to concentrate and wash the cells to remove any residual reagent components that are undesired for the subsequent process steps. The transduction phase, likewise, contains six steps, each of which includes a plurality of user-controllable/selectable parameters and is executed by the controller 210. During the transduction phase, two pre-seeding reagents and two post-seeding reagents can be used. The cellular input to the transduction phase are the cells that have been activated in the previous phase. Following transduction it is possible to concentrate and wash the cells to remove any residual reagent components that are undesired for the subsequent process steps. The expansion phase, for its part, contains three steps (seeding, cell culture and harvest), each of which includes a plurality of user-controllable/selectable parameters and is executed by the controller 210. During seeding step, the system will add media in the transduction vessel to dilute the contents to the desired cells density for expansion. During the cell culture step, the user can select the sampling frequency and define the feeding strategies used to expand the cells within the culture vessels 704, 706. During the harvest step, the harvest can be performed at either a preset time point or initiated by the user once the target cell dose is achieved.

In an embodiment, among the parameters that can be controlled or selected by a user are pre- and post-seeding reagent parameters, input cell volume, incubation, volume reduction, wash, target seeding and cell culture. The pre- or post-seeding reagents step contains parameters for up to two reagents that can be added to the culture vessel before seeding the cells and for up to two reagents that can be added to the culture vessel after seeding the cells. Prior to transferring the reagent to the culture vessel, the user may transfer air or liquid through the system. After incubation of the reagent, the culture vessel can be rinsed before seeding the cells. The input cells volume parameter defines the parameters to add the source cells into the culture vessel. Prior to adding the cells to the culture vessel, the user can manually mix the cells in the source bag. Additionally, the source bag can be rinsed to maximize the transfer of the input cells. The incubation parameter defines the parameters during incubation of the cells within the culture vessels 704, 706. The user can set the target seeding density and the volume for activation, as well as sampling-related parameters. The volume reduction parameter defines the parameters to concentrate the cells after activation. The cells are concentrated using either the hollow-fiber filter (HFF) or via volume reduction by skimming off the liquid without disturbing the cells, and sucking the liquid out of the culture vessel (i.e., perfusion without adding media to the inlet such that the volume within the culture vessel decreases), also referred to as high-speed perfusion (HSP).

The wash parameter defines the parameters for washing the cells after volume reduction, in order to prepare them for transduction. The cells are washed using either the hollow-fiber filter (HFF) or high-speed perfusion (HSP). In an embodiment, the HSP wash protocol includes the following process steps: 1) initial settling phase - the activation vessel remains stable for a fixed amount of time to allow cells to settle down on the vessel membrane; 2) optionally enable a very slow activation vessel mix to enhance supernatant homogeneity without disturbing the cells settling; 3) simultaneous media addition and supernatant removal while maintaining the activation vessel volume stable; 4) in the middle of the wash duration, optionally enable a very slow activation vessel mix to enhance supernatant homogeneity without disturbing the cells settling; 5) simultaneous media addition and supernatant removal while maintaining the activation vessel volume stable until wash target duration is elapsed or wash target media volume is consumed; 6) optionally dilute activation vessel contents with media up to target vessel volume; 7) optionally enable a very slow activation vessel mix to enhance supernatant homogeneity without disturbing the cells settling; and 8) low flow-rate removal of the supernatant without disturbing the cells settling up to target activation vessel volume.

In an embodiment, for the transduction phase, the steps are similar to the activation phase description provided above. In an embodiment, a transfer cells parameter is provided which defines the parameters to transfer the activated cells from the activation vessel into the transduction vessel. Prior to transferring the cells to the culture vessel, the system can mix the cells in the activation vessel. A portion or the entire contents of the activation vessel can be transferred to the transduction vessel. Additionally, the activation vessel can be rinsed to maximize the transfer cells

Finally, the target seeding general parameter defines the parameters to set the starting conditions for the cells during expansion. The cell culture parameter defines the feeding strategy used to culture the cells during expansion. The user can define feeding periods based on user-configurable parameters. Exemplary feeding strategies include single-shot media addition (fed-batch) or continuous media addition (perfusion). The harvest parameter defines the parameters that enable cell harvesting. The user can define the volume of cells to harvest and either initiate the harvest at a defined time point or when desired. As will be appreciated selection and setting of these parameters can be carried out using the interface 609, or through and off-board user interface or terminal that is in communication with the apparatus 600 (e.g., through the data ports on the back of the apparatus 600, although wireless communication means are also possible.

As indicated above, the apparatus 600 and flow architecture 400 also allows for sampling of the contents of the culture vessel(s) 704, 706 using, for example, the sampling tubing tails 748 of the sampling card 722.

In an embodiment, a sampling sequence includes tilting platform rocker assembly 640, 642 to mix and to homogenize the contents in the culture vessel (mixing speed dependent on vessel volume), actuate process pump 456 to circulate vessel contents from vessel outlet port (416 or 426) in the sampling tubing and back to the inlet port of the vessel (412 or 422), prompt a user to take a sample, stop circulation and mixing and, finally, sampling tubing clearing.

In an embodiment, the use of two culture vessels 704, 706 within the processing drawer 604 of the apparatus allows for parallel processing to be carried out in the manner disclosed below. In an embodiment, all activation steps may be carried out in the first culture vessel 704, after which the cells are transferred to the second culture vessel 706 where transduction and expansion of the cells are carried out. In another embodiment, during activation in the first culture vessel 704, transduction reagent actions can be carried out in the second culture vessel 706 (e.g., adding pre-seeding reagent(s) to the second culture vessel 706, incubating, and rinsing the culture vessel 706) before adding the post-activation cells from the first culture vessel 704 to the second culture vessel 706 for transduction and expansion steps. In another embodiment, activation, transduction and expansion steps can be carried out in a single culture vessel (e.g., the first or second culture vessel 704, 706).

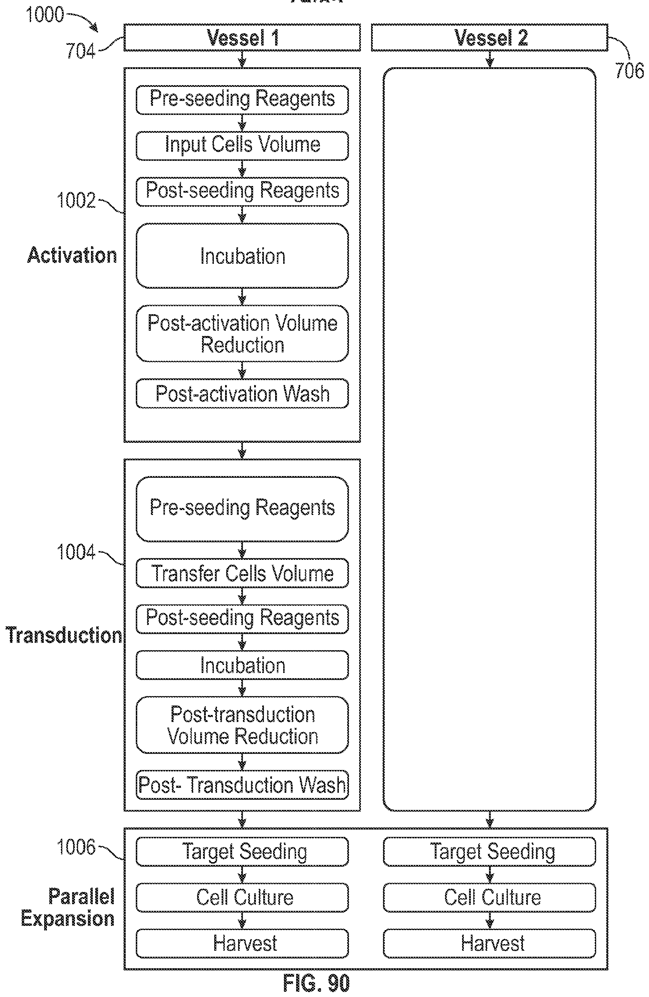

With reference to FIG. 90 another workflow 1000 enabled by the bioprocessing apparatus 600 is illustrated. As shown therein, the workflow or method 1000 includes carrying out the series of activation steps 1002 and the series of transduction steps 1004 in the first culture vessel 704, and expanding the population of genetically modified cells (post-transduction) in a parallel expansion step 1006 using both the first and second culture vessels 704, 706. This involves transferring a fraction of the genetically modified cells from the first culture vessel 704 to the second culture vessel so that parallel expansion 1006 can be carried out using both culture vessels 704, 706, simultaneously.

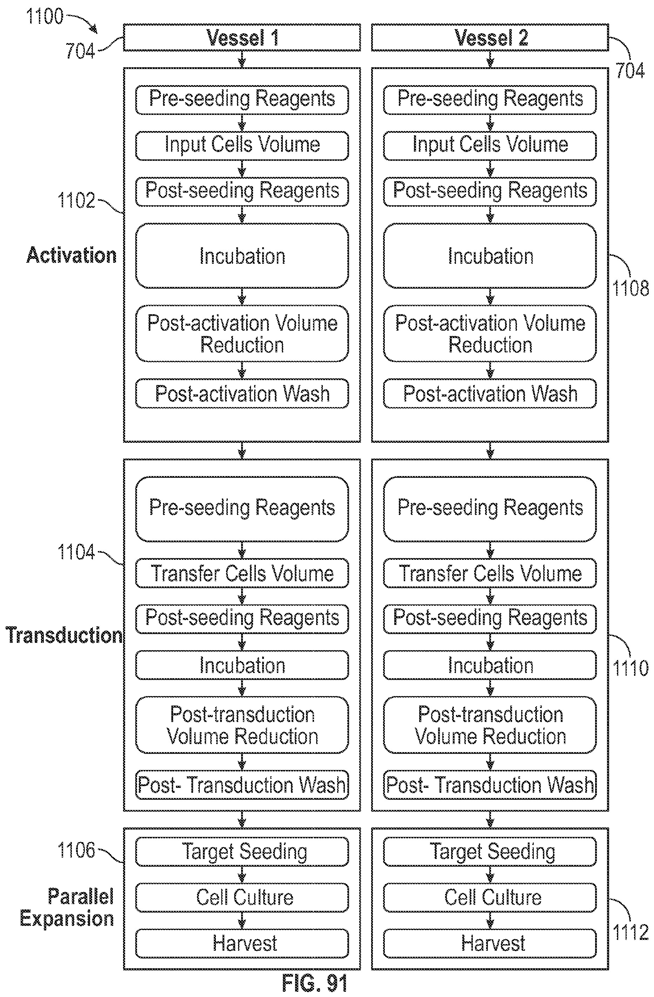

With reference to FIG. 91, yet another workflow 1100 enabled by the bioprocessing apparatus 600 is illustrated. As shown therein, the workflow or method 1100 includes carrying out the steps of activation, transduction and expansion in parallel, but independent, workflows. This includes, for example, carrying out activation steps 1102, transduction steps 1104, and expansion steps 1106 for a first population of cells entirely within the first culture vessel 704, and carrying out parallel activation steps 1108, transduction steps 1110 and expansion steps 1112 for a second population of cells entirely within the second culture vessel 706. In an embodiment, the first and second population of cells may be sourced from a single cell population that is split between the first and second culture vessels 704, 706 during the input stage of the activation phase. In another embodiment, the first and second population of cells may be different (e.g., come from different sources).

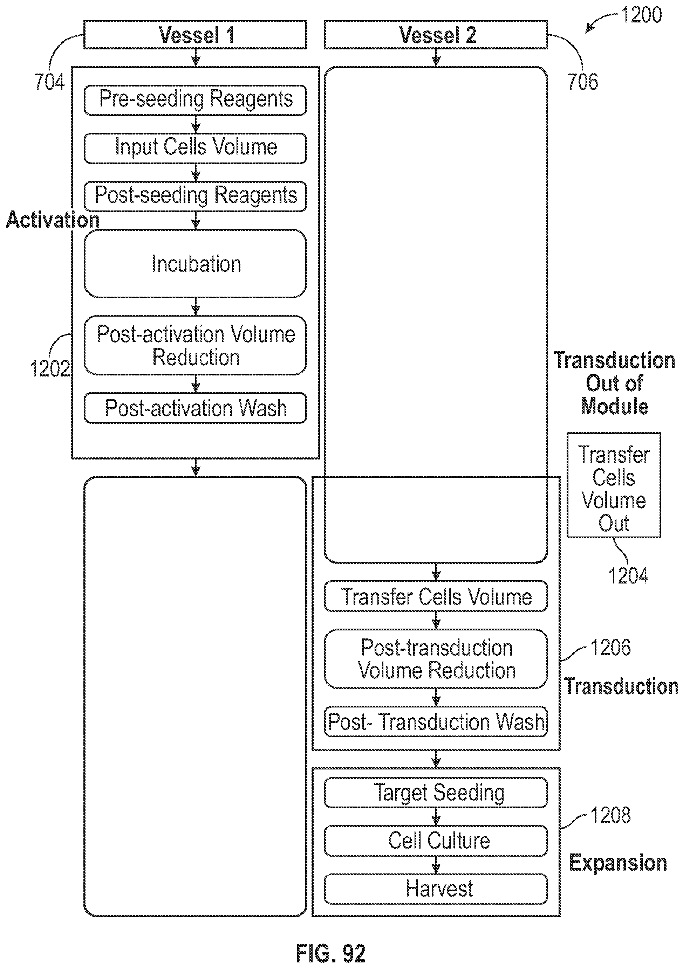

Turning now to FIG. 92, yet another workflow 1200 enabled by the bioprocessing apparatus 600 is illustrated. As shown therein, the workflow or method 1200 includes, for a population of cells, carrying out the activation steps 1202 in the first culture vessel 704, and then transferring the activated population of cells from the first culture vessel 704 entirely out of the bioprocessing apparatus 600 for off-board transduction, at step 1204. After transduction out of the module/apparatus 600, the cell volume is transferred into the second culture vessel 706 of the bioprocessing apparatus 600 for post-transduction volume reduction and post-transduction wash steps 1206 in the second culture vessel 706. As also shown therein, expansion steps 1208 are also carried out in the second culture vessel 706.

In an embodiment, the bioprocessing apparatus 600, disposable bioprocessing kit 700 and flow architecture of the disclosure allow for washing, e.g., using the hollow fiber filter, to be carried out both post-activation, as well as post-transduction.

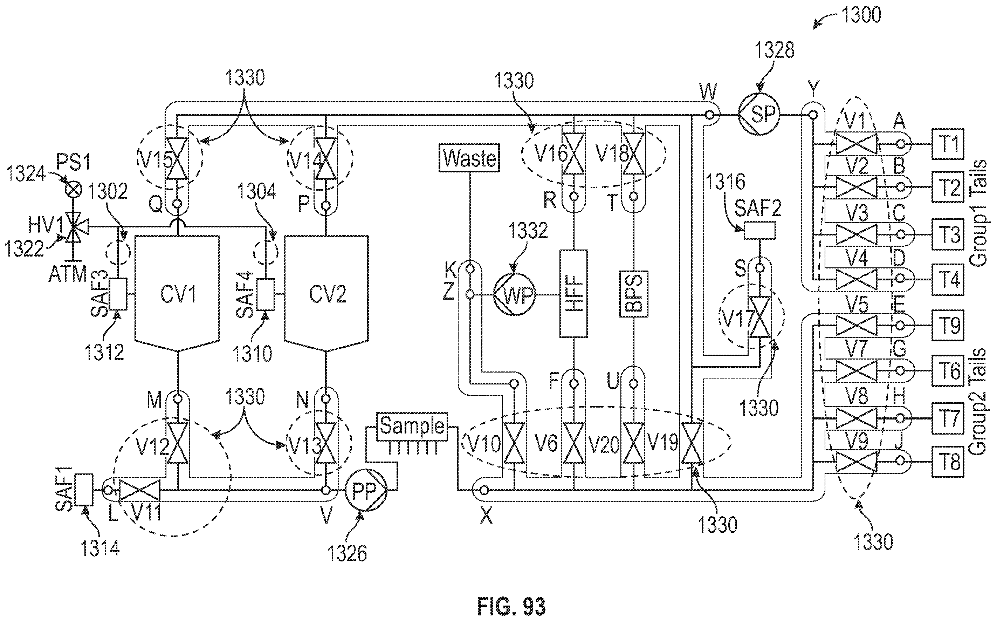

In connection with the use of the bioprocessing apparatus 600 to carry out activation, transduction and expansion of a cell population in the manner described above, it is a common requirement to all disposable devices used in cell culture and bioprocess, in order to ensure the batch quality and product safety, to be sterile but also functionally closed and fully reliable during its operation period. Accordingly, embodiments of the disclosure also provide for leak tightness verification and blockage detection checks to be carried out on the disposable bioprocessing kit 700, including the culture vessels 704, 706 and associated tubing, prior to use. Turning to FIG. 93, a flow architecture 1300 employed by the apparatus 600 and disposable kit 700 according to a disclosed embodiment is illustrated. The flow architecture 1300 is generally similar to flow architecture 400 disclosed above.

As shown therein, the flow architecture/system 1300 includes a plurality of pneumatic interfaces (e.g., two 1302, 1304, or four pneumatic interfaces 1302, 1304, 1306, 1308) that allow air to be drawn into the system 1300. The pneumatic interfaces 1302, 1304, 1306, 1308 allow for a leak tight connection with respect to sterile air filters 1310, 1312, 1314, 1316 associated with each interface, and which form a part of the disposable kit 700. The system 1300 further includes a three-way valve 1322, in addition to three-way valve 1320, that allows for switching the air flow path to connect the kit through the sterile air filters 1310, 1312 to the surrounding atmosphere external to the kit within the process drawer 604 or to a pressure monitoring sensor 1324, and a three way valve 1323. The system 1300 further includes two peristaltic pumps 1326, 1328 (e.g., process pump 456 and source pump 454 of the peristaltic pump assembly 641) intended to act as a pressurization means and pinch valves during the leak tightness verification process, and as a liquid management means during normal operation, as disclosed above, a set of up to twenty pinch valves 1330 (#1 to #20) (e.g., formed by the valve manifold 712 and linear actuator array 643), and one peristaltic pump 1332 (e.g., waste pump 492 of the peristaltic pump assembly 641) intended to act as pinch valve during the leak tightness verification and as a liquid management mean during normal operation.

Air can be drawn into the system via the peristaltic pumps through the pneumatic interfaces that enable selective connection of the flow path to atmosphere via a sterile air filter. In an embodiment, there are two main uses of this interface, (1) to allow for pressurizing portions of the disposable kit 700 during a kit integrity check, as discussed below, and (2) to draw in sterile air to clear fluid from the lines during various automated workflows.

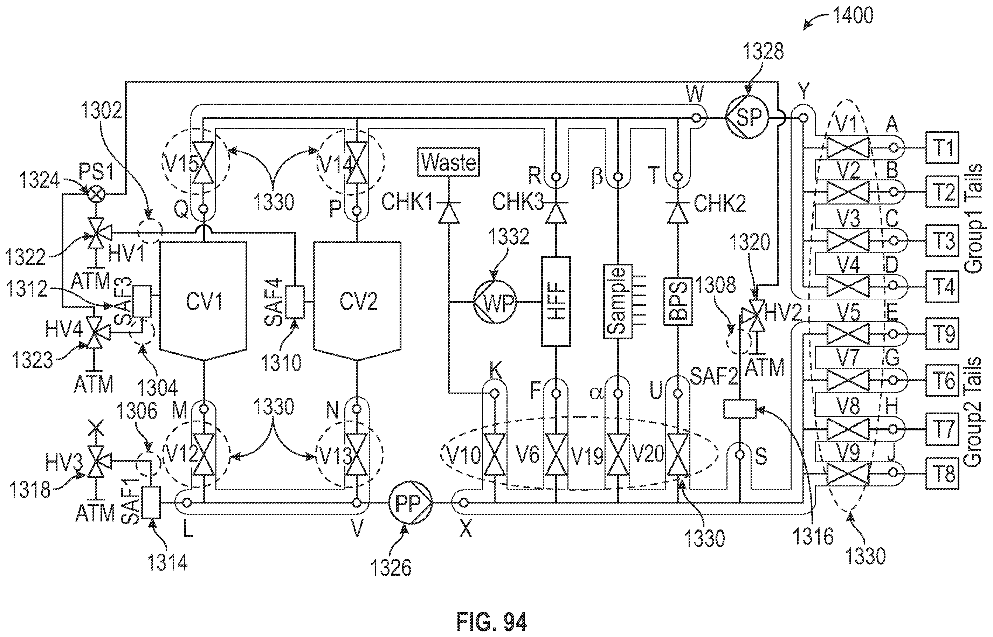

FIG. 94 illustrates another flow architecture/system 1400 that may be employed by the apparatus 600 and disposable kit 700, instead of architecture 1300, according to another disclosed embodiment. Flow architecture/system 1400 is similar to flow architecture/system 1300, where like reference numerals designate like parts. As shown therein, the system 1440 has four pneumatic interfaces 1302, 1304, 1306, 1308, one of which (pneumatic interface 1306) connects to a three-way valve 1318 that switches between atmosphere and the pressure sensor. An advantage of the flow architecture/system 1400 is that the culture vessels can be pressurized independently from the rest of the disposable kit 700 (prior to commencing bioprocessing operations). This allows for checking the culture vessels at one pressure, and the rest of the kit at another pressure (potentially higher than the culture vessels can withstand). This also enables the rest of the kit 700 and flow lines thereof to be tested under negative pressure, which is typically avoided within the culture vessels since the membrane can be dislodged or displaced.

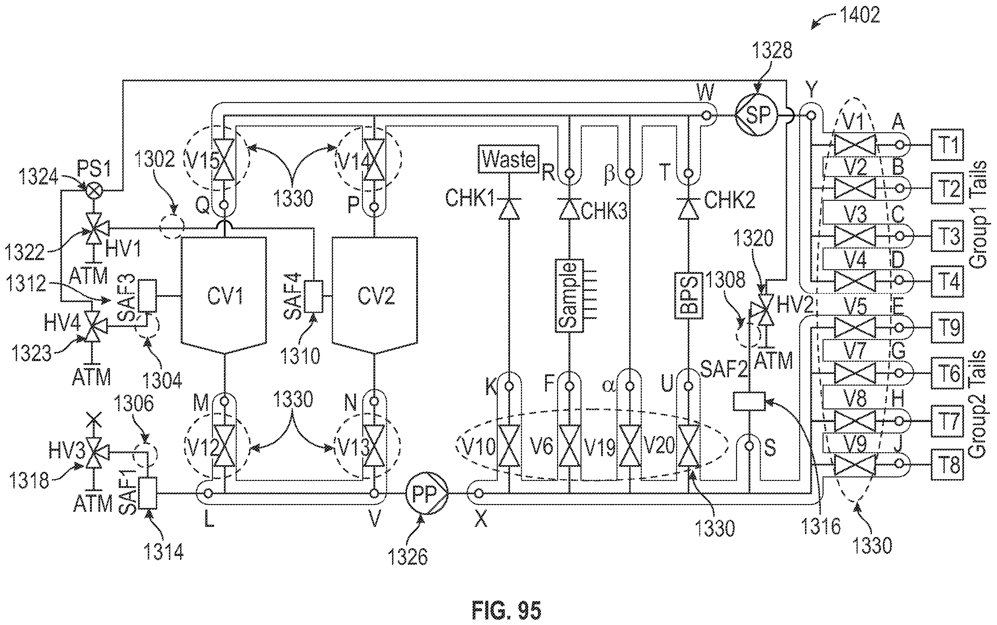

FIG. 95 illustrates another flow architecture/system 1402 that may be employed by the apparatus 600 and disposable kit 700, instead of architecture 1300 or 1400, according to another disclosed embodiment. Flow architecture/system 1402 is similar to flow architecture/system 1400, where like reference numerals designate like parts. As shown therein, however, the flow architecture 1402 of FIG. 95 omits the hollow fiber filter (HFF) and waste pump. The flow architecture 1402 of FIG. 95 is operable in a similar manner as that described above in connection with the flow architecture 1400 of FIG. 94.

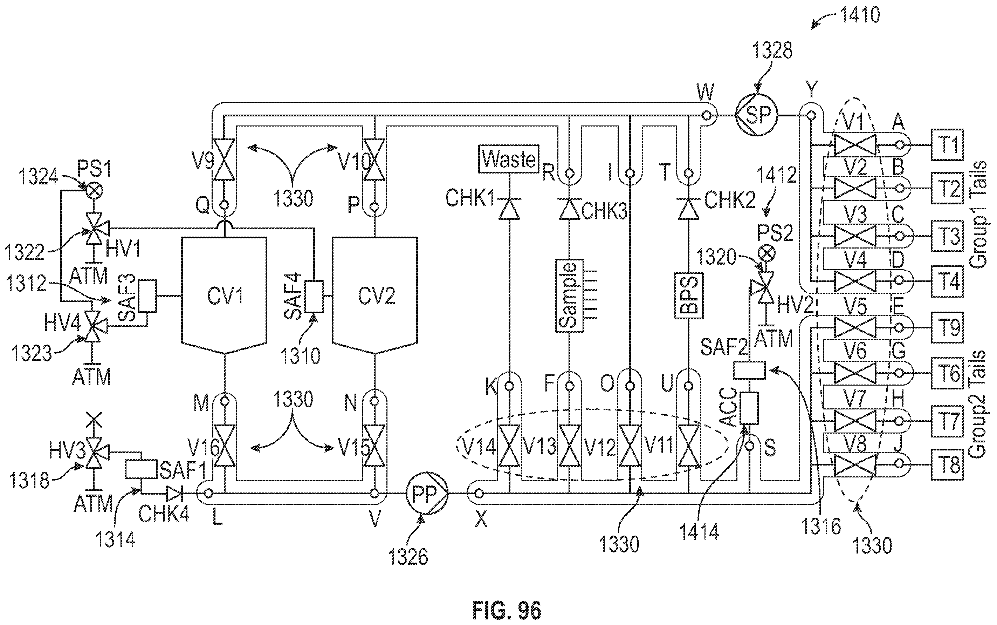

FIG. 96 illustrates yet another flow architecture/system 1410 that may be employed by the apparatus 600 and disposable kit 700, instead of architecture 1300, 1400 or 1402, according to another disclosed embodiment. Flow architecture/system 1410 is similar to flow architecture/system 1402, where like reference numerals designate like parts. As shown therein, however, there is an additional pressure sensor 1412 fluidly connected to the three-way valve 1320 (instead of the flow line running from three-way valve 1320 to pressure sensor 1324 of FIG. 95). In particular, it has been recognized that utilizing more than one pressure sensor may provide certain advantages depending on particular architecture and application (as opposed to the single pressure sensor employed in the architecture of FIG. 95). In an embodiment, the first pressure sensor 1324 and the second pressure sensor 1412 may have different pressure ranges, suited for their particular use. It should be recognized, however, that in certain embodiments, the first and second pressure sensors 1324, 1410 may have the same or similar pressure range.

In an embodiment, the flow architecture/system 1410 may further include an accumulator 1414. The accumulator 1414 functions as a volume buffer, and may be constructed as a reservoir or length of tubing. Regardless of particular construction or configuration, the accumulator 1414 has a volume greater than or equal to the total volume of the fluid flowpath between/from sterile air filter 1316 and the second pressure sensor 1412. In use, in the event there is a clog, the presence and location of the accumulator 1414 ensures that the volume of fluid will build up in the accumulator 1414 and not contact the sterile air filter.

In many of the components, systems, devices and architectures disclosed above, reference has been made to the use, or employment, of sterile air filters. In an embodiment, one or more, or all, of these sterile air filters may be hydrophobic so that they can be exposed to, or come in contact with, fluid, and still maintain their integrity and function as intended. In yet other embodiments, non-hydrophobic filters may be employed, with or without an accumulator or similar device, depending on particular system or architecture layout and application.

In an embodiment, the leak tightness verification referenced above is executed independently on three differentiated segments of the disposable culture kit. In an embodiment, the first segment includes the entire disposable kit (i.e., the entirety of the fluid flow paths thereof) except the tubing segments between the source pump 1328/454 and the tubing tails 1334a-d (e.g., tubing tails of the tubing organizer 720). The test of the first segment takes place in two phases, the pressurization phase and pressure decay monitorization phase. In an embodiment, the second segment includes the two culture vessels and the tubing from the inlet ports until the supply pump and the tubing segments between the source pump 1328/454 and the tubing tails 1334a-d. The test of the second segment takes place in two phases, the pressurization phase and pressure decay monitorization phase. In an embodiment, the third segment includes the entire disposable kit (i.e., the entirety of the fluid flow paths thereof) except T/U loop (sensor bypass) and the tubing segments between the source pump 1328/454 and the tubing tails 1334a-d. The test of the third segment takes place in three phases, the pressurization phase, pressure decay monitorization and the pressure release phase.

As indicated above, the leak tightness verification and blockage detection methods disclosed above enable the end user to run an automated integrity test on the entire disposable kit 700 before commencing bioprocessing operations. This enables the end user to detect possible leaks within the disposable kit and/or blocked/pinched lines that would negatively impact the ability to conduct the automated workflow and, ultimately, the quality of the batch.

As indicated above, mammalian cell cultures processes may require significantly complex liquid transfer management operations which must be executed in an accurate and safe manner. Accordingly, the ability to detect leak events is a key function which should carried out continuously in order raise an alarm if the viability of the batch may potentially be compromised. In view of the above, disclosed embodiments also contemplate verifying leak tightness and detecting blockages in the disposable kit 700 using real-time monitoring of the masses involved in a bioprocess. In most, if not all, of the bioprocessing operations disclosed herein, four situations are customarily present or taking place at all times: (1) a fluid is held within a closed container, (2) a fluid is transfer from a source container to a destination container, (3) a fluid is perfused through an intermediate container from a source container to a destination container, and/or (4) a fluid is recirculated from a container or vessel and back to the same container or vessel. Accordingly so long as the source container, intermediate container and/or destination container include a means/mechanism for measuring the mass of such containers (e.g., one or more load cells associated with each container, as disclosed above), a means for pumping fluid from one container to another or looping from and to the same container in a leak-tight manner (e.g., using the peristaltic pump assembly 641), and a control unit (e.g., controller 210) for monitoring the variation of each container's mass, a number of leak and/or blockage detection processes can be carried out, as disclosed below. The load cells may include, for example, bed plates supporting various containers (e.g., the culture vessels 704, 706, waste bag, etc.) or pegs or hooks having integrated load cells (e.g., the hooks 620 on the vertical storage drawers 614, 616 of the cabinet 608 for suspending media, reagent and other bags), as disclosed above. As disclosed below, the controller (e.g., controller 210) is configured to monitor the variation in the mass of each container, actuate a pumping means to transfer fluid between containers, execute mass balancing equations, and generate an alarm or alert if the mass balancing equation solutions do not indicate leak tightness or the absence of blockages.

In one embodiment, no pumping action takes place and the control unit 210 just verifies that the mass of a first container remains generally constant (e.g., within a predetermined or preset change threshold over a predetermined duration). If the change in mass is below a predetermined threshold amount, this indicates that volume of fluid within the first container has remained constant, indicating that no leaks are present. If, however, the change in mass exceeds the threshold, this indicates that fluid has leaked from the container, and the controller 210 generates an alert to a user.

In another embodiment, a method for detecting leaks or blockages involves monitoring the mass of a first, source container and a second, destination container, and transferring a fluid from the first container to the second container. For example, a pump of the apparatus 600 is controlled by the controller 210 to pump a fluid from the first container to the second while monitoring the masses of each container using the associated load cells. In particular, in such embodiment, the mass of the first container (and thus the mass of a volume of fluid therein) is first determined. The volume of fluid from the first container is then transferred to the second container. Next, the mass of the second container (and thus the mass of the volume of fluid within the second container) is determined. The controller 210 then compares the original mass of the volume of fluid in the first container with the mass of the volume of fluid in the second container, which should be approximately equivalent if no leaks or blockages are present. If the difference between the original mass of the volume of fluid in the first container and the mass of the transferred volume of fluid in the second container exceeds a threshold, the controller 210 generates a notification or alert. In an embodiment, the controller can also carry out the above leak detection process without necessitating that the entire volume of fluid is transferred between containers. In particular, in an embodiment, the controller 210 is configured to verify whether or not the source container mass volume absolute variation is/remains below the transfer flow rate plus a specified leak rate detection threshold, and whether or not the destination container mass volume absolute variation is/remains above or equal to the transfer flow rate minus the specified leak rate detection threshold. If not, a leak alarm will be triggered by the controller 210.

In yet another embodiment of leak tightness verification using real time mass balance monitoring, the objective is to keep the mass of an intermediate (e.g., third) container constant. Therefore, the simultaneous action of two pumps of the peristaltic pump assembly 641 are necessary, where the source to intermediate container liquid transfer must be controlled on the source container variation with respect to a specified flow setpoint, and the intermediate to destination container liquid transfer must be controlled on the destination container variation with respect to a specified flow setpoint. The control unit 210 is configured to verify whether or not the source container mass volume absolute variation is/remains below the transfer flow rate plus the specified leak rate detection threshold, that the intermediate container volume absolute variation is/remains below the specified leak rate detection threshold, and that the destination container mass volume absolute variation is/remains above or equal to the transfer flow rate minus the specified leak rate detection threshold. If any of these conditions is not present, then the controller 210 is configured to generate an alert.

In yet another embodiment, leak tightness verification is carried out by the controller 210 by controlling a pump to recirculate a fluid from a first container, out of the first container, and back to the first container. Accordingly, pumping of the fluid takes place in open loop and control unit 210 is configured to verify that the first container's mass volume absolute variation is/remains below the specified leak rate detection threshold. If not, a leak alarm will be triggered by the controller 210. A variation to this process is if a sample volume is drawn out of the recirculation loop. In this case, the controller 210 verifies whether or not the first container's mass volume absolute variation stays below the specified leak rate detection threshold plus the sampling flow rate.

Disclosed embodiments therefore utilize real time mass balance calculations to check for leak tightness and/or detect blockages in the kit 700 prior to utilizing the kit 700 in bioprocess operations. The method disclosed herein, however are not limited to determining leaks prior to use of the kit 700 in a bioprocess, but can also be utilized during the bioprocess for real-time leak verification or blockage detection. Accordingly, it may be possible to take remedial action with respect to any blockages or leaks detected in order to save or salvage the batch.

In the embodiments disclosed above, the first, source bag/container may be a media bag, the second, destination bag/container may be a waste bag, and the third, intermediate bag/container may be a culture vessel or bioreactor vessel. The disclosure is not intended to be so limited in this regard, however, and a variety of bags/vessels may be used as the first, second and third containers so long as fluid can be transferred between and/or through such containers. Moreover, while the mass balancing processes for verifying leak tightness and for detecting blockages has been described as being carried out on the bioprocessing apparatus 600 and disposable bioprocessing kit 700, the disclosure is not intended to be so limited in this regard. In particular, it is contemplated that the mass balancing techniques may be carried out on a variety of systems and devices, including the processing apparatus 102 and isolation module (and disposable kits therefor) disclosed above in connection with the first and third modules 100, 300.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property.

This written description uses examples to disclose several embodiments of the invention, including the preferred mode, and also to enable one of ordinary skill in the art to practice the embodiments of invention, including making and using any devices or systems and performing any incorporated methods. The patentable scope of the invention is defined by the claims, and may include other examples that occur to one of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A rocking mechanism (640) for a bioreactor vessel (704, 706), comprising:
a base (870);
a motor (874) mounted to the base (870) and having an eccentric roller (876) driven by the motor (874); and
a rocking plate (878) in contact with the eccentric roller (876), the rocking plate (878) being configured to receive a bioreactor vessel (704, 706) thereon;
wherein the motor (874) is controllable to drive the eccentric roller (876) to transmit a force against an underside of the rocking plate (878) to tilt the rocking plate (878) and bioreactor vessel (704, 706), **characterized in that** said rocking mechanism (640) further comprises a compression spring (880) configured to maintain contact between the rocking plate (878) and the eccentric roller (876).

2. The rocking mechanism (640) of claim 1, wherein:
the motor (874) and eccentric roller (876) are configured to produce a continuous, sinusoidal rocking profile for the bioreactor vessel (704, 706).

3. The rocking mechanism (640) of claim 1 or 2, wherein:
the rocking plate (878) includes a plurality of mounting pins (646) for retaining the bioreactor vessel (704, 706) on the rocking plate (878).

4. The rocking mechanism (640) of claim 3, wherein:
the plurality of mounting pins (646) are at least four mounting pins configured to engage the bioreactor vessel (704, 706) adjacent to corners of the bioreactor vessel (704, 706); and/or
the plurality of mounting pins (646) maintain the bioreactor vessel (704, 706) in spaced vertical relation from the rocking plate (878), enabling a bottom surface of the bioreactor vessel (704, 706) to be accessible for heat transfer and/or aeration.

5. The rocking mechanism (640) of any preceding claim, further comprising:
a tilt sensor (884) connected to the rocking plate (878) and being configured to measure a tilt angle of the rocking plate (878);
at least one load cell (882) associated with the base plate (870), the at least one load cell (882) permitting measurement of a mass of the bioreactor vessel (704, 706) received atop the rocking plate (878); and/or
a fulcrum (872) defining a pivot axis of the rocking plate (878), the rocking plate (878) being received atop the fulcrum (872).

6. The rocking mechanism (640) of any preceding claim, wherein:
the eccentric roller (876) is a circular roller configured to more along an eccentric path.

7. The rocking mechanism (640) of claim 3, or any claim when dependent thereon, further comprising:
a load cell (658, 660, 662) associated with at least one of the mounting pins (646).

8. A method of bioprocessing, comprising the steps of:
receiving a bioreactor vessel (704, 706) atop a rocking plate (878);
actuating a motor (874) to cause an eccentric roller (876) to exert a force on an underside of the rocking plate (878) to tilt the rocking plate (878) and bioreactor vessel (704, 706) about a horizontal axis, **characterized in that**
a compression spring (880) maintains contacting between the rocking plate (878) and the eccentric roller (876).

9. The method according to claim 8, wherein:
the rocking plate (878) is received atop a fulcrum (872) such that exertion of the force on the underside of the rocking plate (878) causes the rocking plate (878) to pivot about the fulcrum (872).

10. The method according to claim 8 or 9, further comprising the step of:
detecting a tilt angle of the rocking plate (878); and
controlling the motor (874) to adjust the tilt angle in dependence upon the detected tilt angle.

11. The method according to any of claims 8 to 10, wherein:
the eccentric roller (876) is a circular roller configured to move along an eccentric path.

12. The method according to any of claims 8 to 11, further comprising the step of:
controlling the motor (874) to tilt the rocking plate back (878) and forth to produce a continuous, sinusoidal rocking profile for the bioreactor vessel (704, 706).

13. The method according to claim 12, further comprising the step of:
tilting the rocking plate (878) and maintaining the rocking plate (878) at a predetermined tilt angle to cause a fluid within the bioreactor vessel (704, 706) to collect at one end of the bioreactor vessel (704, 706); and
draining the fluid from the bioreactor vessel (704, 706).

## Patentansprüche

1. Schaukelmechanismus (640) für einen Bioreaktorbehälter (704, 706), umfassend:
eine Basis (870);
einen Motor (874), der an der Basis (870) befestigt ist und eine Exzenterrolle (876) aufweist, die vom Motor (874) angetrieben wird; und
eine Schaukelplatte (878), die mit der Exzenterrolle (876) in Kontakt steht, wobei die Schaukelplatte (878) so konfiguriert ist, dass sie einen Bioreaktorbehälter (704, 706) aufnehmen kann;
wobei der Motor (874) steuerbar ist, um die Exzenterrolle (876) anzutreiben, um eine Kraft auf die Unterseite der Schaukelplatte (878) auszuüben, um die Schaukelplatte (878) und den Bioreaktorbehälter (704, 706) zu neigen, **dadurch gekennzeichnet, dass** der Schaukelmechanismus (640) weiter eine Druckfeder (880) umfasst, die so konfiguriert ist, dass sie den Kontakt zwischen der Schaukelplatte (878) und der Exzenterrolle (876) aufrechterhält.

2. Schaukelmechanismus (640) nach Anspruch 1, wobei:
der Motor (874) und die Exzenterrolle (876) so konfiguriert sind, dass sie ein kontinuierliches, sinusförmiges Schaukelprofil für den Bioreaktorbehälter (704, 706) erzeugen.

3. Schaukelmechanismus (640) nach Anspruch 1 oder 2, wobei:
die Schaukelplatte (878) eine Vielzahl von Befestigungsstiften (646) einschließt, um den Bioreaktorbehälter (704, 706) auf der Schaukelplatte (878) zu halten.

4. Schaukelmechanismus (640) nach Anspruch 3, wobei:
die Vielzahl von Befestigungsstiften (646) aus mindestens vier Befestigungsstiften besteht, die konfiguriert sind, um mit dem Bioreaktorbehälter (704, 706) in Eingriff zu kommen, der an die Ecken des Bioreaktorbehälters (704, 706) angrenzt; und/oder
die Vielzahl von Befestigungsstiften (646) den Bioreaktorbehälter (704, 706) in einem Abstand in vertikaler Beziehung zur Schaukelplatte (878) hält, wodurch eine Bodenfläche des Bioreaktorbehälters (704, 706) für eine Wärmeübertragung und/oder Belüftung zugänglich ist.

5. Schaukelmechanismus (640) nach einem der vorstehenden Ansprüche, weiter umfassend:
ein Neigungssensor (884), der mit der Schaukelplatte (878) verbunden ist und so konfiguriert ist, dass er einen Neigungswinkel der Schaukelplatte (878) misst;
mindestens eine der Basisplatte (870) zugeordnete Wägezelle (882), wobei die mindestens eine Wägezelle (882) die Messung einer Masse des auf der Schaukelplatte (878) aufgenommenen Bioreaktorbehälters (704, 706) ermöglicht; und/oder
einen Drehpunkt (872), der eine Schwenkachse der Schaukelplatte (878) definiert, wobei die Schaukelplatte (878) oben auf dem Drehpunkt (872) aufgenommen ist.

6. Schaukelmechanismus (640) nach einem der vorstehenden Ansprüche, wobei:
die Exzenterrolle (876) eine kreisrunde Rolle ist, die so konfiguriert ist, dass sie sich entlang einer exzentrischen Bahn bewegt.

7. Schaukelmechanismus (640) nach Anspruch 3, oder jedem davon abhängigen Anspruch, weiter umfassend:
eine Wägezelle (658, 660, 662), die mindestens einem der Befestigungsstifte (646) zugeordnet ist.

8. Verfahren zur biologischen Verarbeitung, umfassend die folgenden Schritte:
Aufnehmen eines Bioreaktorbehälters (704, 706) oben auf einer Schaukelplatte (878);
Betätigen eines Motors (874), um zu bewirken, dass eine Exzenterrolle (876) eine Kraft auf eine Unterseite der Schaukelplatte (878) ausübt, um die Schaukelplatte (878) und den Bioreaktorbehälter (704, 706) um eine horizontale Achse zu neigen, **dadurch gekennzeichnet, dass**
eine Druckfeder (880) den Kontakt zwischen der Schaukelplatte (878) und der Exzenterrolle (876) aufrechterhält.

9. Verfahren nach Anspruch 8, wobei:
die Schaukelplatte (878) oben auf einem Drehpunkt (872) so aufgenommen ist, sodass die Ausübung einer Kraft auf die Unterseite der Schaukelplatte (878) bewirkt, dass sich die Schaukelplatte (878) um den Drehpunkt (872) dreht.

10. Verfahren nach Anspruch 8 oder 9, weiter umfassend den Schritt von:
Erfassen eines Neigungswinkels der Schaukelplatte (878); und
Steuern des Motors (874), um den Neigungswinkel in Abhängigkeit vom erfassten Neigungswinkel einzustellen.

11. Verfahren nach einem der Ansprüche 8 bis 10, wobei:
die Exzenterrolle (876) eine kreisrunde Rolle ist, die so konfiguriert ist, dass sie sich entlang einer exzentrischen Bahn bewegt.

12. Verfahren nach einem der Ansprüche 8 bis 11, weiter umfassend den Schritt von:
Steuern des Motors (874), um die Schaukelplatte (878) hin und her zu neigen, und um ein kontinuierliches, sinusförmiges Schaukelprofil für den Bioreaktorbehälter (704, 706) zu erzeugen.

13. Verfahren nach Anspruch 12, weiter umfassend den Schritt von:
Neigen der Schaukelplatte (878) und Halten der Schaukelplatte (878) in einem vorbestimmten Neigungswinkel, um zu bewirken, dass sich eine Flüssigkeit innerhalb des Bioreaktorbehälters (704, 706) an einem Ende des Bioreaktorbehälters (704, 706) sammelt; und
Ablassen der Flüssigkeit aus dem Bioreaktorbehälter (704, 706).

## Revendications

1. Mécanisme de basculement (640) pour récipient de bioréacteur (704, 706), comprenant :
une base (870) ;
un moteur (874) monté sur la base (870) et présentant un rouleau excentrique (876) entraîné par le moteur (874) ; et
une plaque basculante (878) en contact avec le rouleau excentrique (876), la plaque basculante (878) étant configurée pour recevoir un récipient de bioréacteur (704, 706) sur celle-ci ;
dans lequel le moteur (874) peut être commandé pour entraîner le rouleau excentrique (876) de manière à transmettre une force contre un côté inférieur de la plaque basculante (878) pour incliner la plaque basculante (878) et le récipient de bioréacteur (704, 706), **caractérisé en ce que** ledit mécanisme de basculement (640) comprend en outre un ressort de compression (880) configuré pour maintenir un contact entre la plaque basculante (878) et le rouleau excentrique (876).

2. Mécanisme de basculement (640) selon la revendication 1, dans lequel :
le moteur (874) et le rouleau excentrique (876) sont configurés pour produire un profil de basculement sinusoïdal continu pour le récipient de bioréacteur (704, 706).

3. Mécanisme de basculement (640) selon la revendication 1 ou 2, dans lequel :
la plaque basculante (878) inclut une pluralité de tiges de montage (646) permettant de retenir le récipient de bioréacteur (704, 706) sur la plaque basculante (878).

4. Mécanisme de basculement (640) selon la revendication 3, dans lequel :
la pluralité de tiges de montage (646) sont au moins quatre tiges de montage configurées pour mettre en prise le récipient de bioréacteur (704, 706) de manière adjacente à des angles du récipient de bioréacteur (704, 706) ; et/ou
la pluralité de tiges de montage (646) maintiennent le récipient de bioréacteur (704, 706) dans une relation verticale espacée par rapport à la plaque basculante (878), permettant à une surface inférieure du récipient de bioréacteur (704, 706) d'être accessible pour un transfert de chaleur et/ou une aération.

5. Mécanisme de basculement (640) selon une quelconque revendication précédente, comprenant en outre :
un capteur d'inclinaison (884) relié à la plaque basculante (878) et étant configuré pour mesurer un angle d'inclinaison de la plaque basculante (878) ;
au moins une cellule de charge (882) associée à la plaque de base (870), la au moins une cellule de charge (882) permettant un mesurage d'une masse du récipient de bioréacteur (704, 706) reçu au-dessus de la plaque basculante (878) ; et/ou
un point d'appui (872) définissant un axe de pivot de la plaque basculante (878), la plaque basculante (878) étant reçue au-dessus du point d'appui (872).

6. Mécanisme de basculement (640) selon une quelconque revendication précédente, dans lequel :
le rouleau excentrique (876) est un rouleau circulaire configuré pour se déplacer le long d'un trajet excentrique.

7. Mécanisme de basculement (640) selon la revendication 3, ou toute revendication lorsque dépendante de celle-ci, comprenant en outre :
une cellule de charge (658, 660, 662) associée à au moins l'une des tiges de montage (646).

8. Procédé de biotraitement, comprenant les étapes consistant à :
recevoir un récipient de bioréacteur (704, 706) au-dessus d'une plaque basculante (878) ;
actionner un moteur (874) pour amener un rouleau excentrique (876) à exercer une force sur un côté inférieur de la plaque basculante (878) de manière à incliner la plaque basculante (878) et le récipient de bioréacteur (704, 706) autour d'un axe horizontal, **caractérisé en ce que**
un ressort de compression (880) maintient une mise en contact entre la plaque basculante (878) et le rouleau excentrique (876).

9. Procédé selon la revendication 8, dans lequel :
la plaque basculante (878) est reçue au-dessus d'un point d'appui (872) de sorte que l'exercice de la force sur le côté inférieur de la plaque basculante (878) amène la plaque basculante (878) à pivoter autour du point d'appui (872).

10. Procédé selon la revendication 8 ou 9, comprenant en outre les étapes consistant à :
détecter un angle d'inclinaison de la plaque basculante (878) ; et
commander le moteur (874) pour régler l'angle d'inclinaison en fonction de l'angle d'inclinaison détecté.

11. Procédé selon l'une quelconque des revendications 8 à 10, dans lequel :
le rouleau excentrique (876) est un rouleau circulaire configuré pour se déplacer le long d'un trajet excentrique.

12. Procédé selon l'une quelconque des revendications 8 à 11, comprenant en outre l'étape consistant à : commander le moteur (874) pour incliner la plaque basculante (878) d'avant en arrière de manière à produire un profil de basculement sinusoïdal continu pour le récipient de bioréacteur (704, 706).

13. Procédé selon la revendication 12, comprenant en outre les étapes consistant à :
incliner la plaque basculante (878) et maintenir la plaque basculante (878) selon un angle d'inclinaison prédéterminé pour amener un fluide à l'intérieur du récipient de bioréacteur (704, 706) à se rassembler au niveau d'une extrémité du récipient de bioréacteur (704, 706) ; et
drainer le fluide à partir du récipient de bioréacteur (704, 706).
